(19) Europäisches Patentamt
European Patent Office
Office européen des brevets

(11) Publication number: **0 168 707 A1**

(12) **EUROPEAN PATENT APPLICATION**

(21) Application number: **85108134.9**

(22) Date of filing: **01.07.85**

(51) Int. Cl.⁴: **C 07 D 487/04**, A 61 K 31/40, C 07 D 519/00

(30) Priority: **02.07.84 US 626822**

(43) Date of publication of application: **22.01.86 Bulletin 86/4**

(84) Designated Contracting States: **AT BE CH DE FR GB IT LI LU NL SE**

(71) Applicant: **MERCK & CO. INC., 126, East Lincoln Avenue P.O. Box 2000, Rahway New Jersey 07065 (US)**

(72) Inventor: **Christensen, Burton G., 770 Anderson Avenue, Apt. 19H, Cliffside Park New Jersey 07010 (US)**
Inventor: **Johnston, David B.R., 53 Round Top Road, Warren New Jersey 07060 (US)**
Inventor: **Schmitt, Susan M., 50 M Southwyck Village, Scotch Plains New Jersey 07076 (US)**

(74) Representative: **Abitz, Walter, Dr.-Ing. et al, Abitz, Morf, Gritschneder, Freiherr von Wittgenstein Postfach 86 01 09, D-8000 München 86 (DE)**

(54) **1-Methylcarbapenems having a 2-quaternary heteroarylalkylthio substituent.**

(57) Compounds of the formula:

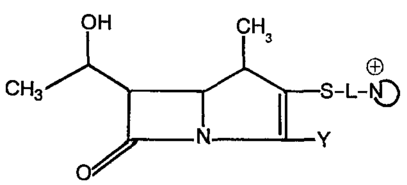

wherein 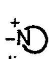 is a substituted or unsubstituted quaternized, monocyclic or bicyclic heteroaryl group, their preparation and use as antibiotics are disclosed.

EP 0 168 707 A1

TITLE OF THE INVENTION

1-METHYLCARBAPENEMS HAVING A 2-QUATERNARY HETEROARYLALKYLTHIO SUBSTITUENT

BACKGROUND OF THE INVENTION

The present invention is concerned with improved carbapenem antibiotics characterized by having a monocyclic or bicyclic, quaternary heteroarylalkylthio substituent in the 2-position and a methyl group in the 1-position.

Thienamycin is a known carbapenem, broad spectrum antibiotic of the formula:

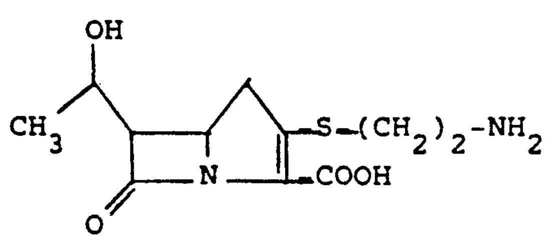

A

Other derivatives of A are also known. The present quaternary, monocyclic or bicyclic heteroarylalkylthio substituted carbapenems have an antibiotic spectrum equal to or better than A. The present carbapenems also are more resistant than A to degradation by the dehydropeptidase enzyme DHP-I. The present carbapenems are also more resistant to DHP-I mediated degradation and are chemically more stable, both in solution and in the solid state, than the corresponding carbapenems lacking the 1-methyl substituent.

SUMMARY OF THE INVENTION

Carbapenems having the formula

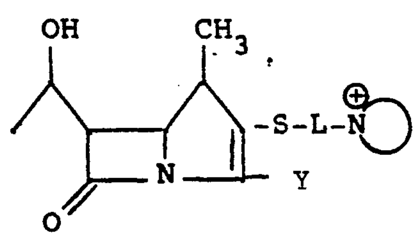

where 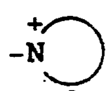 is a quaternary, monocyclic or bicyclic heteroaryl group and their use as antibiotics.

DETAILED DESCRIPTION OF THE INVENTION

An embodiment of the present invention is a compound having the formula

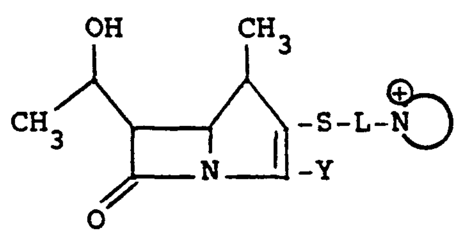

I

wherein

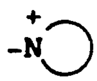 is a quaternary, monocyclic or bicyclic, substituted or unsubstituted heteroaryl group containing (a) when monocyclic, up to 3 heteroatoms and up to 6 total ring atoms or (b) when bicyclic up to 5 heteroatoms and 9-10 ring atoms, which is optionally substituted by one or more of the groups independently selected from

1) a substituted or unsubstituted $C_1$-$C_6$ alkyl, $C_2$-$C_6$ alkenyl, $C_5$-$C_7$ cycloalkenyl, $C_3$-$C_7$ cycloalkyl, or ($C_3$-$C_7$ cycloalkyl) methyl;
2) a substituted or unsubstituted $C_3$-$C_7$ heterocycloalkyl or ($C_3$-$C_7$ heterocycloalkyl) methyl having up to 3 hetero ring atoms;
3) an unsubstituted or substituted phenyl or heteroaryl radical;
4) an unsubstituted or substituted phenyl ($C_1$-$C_4$ alkyl) or heteroaryl ($C_1$-$C_4$ alkyl) radical;
5) a trifluoromethyl or pentafluoroethyl group;
6) a halogen atom;
7) an unsubstituted or substituted $C_1$-$C_4$ alkoxy radical;
8) a hydroxyl group;
9) an unsubstituted or substituted ($C_1$-$C_6$ alkyl) carbonyloxy radical;
10) a carbamoyloxy radical which is unsubstituted, monosubstituted or disubstituted on the nitrogen with a $C_1$-$C_4$ alkyl group;

11) a $C_1$-$C_6$ alkylthio radical, $C_1$-$C_6$ alkylsulfinyl radical or a $C_1$-$C_6$ alkylsulfonyl radical each of which is unsubstituted or substituted in the alkyl group;

12) a sulfamoyl group which is unsubstituted, monosubstituted, or disubstituted on nitrogen with a $C_1$-$C_4$ alkyl group,

13) an amino group;

14) a mono ($C_1$-$C_4$ alkyl) amino or di ($C_1$-$C_4$ alkyl) amino radical each of which is unsubstituted or substituted in the alkyl group;

15) a formylamino group;

16) an unsubstituted or substituted ($C_1$-$C_6$ alkyl) carbonylamino radical;

17) a ($C_1$-$C_4$ alkoxy) carbonylamino radical;

18) a ureido group in which the terminal nitrogen atom is unsubstituted or monosubstituted with a $C_1$-$C_6$ alkyl group;

19) a ($C_1$-$C_6$ alkyl) sulfonamido group;

20) a cyano group;

21) a formyl or acetalized formyl radical;

22) an unsubstituted or substituted ($C_1$-$C_6$ alkyl) carbonyl radical wherein the carbonyl group is free or acetalized;

23) an unsubstituted or substituted phenylcarbonyl or heteroarylcarbonyl radical;

24) a hydroxyiminomethyl radical in which the oxygen or carbon atom is optionally substituted by a $C_1$-$C_4$ alkyl group;

25) a ($C_1$-$C_6$ alkoxy) carbonyl radical;

26) a carbamoyl radical which is unsubstituted, monosubstituted, or disubstituted on the nitrogen atom with a $C_1$-$C_4$ alkyl group;

27) a N-hydroxy carbamoyl or N-($C_1$-$C_4$ alkoxy) carbamoyl radical in which the nitrogen atom may be additionally substituted by a $C_1$-$C_4$ alkyl group;

28) a thiocarbamoyl group;

29) an amidino group 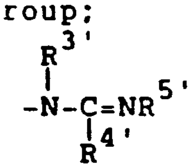 or

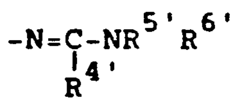

wherein $R^{3'}$, $R^{4'}$, $R^{5'}$, $R^{6'}$ are hydrogen, $C_1$-$C_4$ alkyl, or wherein two of the groups together form a $C_3$-$C_6$ alkylidine radical optionally interupted by a heteroatom and joined to either one or two nitrogen atoms to form a ring;

30) a guanidino group in which $R^{4'}$ above is $NR^{5'}R^{6'}$;

31) a carbamimidoyl group (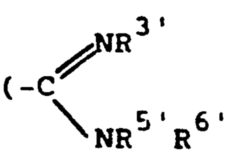) wherein $R^{3'}$, $R^{5'}$ and $R^{6'}$ are as defined above;

32) a cyano ($C_1$-$C_4$ alkyl) radical;

33) a carbamoyl ($C_1$-$C_4$ alkyl) radical;

34 ) a hydroxy ($C_1$-$C_4$ alkyl) radical;

35 ) an amino ($C_1$-$C_6$ alkyl) radical which is unsubstituted, monosubstituted, or disubstituted on the nitrogen atom with $C_1$-$C_4$ alkyl groups, and

36) an acidic side-chain of the structure -A or $-(CH_2)_n-X-(CH_2)_m-Y-A$ where:

n = 0-4

m = 0-4

X = $CHR^s$, CH=CH, phenylene ($-C_6H_4-$), NH, N(C1-C4 alkyl), O, S, S=O, C=O, $SO_2$, $SO_2NH$, $CO_2$, CONH,$OCO_2$, OC=O, NHC=O;

$R^s$ = H, O(C1-C4 alkyl), $NH_2$, NH(C1-C4 alkyl), N(C1-C4 alkyl)$_2$, CN, $CONH_2$, CON(C1-C4 alkyl)$_2$, $CO_2H$, $SO_2NH_2$, $SO_2$NH(C1-C4 alkyl);

Y = single bond, NH, N(C1-C4 alkyl), O, S;

A = an acidic function such as carboxy ($CO_2H$), phosphono [P=O$(OH)_2$], alkylphosphono P=O(OH)[O(C1-C4 alkyl)] , alkylphosphinyl [P=O(OH)(C1-C4 alkyl)], substituted phosphoramido [P=O(OH)NH(C1-C4 alkyl) and P=O(OH)$NHR^x$], sulfino ($SO_2H$), sulfo ($SO_3H$), 5-tetrazolyl ($CN_4H$), arylsulfonamido ($SO_2NHR^x$) and acylsulfonamides represented by the structures $CONHSO_2$(C1-C4 alkyl), $CONHSO_2$N(C1-C4 alkyl)$_2$$SO_2$NHCO(C1-C4 alkyl) and $SO_2NHCOR^x$.

$R^x$ = aryl or heteroaryl as defined above;

wherein the substituents in groups 7), 9), 11), 14), 16 ), 22), and 23 ) are selected from hydroxy, $C_1$-$C_4$alkoxy, mercapto, amino, mono- or di($C_1$-$C_4$alkyl)amino, cyano, halo, $CF_3$, COOH, sulfo, carbamoyl, and sulfamoyl, and wherein the substituents in grous 1)-4) are selected from those defined in groups 5)- 31).

L is a substituted or unsubstituted, straight or branched chain, bivalent $C_1$-$C_6$ alkyl, $C_2$-$C_6$ alkenyl or $C_3$-$C_6$ cycloalkyl, or -$C_1$ $C_4$ alkyl-X-$C_1$-$C_4$ alkyl wherein X is O, S, NH, or N($C_{1-4}$ alkyl), wherein the substituents are selected from groups 2) through 31);

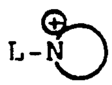 together, in addition to the individual definitions of L and 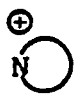 above, may be the group

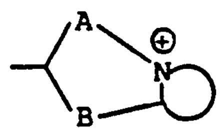

where

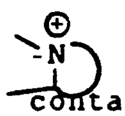 is a 5- to 6-membered heteroaryl group containing from one to three heteroatoms, which is optionally substituted by one or more members independently selected from the group consisting of the substituents defined as 1) - 36 ) under 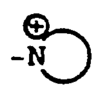 ; and

A and B are independently selected from a direct bond or a substituted or unsubstituted $C_1$-$C_4$alkylene group optionally interrupted by a heteroatom; provided that, A and B cannot both be a direct bond, and that the ring containing A and B contains from 5 to 8 atoms; and

wherein the substituents on A and B are selected from hydroxyl, $C_1$-$C_3$alkoxy, amino, carboxyl, cyano, carbamoyl, trifluoromethyl, $C_1$-$C_4$alkyl, and di($C_1$-$C_4$alkyl)amino;
and

Y is
i) -COOH, a pharmaceutically acceptable ester or salt thereof;
ii) COOR wherein R is a removeable carboxy protecting group, e.g., p-nitrobenzyl, o-nitrobenzyl, benzyl, or allyl;
iii) COOM wherein M is an alkali metal; or
iv) $COO^{\ominus}$ provided that when Y is other than iv) a counterion $Z^{\ominus}$ is present.

As used herein, the term "heteroatom" means nitrogen, oxygen, or sulfur, independently selected where more than one heteroatom is involved.

A preferred group of compounds of Formula I are those where L is $C_1$-$C_6$ branched or linear alkyl, both substituted and unsubstituted. The preferred substituents are OH, $CF_3$, $OC_{1-4}$alkyl, CN, $CONH_2$, CONH($C_1$-$C_4$alkyl), CON($C_1$-$C_4$alkyl)$_2$, COOH, $NH_2$, NH($C_1$-$C_4$alkyl), and N($C_1$-$C_4$alkyl)$_2$ and especially $OCH_3$, OH, $NH_2$, and $CF_3$. Examples of preferred L groups are

$-CH_2-$, $-CH(CH_3)-$, $-CH(CH_2CH_3)-$,

$-CH(CH_2OCH_3)$, $-CH_2-CH_2-$, $-CH(CH_3)-CH_2-$

$-CH(CH_2CH_3)-CH_2-$, $-C(CH_3)_2-CH_2-$,

$-(CH_2)_3-$, $-CH(CH_3)-(CH_2)_2-$, $CH_2-CH(CH_3)-$,

$-CH_2-C(CH_3)_2-CH_2-$, $-(CH_2)_4-$,

$-CH(CF_3)-CH_2-$, $-CH(CH_2OH)-CH_2-$

$-CH(CH_2NH_2)-CH_2-$, $-CH(CH_2OCH_3)-CH_2-$,

and the like.

Especially preferred compounds of Formula I compounds are those where L is $-CH_2-$, $-CH_2CH_2-$ or $-CH(CH_3)-CH_2$. Of course it is understood that where any substituent group has an asymmetric center e.g. $\begin{matrix}CH_3\\|\\-CH-CH_2-\end{matrix}$, then all stereoisomers are included as mixtures or as separate isomers.

In the preferred embodiment, the 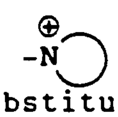 group is quaternized, monocyclic heteroaryl, substituted

and unsubstituted, containing in addition to the quaternary N, up to 2 additional hetero atoms selected from O, N and S.

Representative useful monocyclic 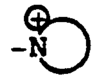 groups are substituted and unsubstituted pyridinium, pyridazinium, pyrimidinium, pyrazinium, pyrazolium, triazolium, imidiazolium, thiazolium, oxazolium, isoxazolium and the like.

Preferred Formula I compounds are those where monocyclic 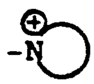 is a six membered heterocycle, such as substituted or unsubstituted pyridinium, pyridazinium or pyrazinium, and preferably substituted or unsubstituted pyridinium, wherein the substituents (one or more) are selected from OH, $NH_2$, $NHCH_3$, $OCH_3$, COO-$C_1$-$C_3$ alkyl, C(O)NHOH, phenyl, $N(CH_3)_2$, $C(O)CH_3$, $C(O)N(CH_3)OH$, $SO_3H$, $SCH_3$, CHO, COOH, $S(O)CH_3$, $SO_2CH_3$, $SO_2NH_2$, $SO_2NHCH_3$, $SO_2CH_3$, CN, $CSNH_2$, $CONH_2$, $CONH(CH_3)$, CH=N-OH, $C_1$-$C_6$ alkenyl and substituted and unsubstituted $C_1$-$C_6$ alkyl.

The preferred substituents are unsubstituted $C_1$-$C_6$ alkyl, $C_1$-$C_6$ alkenyl, and substituted $C_1$-$C_6$ alkyl wherein the substituents (one or more) are selected from OH, $NH_2$, $NHCH_3$, $OCH_3$,

-COO-$C_1$-$C_3$alkyl, C(O)NHOH, 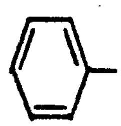 , $N(CH_3)_2$,

$C(O)CH_3$, C(O)-$N(CH_3)OH$, $SO_3H$, $SCH_3$, CHO, COOH, $S(O)CH_3$, $SO_2CH_3$, $SO_2NH_2$, $SO_2NHCH_3$, CN, $CSNH_2$, CH=N-OH, $CONH_2$, $CONH(CH_3)$.

Of particular interest and the most preferred group are compounds of the present invention wherein the substituent on the N-containing mono- or bicyclic quaternary heteroaryl group in the 2-position is an acidic function as defined above and the Y substituent in the 3-position is $COO^{\ominus}$ as defined above, thus forming a zwitterion with the positive charge of the quaternary nitrogen. The acidic function is anionic and the compounds are thus anionic zwitterions, i.e., they have a net negative charge. This novel characteristic has been found to result in at least one surprising and important improvement in the biological properties of the compounds reduced CNS side-effects. A more particular group of the compounds, those wherein the acidic function is a sulfoalkyl group of the formula $(C_{1-4}\ alkyl)SO_3^{\ominus}$, have been found to have the additional surprising and important biological property of enhanced potency against Pseudomonas species, an especially important nosocomial pathogen. In this most preferred group of compounds, it is preferred that the N-containing mono- or bicyclic quaternary heteroaryl group in the 2-position is pyridinium.

Representative examples of preferred

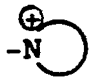 pyridinium groups are those having the formulae

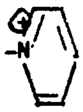 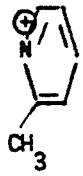 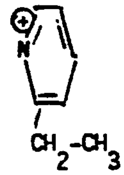 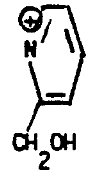

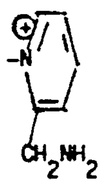 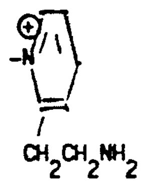 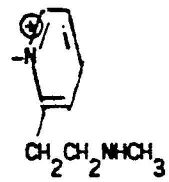 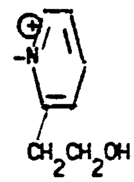

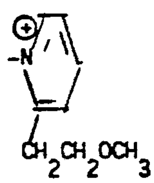 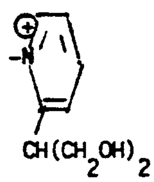 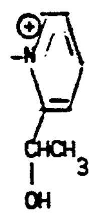 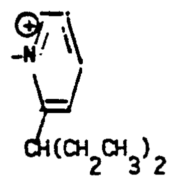

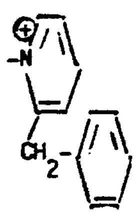 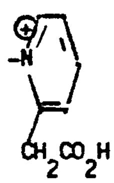 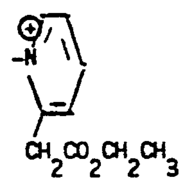 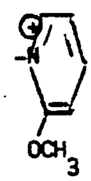

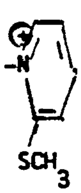 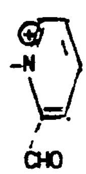 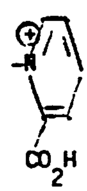 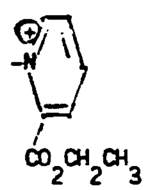

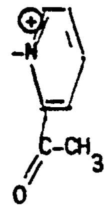 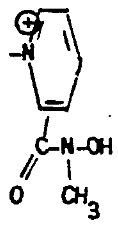 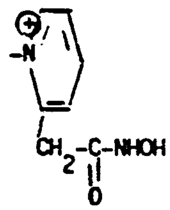 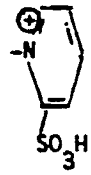

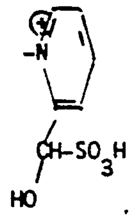 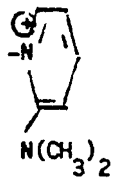 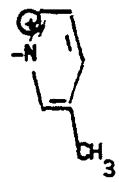 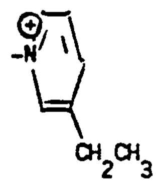

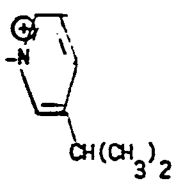 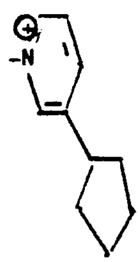 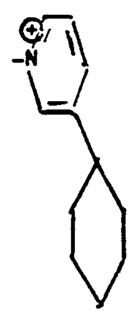 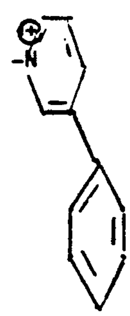

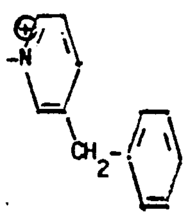 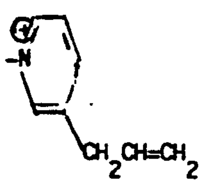 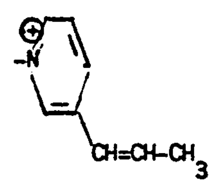 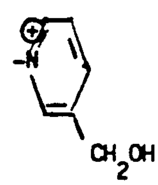

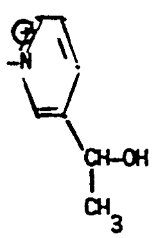 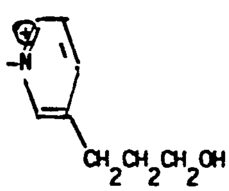 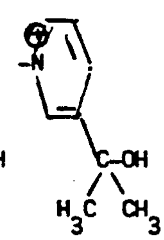 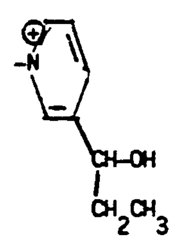

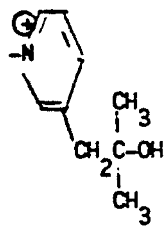 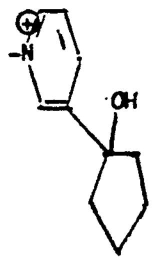 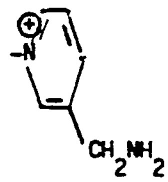 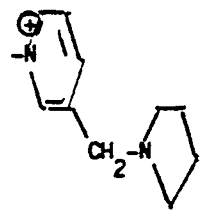

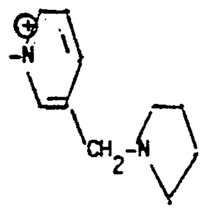 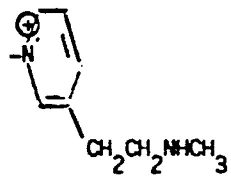 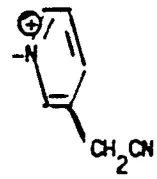 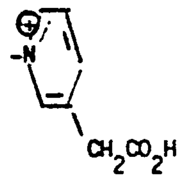

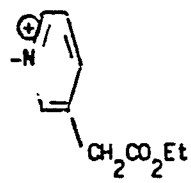 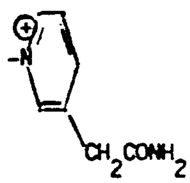 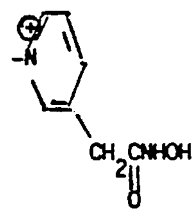 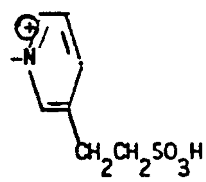

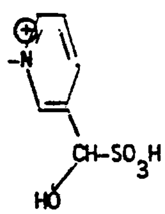 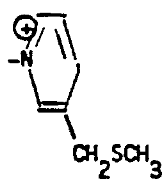 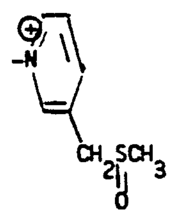 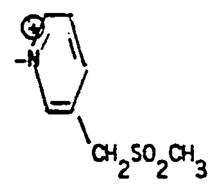

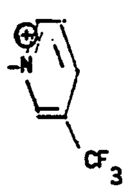 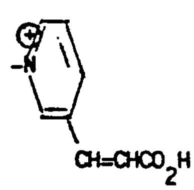 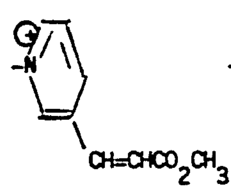 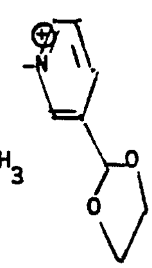

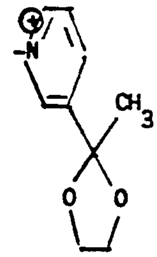 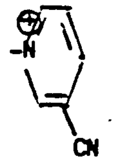 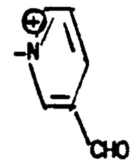 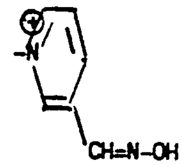

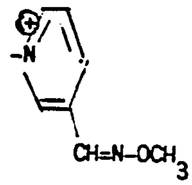 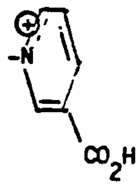 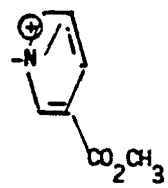 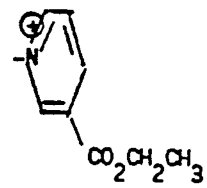

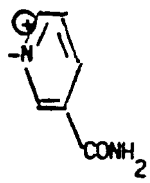 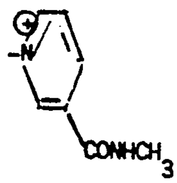 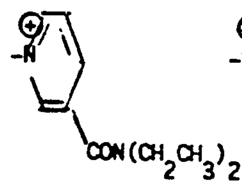 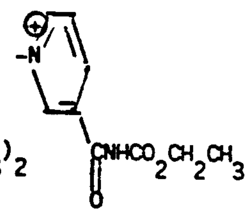

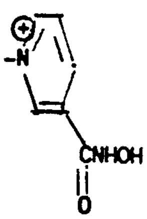 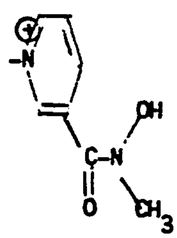 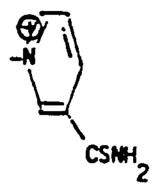 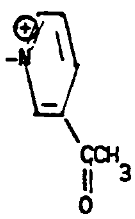

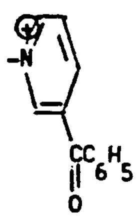 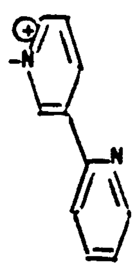 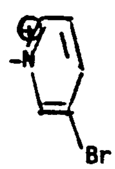 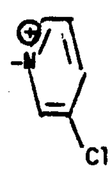

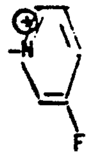
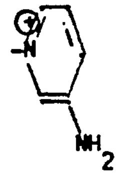
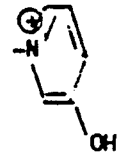
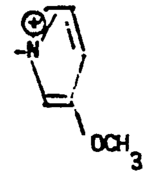

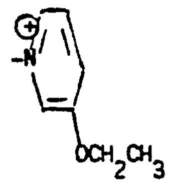
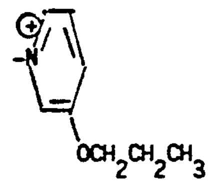
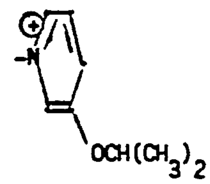
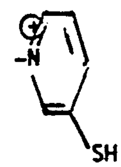

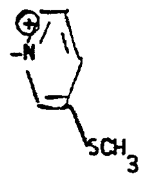
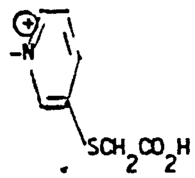
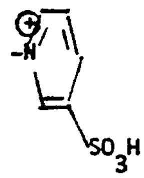
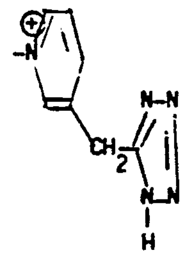

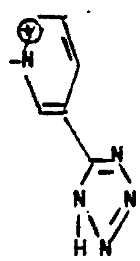
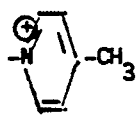
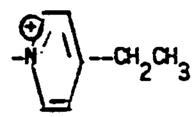
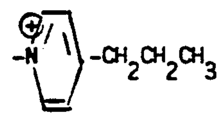

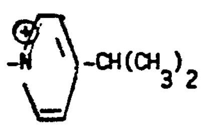
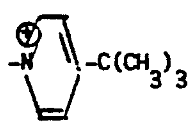
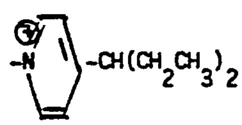
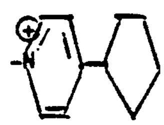

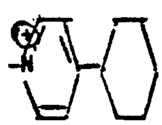
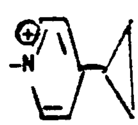
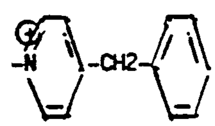
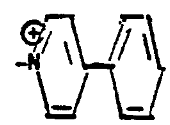

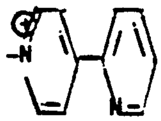 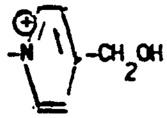 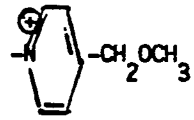 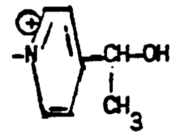

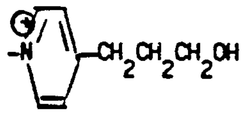 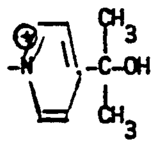 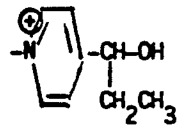 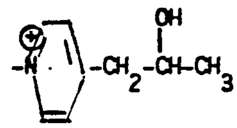

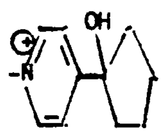 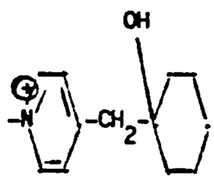 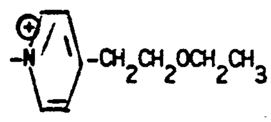 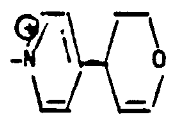

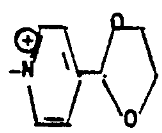 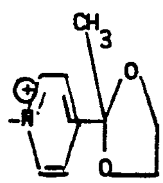 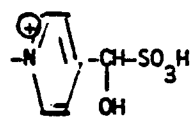 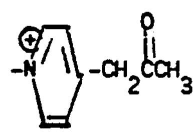

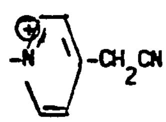 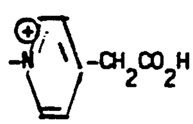 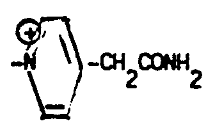 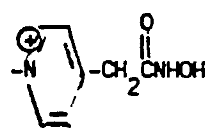

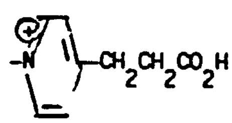 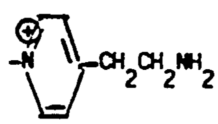 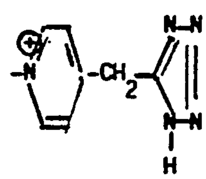 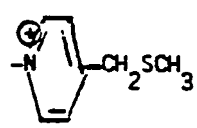

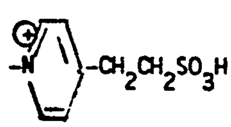 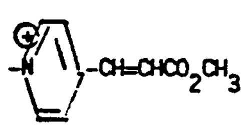 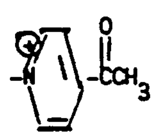 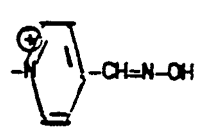

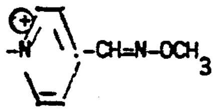 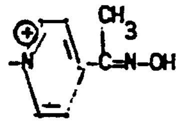 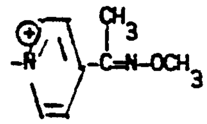 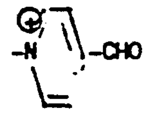

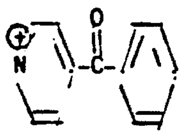 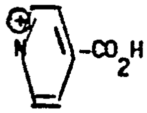 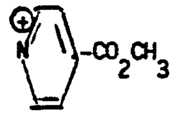 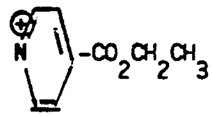

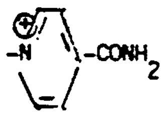 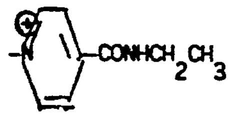 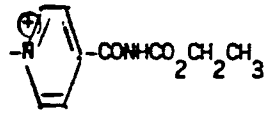 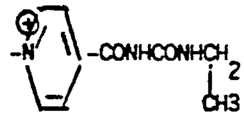

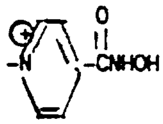 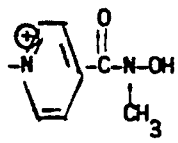 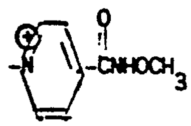 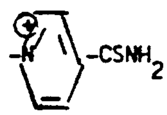

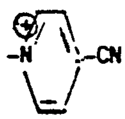 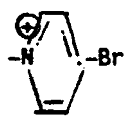 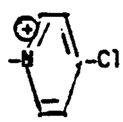 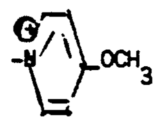

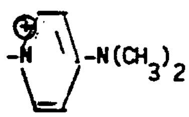 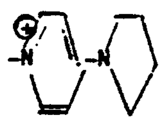 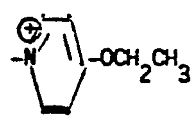

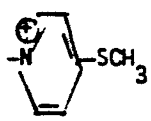 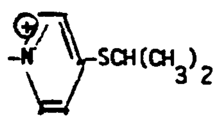 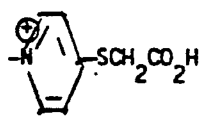 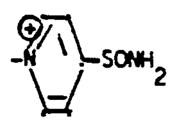

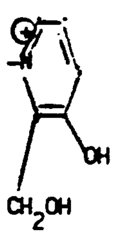 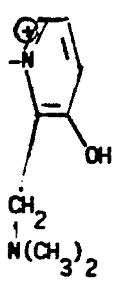 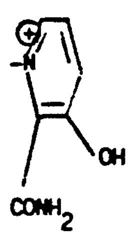 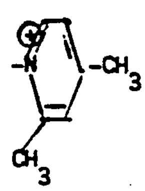

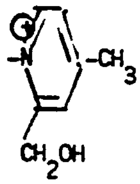
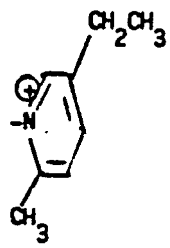
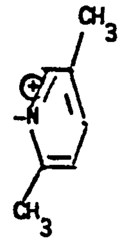
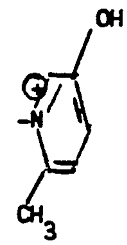

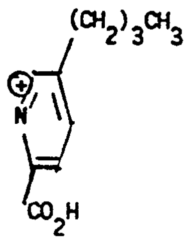
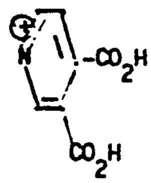
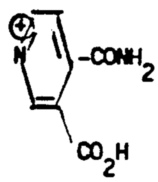
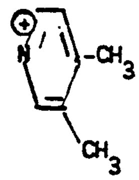

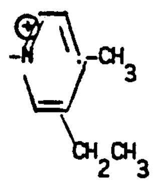
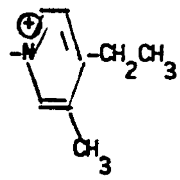
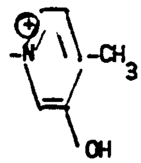
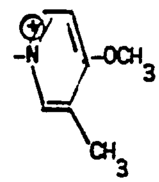

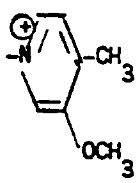
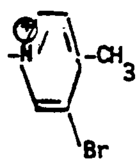
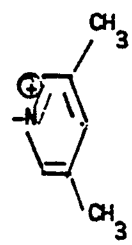
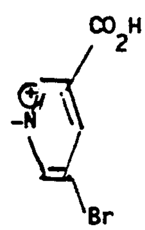

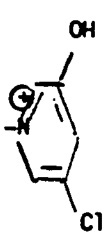
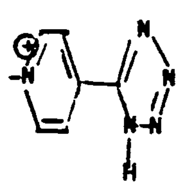
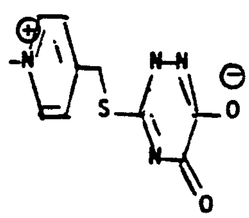

Representative examples of preferred monocyclic 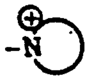 other then pyridinium are those having the following formulae:

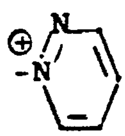
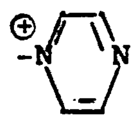
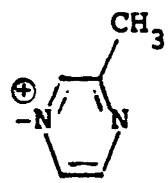
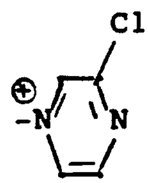
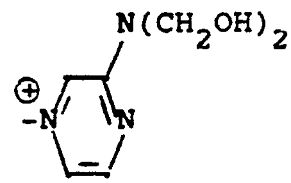

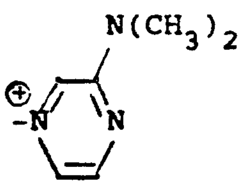
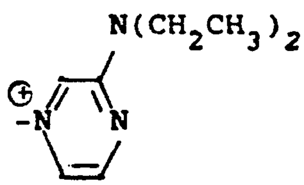
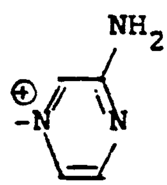
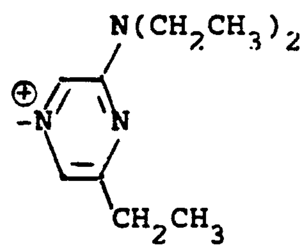

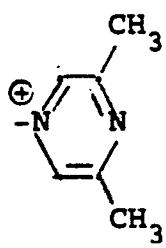
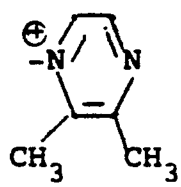
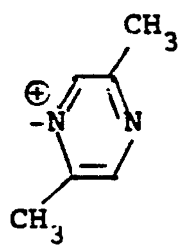
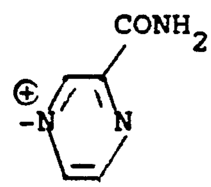

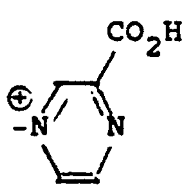
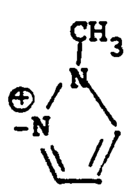
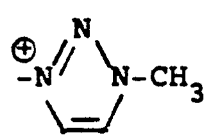
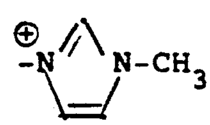

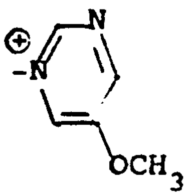 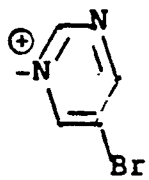 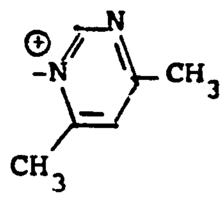 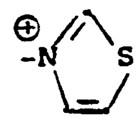 and

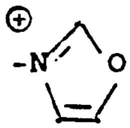

In another preferred embodiment, the 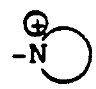 group is a quaternized, bicyclic, substituted or unsubstituted heteroaryl, containing in addition to the quaternary N, up to 4 additional heteroatoms independently selected from O, N and S, and 9-10 total ring atoms.

Representative useful 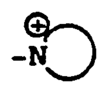 groups are substituted and unsubstituted quinolinium, isoquinolinium, quinoxalinium, isocinolinium, thienopyridinium, furopyridinium, naphthyridinium, pyrazinopyridinium, 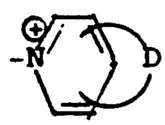 where D is a $C_{2-6}$ alkylene ring which may be interrupted by one or more O, S or N heteroatoms.

Preferred Formula I compounds are those where 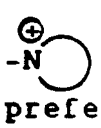 is a bicyclic 9 or 10 membered ring, and more preferably substituted or unsubstituted quinolinium, isoquinolinium, or thienopyridinium.

The preferred substituents on the bicyclic heteroaryl groups are OH, $C_1$-$C_3$alkyl, $NH_2$, $CH=NOCH_3$, $CF_3$, halo, preferably Br or Cl, O-$C_1$-$C_3$alkyl, COOH, CHO, $SO_3H$, $CONH_2$, $SO_2NH_2$, $N(C_1$-$C_3$alkyl$)_2$, $CH_2CO_2H$, $CH_2OH$, 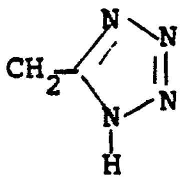 , 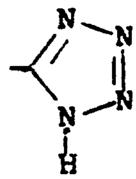 , $CH_2SO_3H$, CN, $CONH_2$, $CH_2CN$, $CH_2CONH_2$, $CH_2N(C_1$-$C_3$alkyl$)_2$ and the like.

Representative examples of useful bicyclic

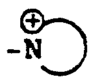 groups are:

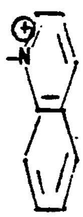
quinolinium

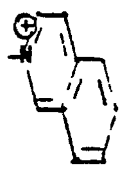
isoquinolinium

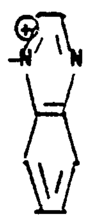
quinoxalinium

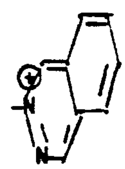
isocinolinium

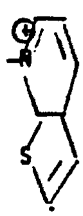
thieno[2,3-b]pyridinium

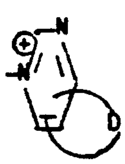

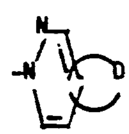

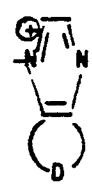

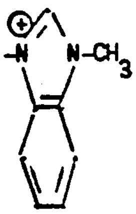
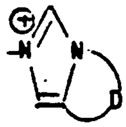
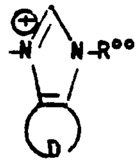
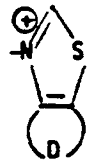
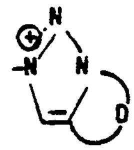

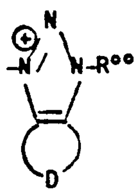
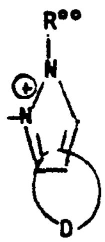
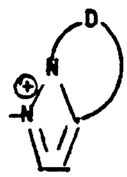

where $R^{\circ\circ}$ is unsubstituted or substituted $C_1$-$C_4$ alkyl and D is $C_3$-$C_5$ alkylene.

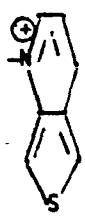
thieno[3,4-b] pyridinium

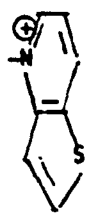
thieno[3,2-b] pyridinium

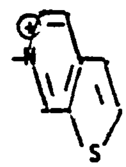
thieno[2,3-c] pyridinium

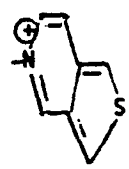
thieno[3,4-c] pyridinium

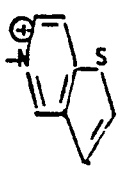
thieno[3,2-c] pyridinium

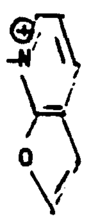
furo[2,3-b] pyridinium

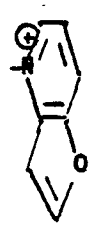
furo[3,2-b] pyridinium

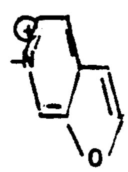
furo[2,3-c] pyridinium

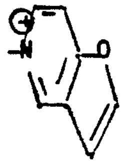 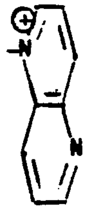 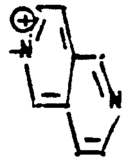 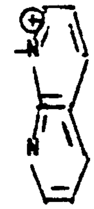

1-7,napthyridinium

furo[3,2-c] pyridinium

1,5-napthyridinium

1,8-napthyridinium

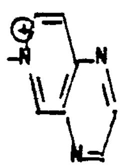 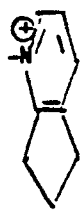 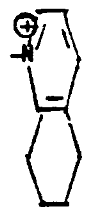 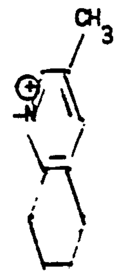

pyrazino[2,3-c] pyridinium

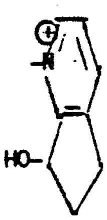 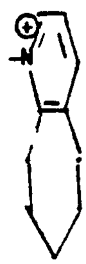 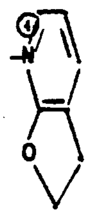 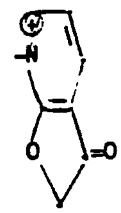

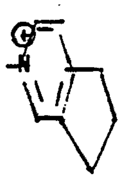 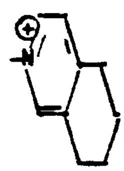 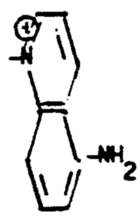 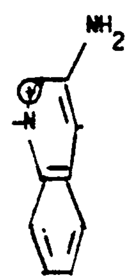

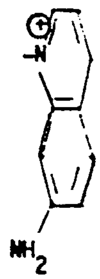
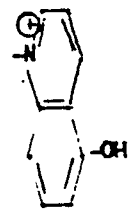
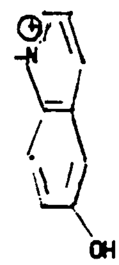
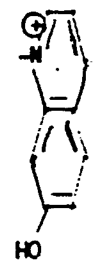

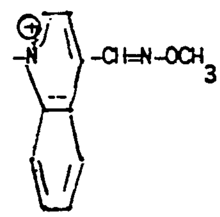
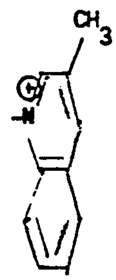
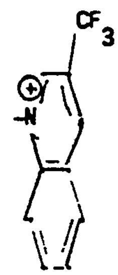

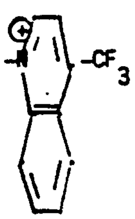
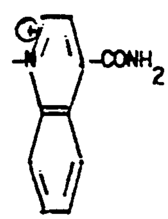
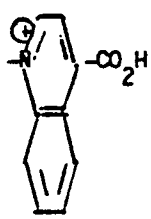
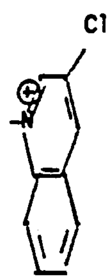

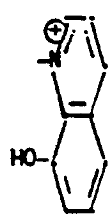
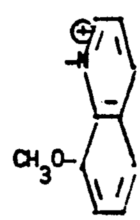
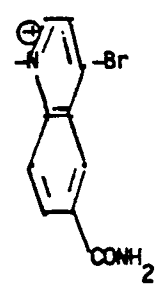
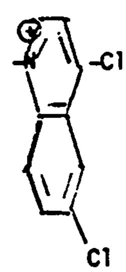

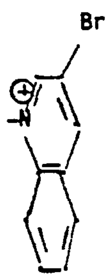 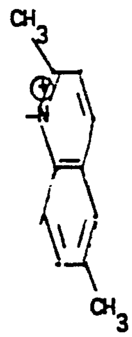 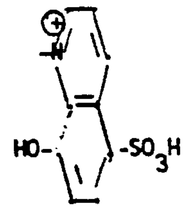 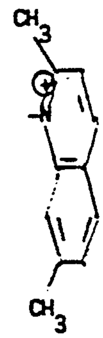

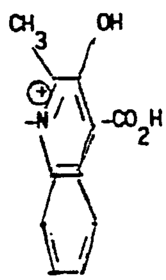 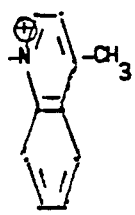 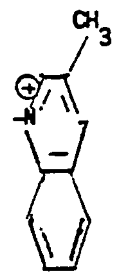 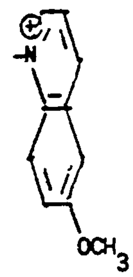

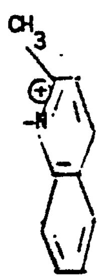 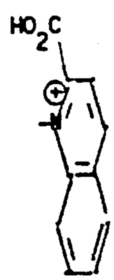 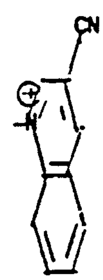 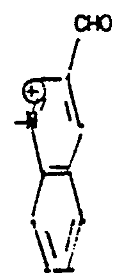

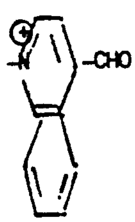 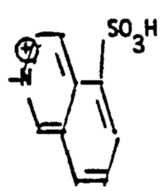 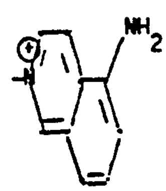 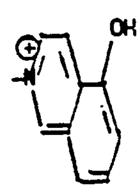

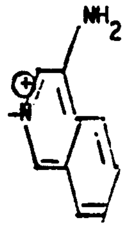
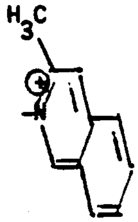
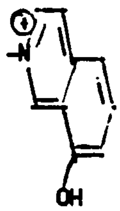
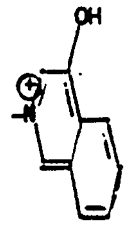

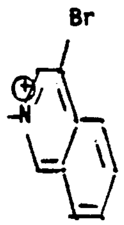
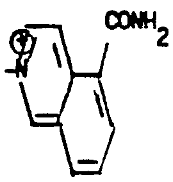
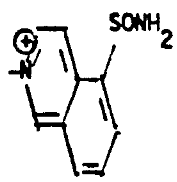
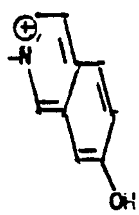

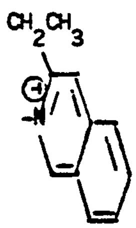
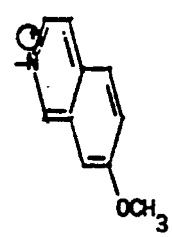
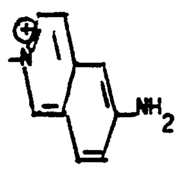
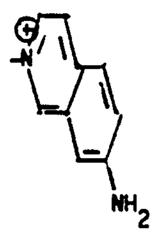

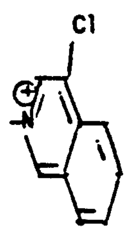
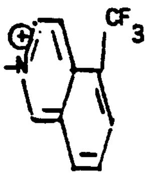
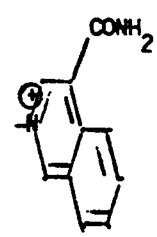
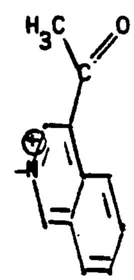

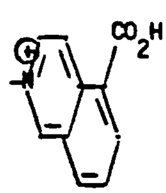
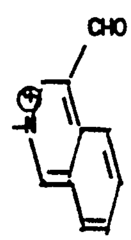
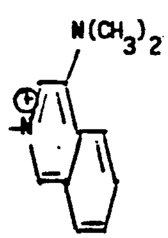
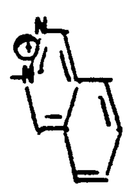
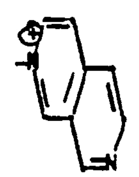

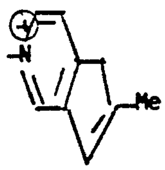
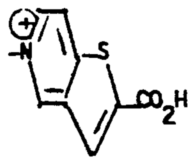
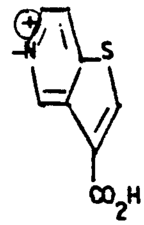
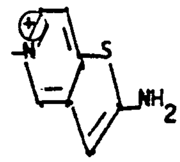
and
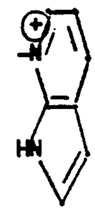

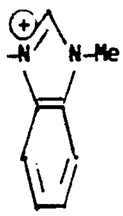
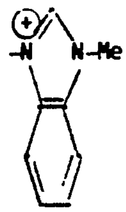
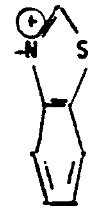
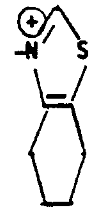
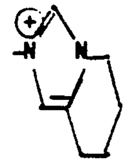

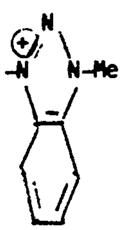
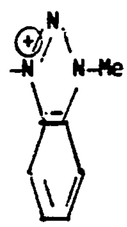
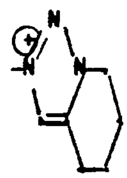
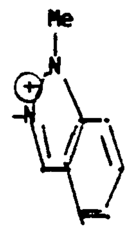
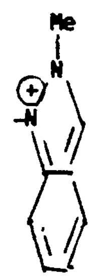

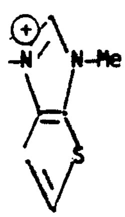
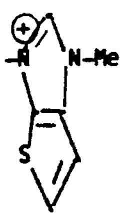
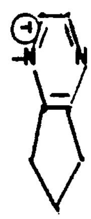
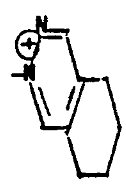
and
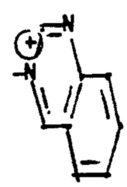

Where 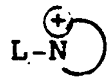 is 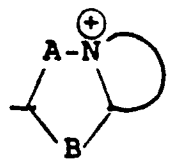 , representative examples of preferred groups are:

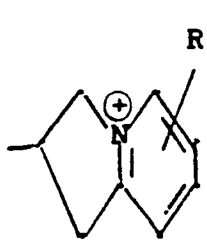
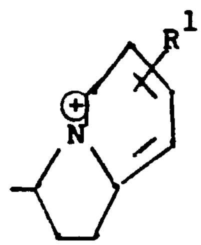
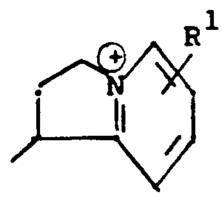

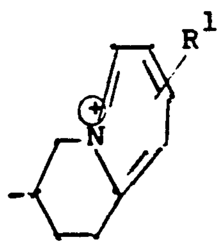
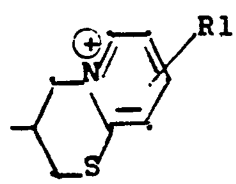
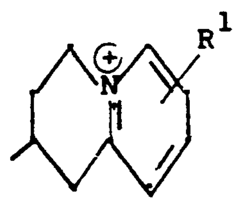

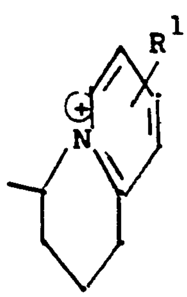
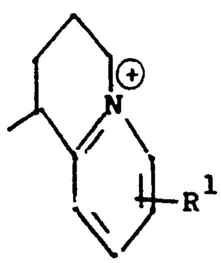
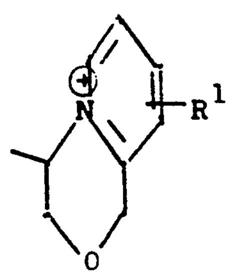
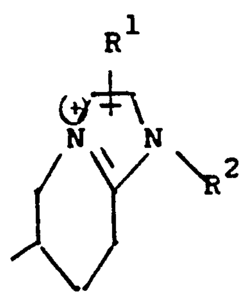

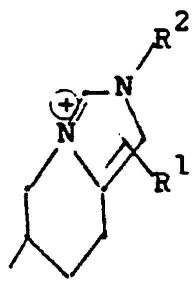 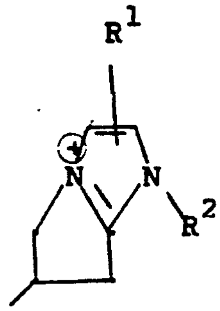 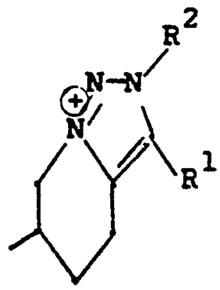

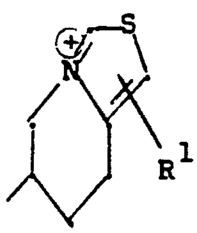 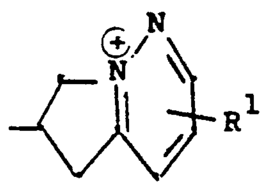 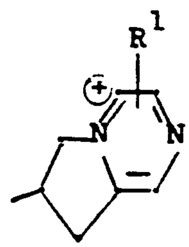 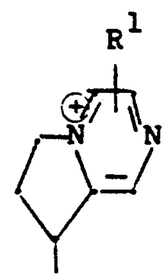

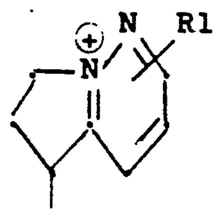 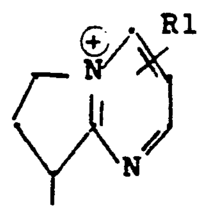 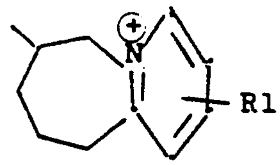

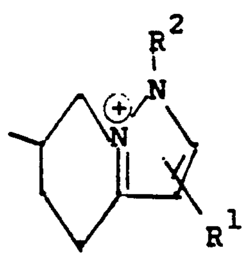 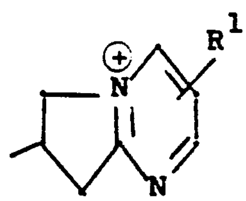

wherein $R^1$ is H or a suitable substituent such as $C_1$-$C_3$alkyl, CN, $CO_2H$, $SO_3H$, 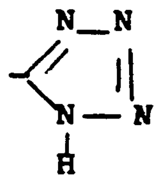 $CONH_2$, $NH_2$, NH($C_1$-$C_3$alkyl), N($C_1$-$C_3$alkyl)$_2$, OH, O($C_1$-$C_4$alkyl), $SO_2NH_2$, $CH_2OH$, $CH_2N$($C_1$-$C_3$alkyl)$_2$, $CH_2CO_2H$, $CH_2CN$, $CH_2CONH_2$, halo, $CH_2$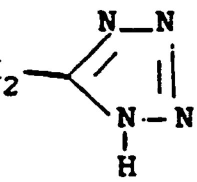, and

$R^2$ substituted or unsubstituted $C_1$-$C_4$alkyl wherein the substituents are selected from OH, N($C_1$-$C_3$alkyl)$_2$, $CO_2H$, $SO_3H$, CN, $CONH_2$, and O($C_1$-$C_4$alkyl).

Expecially preferred 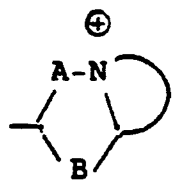 groups are the unsubstituted groups especially where a) A is $(CH_2)_2$ or $(CH_2)_3$, and B is a single bond, b) A is $CH_2$ and B is $CH_2$ or $(CH_2)_2$, or c) A is a single bond and B is $(CH_2)_{2-3}$, and the heteroaryl moiety is preferably pyridinium, thiazolium, or imidazolium.

The compounds of Formula I include inner (Zwitterion) salts when Y is $COO^{\ominus}$ e.g.

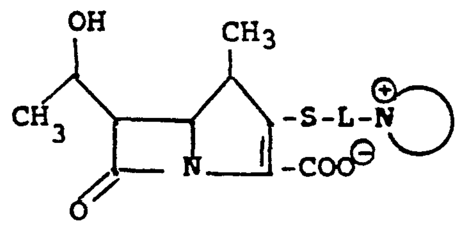

or, when Y is other than $COO^{\ominus}$, salts with an external, physiologically acceptable counterion $Z^{\ominus}$ e.g.

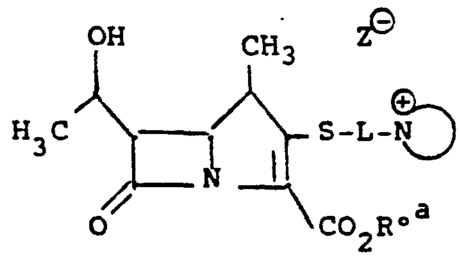

$R°^a$ is a pharmaceutically acceptable ester, e.g., pivaloyloxymethyl, phthalidyl, phthalimidomethyl, acetoxymethyl, ethoxycarbonyloxyethyl, pivaloyloxyethyl, 4-methyl-2-oxo-1,3-dioxolen-5-yl-methyl or salt group; and $Z^{\ominus}$ is $Cl^{\ominus}$, $Br^{\ominus}$, $I^{\ominus}$, $OH^{\ominus}$, $HCO_3^{\ominus}$, $CH_3CO_2^{\ominus}$ and the like. The inner salts are preferred.

Again, the compounds of Formula I include the stereoisomers as mixtures and as separate isomers.

Compounds having the (1R,5S,6S,8R) stereochemistry shown below are preferred

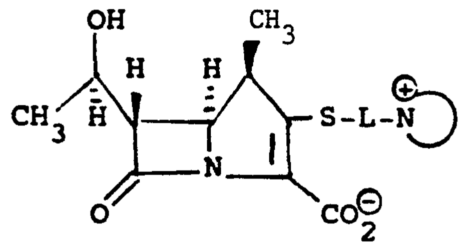

Where 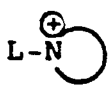 is 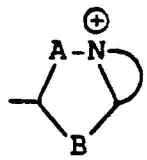 or when L contains a chiral center, the side chain chirality leads to diastereomeric products. The products can be separated by conventional methods, used as mixtures or synthesized stereospecifically from optically active mercaptans. Representative zwitterionic structures are shown below:

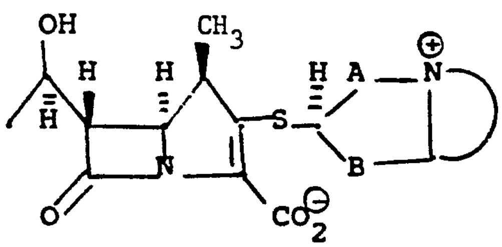

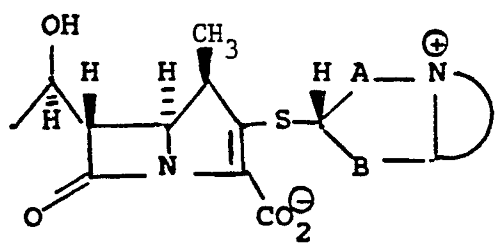

The compounds of the present invention (I) are valuable antibiotics active against various Gram-positive and Gram-negative bacteria and accordingly find utility in human and veterinary medicine. Representative pathogens which are sensitive to antibiotics I include: <u>Staphylococcus</u>

aureus, Escherichia coli, Klebsiella pneumoniae, Bacillus subtilis, Salmonella typhosa, Psuedomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The products of this invention may be used in any of a variety of pharmaceutical preparations. They may be employed in capsule, powder form, in liquid solution, or in suspension. They may be administered by a variety of means; those of principal interest include: topically or parenterally by injection (intravenously or intramuscularly).

Compositions for injection, a preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a

suitable vehicle, such as sterile water. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg. of body weight of the subject in one or more treatments per day. A preferred daily dosage for adult humans lies in the range of from about 10 to 240 mg. of active ingredient per kg. of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will generally contain from about 15 mg. to about 1500 mg. of the active ingredient; however, in general, it is preferable to employ a dosage amount in the range of from about 250 mg to 1000 mg. In parenteral administration, the unit dosage is usually the pure

compound I in sterile water solution or in the form of a soluble powder intended for solution.

The preferred method of administration of the formula I antibiotic is parenteral by i.v. infusion, i.v. bolus, or i.m. injection.

For adults, 5-50 mg of Formula I antibiotic per kg of body weight given 2, 3, or 4 times per day is preferred. Preferred dosage is 250 mg to 1000 mg of the Formula I antibiotic given two (b.i.d.) three (t.i.d.) or four (q.i.d.) times per day. More specifically, for mild infections, and particularly urinary tract infections, a dose of 250 mg t.i.d. or q.i.d. is recommended. For moderate infections against highly susceptible gram positive and gram negative organisms, a dose of 500 mg t.i.d. or q.i.d. is recommended. For severe, life-threatening infections against organisms at the upper limits of sensitivity to the antibiotic, a dose of 1000 t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibiotic compounds of Formula I are of the broad class known as carbapenems or 1-carbadethiapenems. Certain of these carbapenems are susceptible to attack by a renal enzyme known as dehydropeptidase (DHP). This attack or degradation may reduce the efficacy of the carbapenem antibiotic. Inhibitors of DHP and their use with carbapenem antibiotics are disclosed in the prior art [see published European Patent Applications No. 79102616.4 filed July 24, 1979 (patent no. 10573); 79102615.6,

filed July 24, 1979 (application no. 15573); and No. 82107174.3, filed August 9, 1980 (application no. 72014)].

The present I compounds may, where DHP inhibition is desired or necessary, be combined or used with the appropriate DHP inhibitor as described in the aforesaid published applications. Thus, to the extent that the cited European patent applications 1.) define the procedure for determining DHP susceptibility of the present carbapenems and 2.) disclose suitable inhibitors, combination compositions and methods of treatment, they are incorporated herein by reference. A preferred weight ratio of I compound:DHP inhibitor in the combination compositions is about 1:1. A preferred DHP inhibitor is 7-(L-2-amino-2-carboxyethylthio)-2-(2,2-dimethylcyclopropanecarboxamide)-2-heptenoic acid or a useful salt thereof.

These combination compositions and their use is another embodiment of the present invention.

The compounds of Formula I may be prepared by any convenient process.

One such process is illustrated of the following reaction equations:

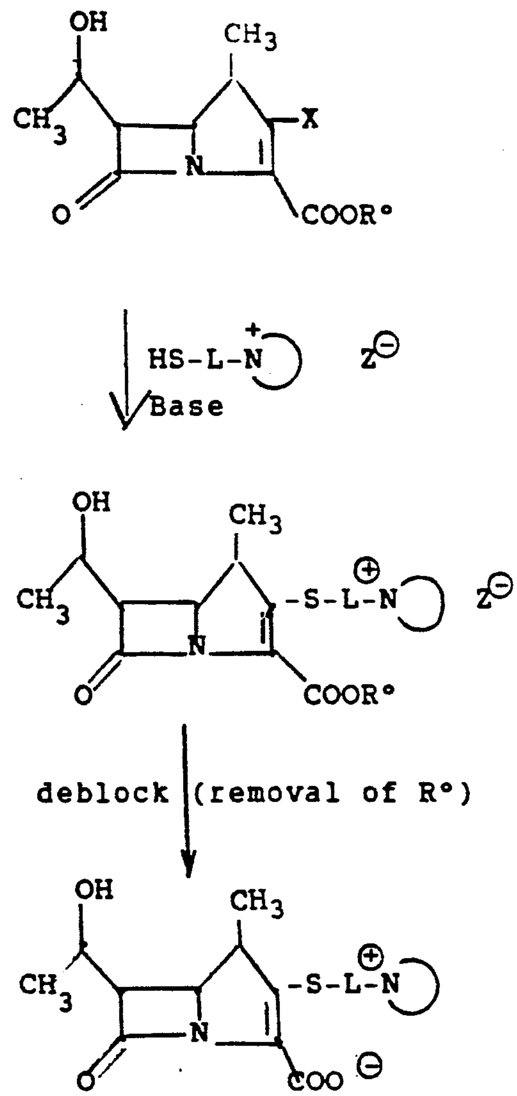


where R° is other than (-) or H and is a readily removable blocking group e.g. p-$NO_2$benzyl or allyl. X is a leaving group such as $OP(O)(OØ)_2$,

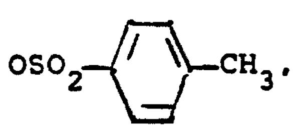

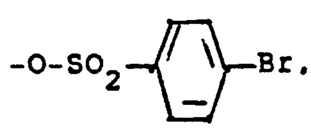

$OSO_2CF_3$, S(O)R'' where R'' is $CH_3$, $CH_2CH_3$, $CH_2CH_2NHC(O)CH_3$ and the like and $Z^{\ominus}$ is an anion such as $Cl^{\ominus}$, $Br^{\ominus}$, $I^{\ominus}$, $CH_3CO_2^{\ominus}$, $CH_3SO_3^{\ominus}$, $CF_3SO_3^{\ominus}$.

The process conditions are generally those available in the prior art. Thus, the side chain addition reaction is carried out in a solvent such as acetonitrile, dimethylformamide, dimethylacetamide, or N-ethylpyrrolidinone in the presence of a base such as N,N-diisopropylethylamine, triethylamine, or 4-dimethylaminopyridine at a temperature of from -40°C to 25°C for a period of 5 minutes to 10 hours. The deblocking reaction wherein R° is p-nitrobenzyl is usually conducted in an aqueous system containing cosolvents such as tetrahydrofuran, ethanol, n-butanol, 2-amyl alcohol, or ethyl acetate and a pH 6.8-7.0 aqueous buffer. Suitable buffers include phosphate buffers and buffers derived from non-nucleophilic amines such as N-methylmorpholine or morpholinopropane sulfonic acid. The reaction is conducted at 0°C to 40°C for 0.5 to 5 hours under 1-100 atmospheres of hydrogen in the presence of a catalyst such as 10% palladium on carbon or 20% palladium hydroxide on carbon. The final products are purified by ion exchange chromatography and/or reverse phase chromatography. When a pharmaceutically acceptable ester of the final product is desired, the deblocking step is omitted and the appropriate R° group is incorporated into the starting material.

A second process for preparing Formula I compounds is illustrated by the following reaction equations:

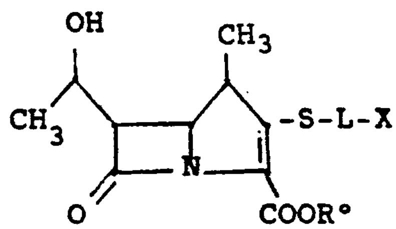

where X is a leaving group such as Cl, Br, I, $OSO_2CH_3$, 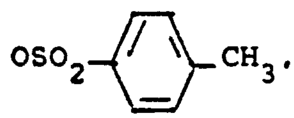 $-OSO_2CF_3$ and the like, and R° is as defined above

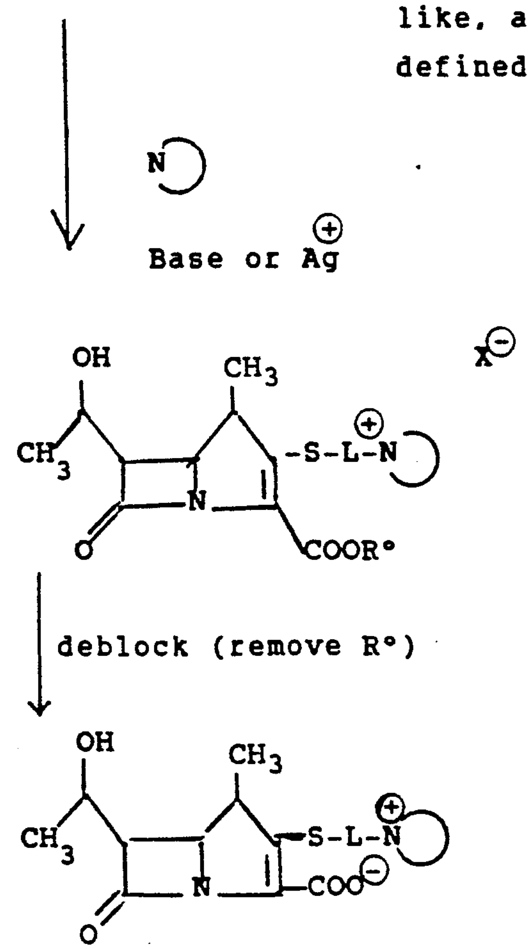

Again, the process conditions are those available in the prior art.

A third process for preparing Formula I compounds is illustrated by the following equations:

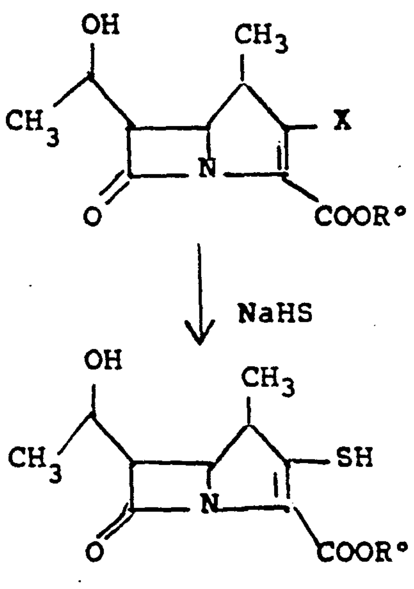


where R°, X and Z are as previously defined and X' is a leaving group such as Cl, Br, I, $OSO_2CH_3$, $OSO_2$-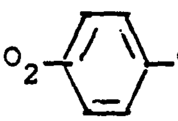-$CH_3$, $OSO_2CF_3$ and the like.

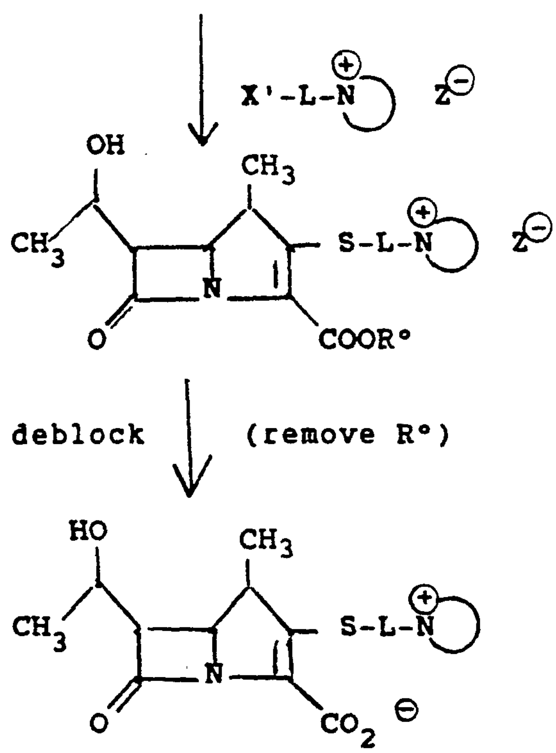

EXAMPLE 1

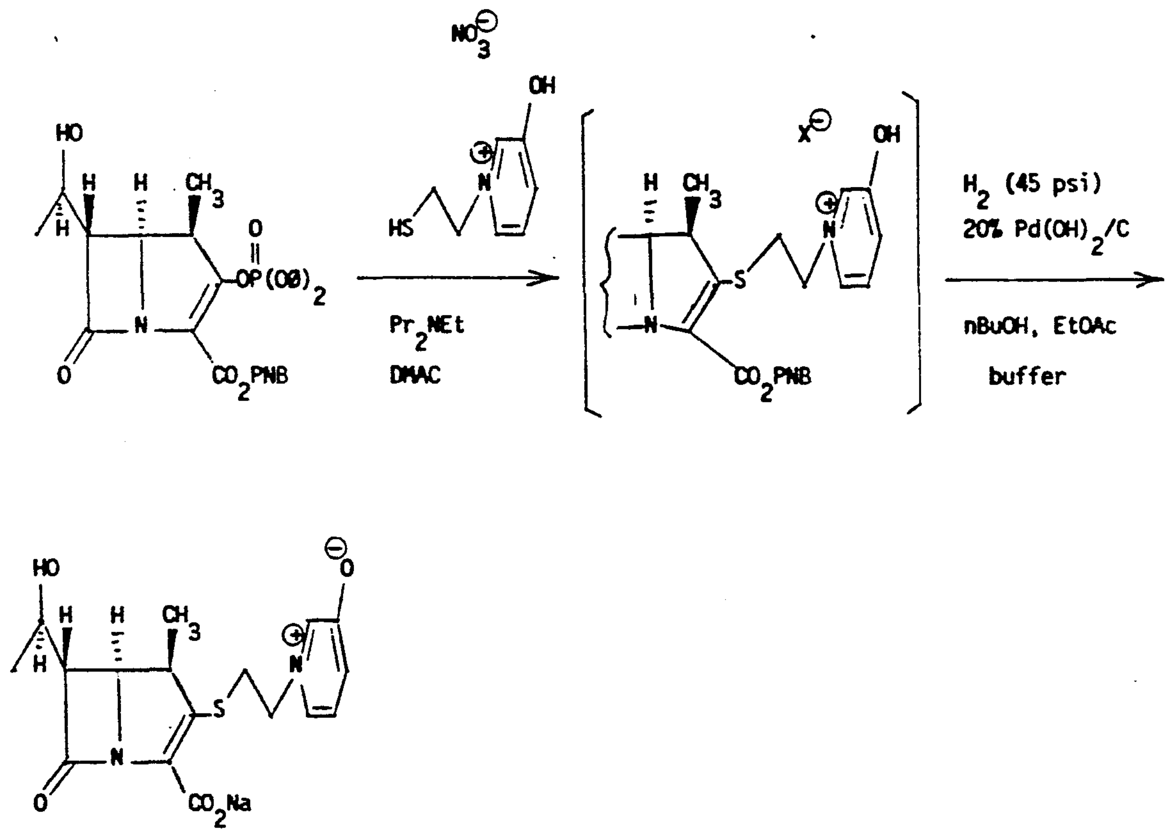


Sodium (5S,6S)-6-[1(R)-hydroxyethyl]-1(R)-methyl-2-[2-(3-oxidopyridinium)ethylthio]-carbapen-2-em-3-carboxylate

A solution of p-nitrobenzyl (5S,6S)-2-(diphenylphosphono)oxy-6[1(R)-hydroxyethyl]-1(R)-methyl-carbapen-2-em-3-carboxylate (60 mg, 0.1 mmol) and crude 3-hydroxy-1-(mercaptoethyl)pyridinium nitrate (33 mg, ca. 0.15 mmol) in anhydrous dimethylacetamide (0.7 ml) was cooled to ca. -20°C and treated with N,N-diisopropylethylamine (0.026 ml, 0.15 mmol). After stirring at -15° to -20°C for 50

minutes, the reaction mixture was diluted with n-butanol (5 ml), ethyl acetate (2.5 ml), water (5 ml) and 0.5M pH 6.8 N-methylmorpholine hydrochloric acid buffer, treated with 20% palladium hydroxide on carbon, and hydrogenated on a Parr shaker at 45 psi for 60 minutes. The catalyst was removed by filtration through a celite pad, and the filtrate was washed with methylene chloride. The aqueous phase was concentrated under vacuum to ca. 2 ml and charged onto a column of Dowex 50W-X4 (sodium form, 200-400 mesh, 1.5 X 30 cm). The column was eluted with water in a cold room at 10 ml fractions/4 minutes. Fraction 3, which contained most of the product by UV, was lyophilized to an off-white powder. This material was chromatographed on a 0.5 mm X 20 X 20 cm Analtech RPS-F plate using 5% ethanol in water as developing solvent in a cold room. The major UV visible band at $R_f$ 0.2-0.4 was removed and eluted with 4:1 acetonitrile-water. The eluant was diluted with water, washed with petroleum ether, condensed under vacuum to ca. 5 ml volume, passed through a Gelman CR acrodisc, and lyophilized to afford the title compound (12.7 mg) as an amorphous, white powder.

IR (Nujol) $\nu$ max 3300 (br), 1747, 1592, 1567, 1518 $cm^{-1}$;
UV (0.05M pH 7.0 buffer) $\lambda$max 300 nm ($\epsilon$ 8000);
UV (buffer + $NH_2OH.HCl$) $\lambda$max 322 nm ($\epsilon$ 3700) and extinguished $\lambda$max 296 nm ($\epsilon$ 6400);
NMR ($D_2O$) $\delta$ 1.09 (d, J=7.2 Hz, $C\underline{H}_3CH$), 1.27 (d, J=6.4 Hz, $C\underline{H}_3CHOH$), 3.14 (dd, J=7.2 and 9.2 Hz, $CH_3C\underline{H}$), 3.23 (m, SCHa$\underline{H}$b), 3.36 (dd, J=2.6 and 6.2 Hz, H6), 3.51 (m, SC$\underline{H}$aHb), 3.86 (dd, J=2.6 and 9.2 Hz, H5), 4.21 (p, J~6.4 Hz, $CH_3\underline{CH}OH$), 4.45 (m, $CH_2N$), 7.23 (m, ArH), 7.42 (m, ArH, 7.50 (m, ArH), 7.79 (m, ArH).

EXAMPLE 2

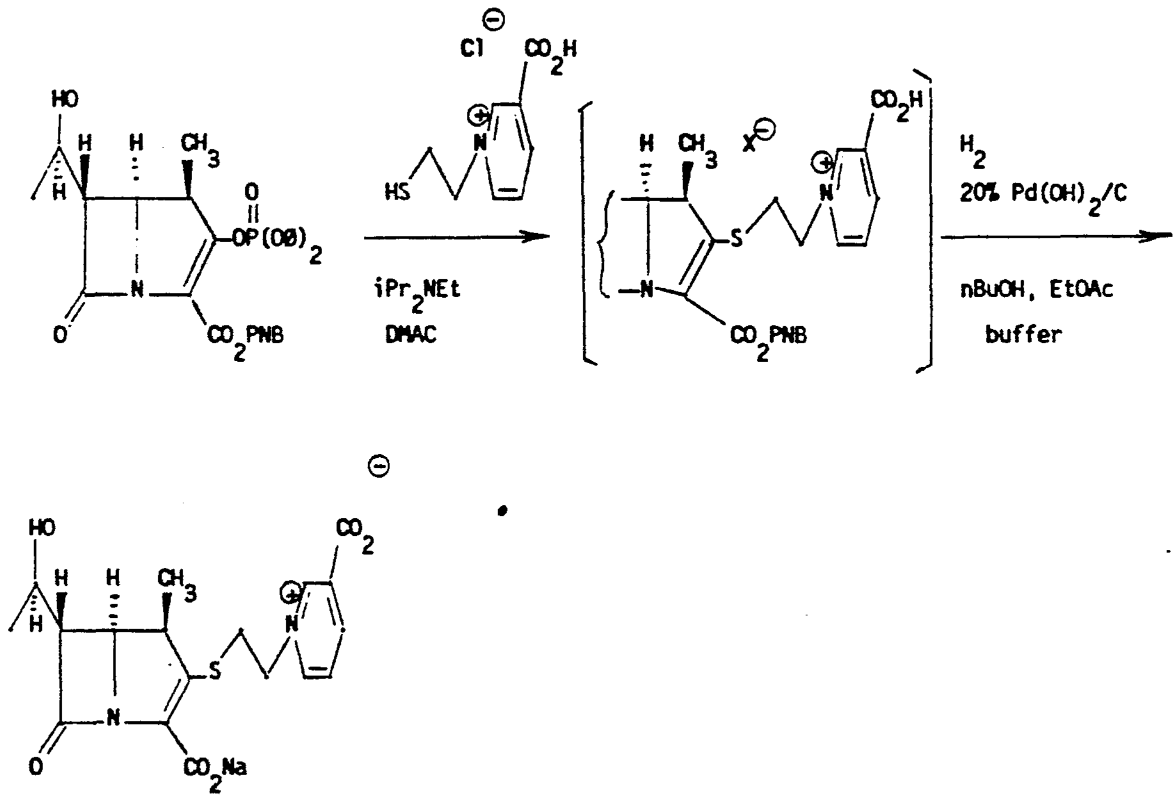


Sodium (5S,6S)-2-[2-(3-carboxylatopyridinium)ethyl]thio-6-[1(R)-hydroxyethyl]-1(R)-methylcarbapen-2-em-3-carboxylate

An ice-cold, stirring suspension of 3-carboxy-1-(2-mercaptoethyl)-pyridinium chloride (20 mg, 0.093 mmol) and p-nitrobenzyl (5S,6S)-2-(diphenylphosphono)oxy-6-[1(R)-hydroxyethyl]-1(R)-methylcarbapen-2-em-3-carboxylate (50 mg, 0.084 mmol) in anhydrous N,N-dimethylacetamide (0.5 ml) was treated with N,N-diisopropylethylamine (0.033 ml, 0.188 mmol) and stirred at ca. 0°C for 35 minutes. The mixture was diluted with n-butanol (1.7 ml), ethyl acetate

(0.8 ml), water (1.7 ml) and 0.5 M pH 6.8 N-methylmorpholine-hydrochloric acid buffer (0.8 ml), treated with 20% palladium hydroxide on carbon (25 mg) and hydrogenated at 42 psi for 1 hour on a Parr shaker. The mixture was centrifuged to remove the catalyst. The supernatant was washed with methylene chloride, concentrated under vacuum, and chromatographed on Dowex 50W-X4 (sodium form, 200-400 mesh, 1.5 X 25 cm) using water as eluant in a cold room. The product containing fractions were located by UV, concentrated under vacuum, and chromatographed on three 0.5 mm X 20 X 20 cm Analtech RPS-F plates that were developed with 2% ethanol in water in a cold room. The UV visible band at Rf 0.57-0.63 was removed and extracted with 4:1 acetonitrile-water (100 ml). The extracts were washed with hexane, concentrated under vacuum to ca. 10 ml, filtered through a Gelman CR acrodisc, and lyophilized to yield the title compound (21.9 mg) as a yellow solid. IR (Nujol) $\nu$max 3300 (br.), 1755, 1645, 1610 $cm^{-1}$; UV ($H_2O$) $\lambda$max 294 nm ($\epsilon$ 7,440); ($H_2O$ + $NH_2OH$) extinguished $\lambda$max 296 nm ($\epsilon$6,470); NMR ($D_2O$) $\delta$ 1.12 (d, J=7.2 Hz, $CHC\underline{H}_3$), 1.27 (d, J=6.3 Hz, $C\underline{H}_3CHOH$), 3.21 (qd. J=7.2 and 9.5 Hz, H1), 3.34 (td, J=5.5 and 15.5 Hz, SC$\underline{Ha}$Hb), 3.39 (dd, J=2.6 and 6.4 Hz, H6), 3.59 (ddd, J=5.0, 8.0, and 15.5 Hz, SCHa$\underline{Hb}$), 3.85 (dd, J=2.6 and 9.5 Hz, H5), 4.20 ($\sim$p, J=6 Hz, $CH_3C\underline{H}OH$), 8.07 (dd, J=6.1 and 8.1 Hz, ArH), 8.86-8.95 (m, 2 ArH), 9.27 (s, ArH).

## EXAMPLE 3

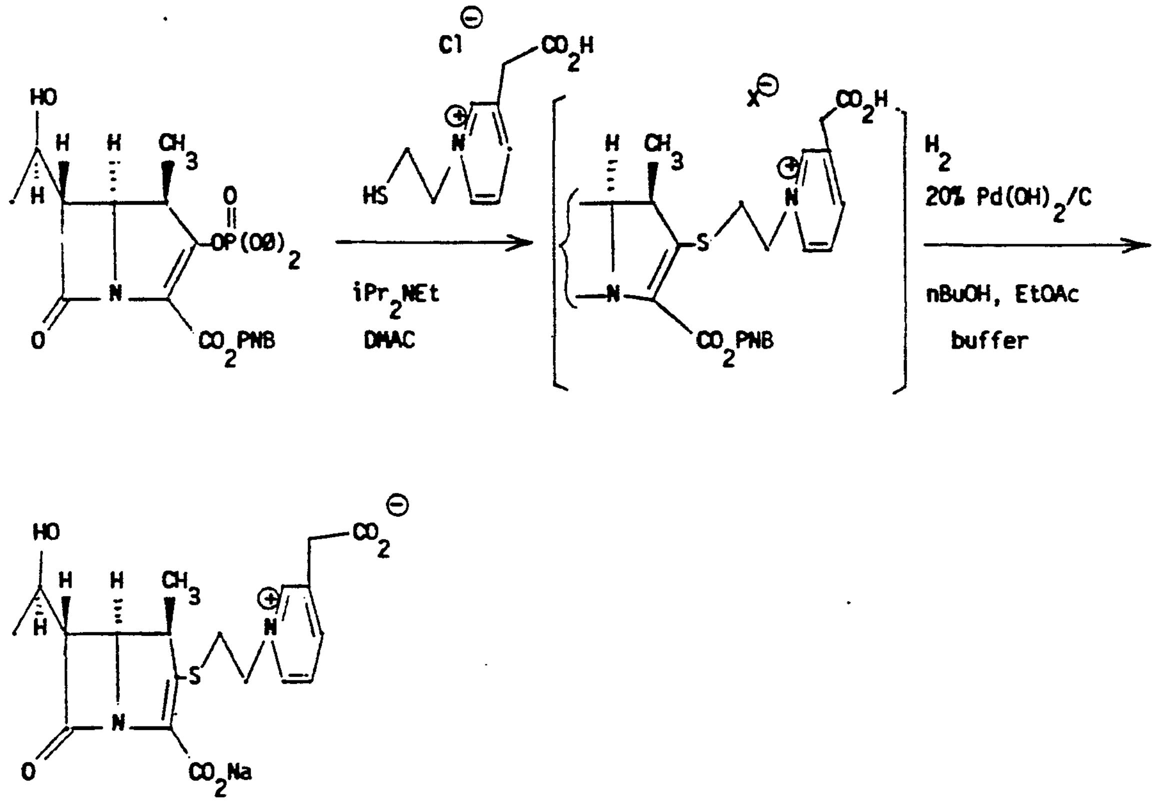


Sodium (5S,6S)-2-[2-(3-(carboxylatomethyl)pyridinium) ethyl]thio-6-[1(R)-hydroxyethyl]-1(R)-methylcarbapen-2-em-3-carboxylate

A solution of p-nitrobenzyl (5S,6S)-2-(diphenylphosphono)oxy-6-[1(R)-hydroxyethyl]-1(R)-methylcarbapen-2-em-3-carboxylate (31 mg, 0.052 mmol) and 3-carboxymethyl-1-(2-mercaptoethyl)pyridinium chloride (21 mg, 0.09 mmol) in anhydrous N,N-dimethylacetamide (0.5 ml) was cooled in an ice bath and treated with N,N-diisopropylethylamine (0.02 ml, 0.11 mmol). After stirring 20 minutes in the cold, the mixture was treated with more N,N-diisopropylethylamine (0.01 ml) and stirred an additional 5 minutes at ca. 0°C. The mixture was diluted with n-butanol (1.0 ml), ethyl acetate (0.5 ml), water (1.0 ml) and 0.5 M pH 6.8 N-methylmorpholine-hydrochloric acid buffer (0.5 ml), mixed with 20% palladium hydroxide on carbon (15 mg), and hydrogenated at 42 psi for 1 hour on a Parr shaker. The catalyst was removed by centrifugation and the supernatant was diluted with water, washed with methylene chloride, filtered through a Gelman CR acrodisc, and concentrated under vacuum to ca. 3 ml volume. The aqueous solution was added to a Dowex 50W-X4 column (sodium form, 200-400 mesh, 1.5 X 27 cm) which was eluted with water in a cold room. The product containing fractions were located by UV, combined, and concentrated under vacuum. The resulting solution was streaked onto two 0.5 mm X 20 X 20 cm Analtech RPS-F plates which were developed in a cold room using 2% ethanol in water. The UV visible band at Rf 0.13-0.4 was removed and eluted with 4:1 acetonitrile-water (100 ml). The eluant was washed with hexane, concentrated under vacuum to ca. 10 ml, filtered through a Gelman CR acrodisc, and lyophilized to afford the title compound (6.8 mg) as an amorphous, off-white solid.

UV ($H_2O$) $\lambda$max 295 nm ($\epsilon$ 5,210); ($H_2O$ + $NH_2OH$) extinguished $\lambda$max 298 nm ($\epsilon$ 4,920);

NMR ($D_2O$) $\delta$ 1.13 (d, J=7.3 Hz, C<u>H</u>$CH_3$), 1.27 (d, J=6.4 Hz, C<u>H</u>$_3$CHOH), 3.19 (qd. J=7.3 and 9.5 Hz, H1), 3.2-3.3 (m, SC<u>Ha</u>Hb), 3.40 (dd, J=2.6 and 6.1 Hz, H6), 3.55-3.65 (m, SCHa<u>Hb</u>), 3.79 (s, $CH_2CO_2$), 3.91 (dd, J=2.6 and 9.5 Hz, H5), 4.22 (p, J$\sim$6 Hz, $CH_3$C<u>H</u>OH), 8.00 (dd, J=6 and 8 Hz, ArH), 8.49 (d, J=8 Hz, ArH), 8.72 (d, J=6 Hz, ArH), 8.79 (s, ArH).

EXAMPLE 4

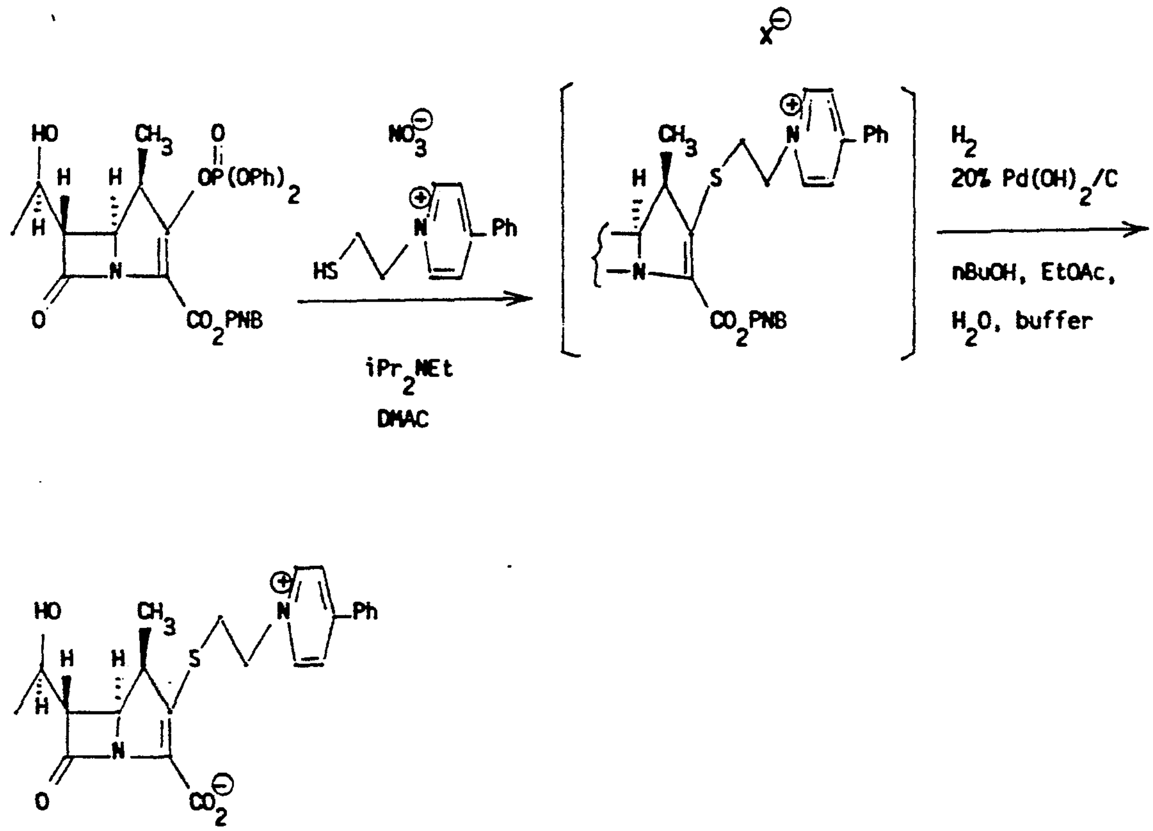


(5S,6S)-6-[(1R)-Hydroxyethyl]-1(R)-methyl-2-[2-(4-phenyl-1-pyridinium)ethylthio]-carbapen-2-em-3-carboxylate

A solution of p-nitrobenzyl(5S,6S)-2-(diphenylphosphono)oxy-6-[(1R)-hydroxyethyl]-1(R)-methylcarbapen-2-em-3-carboxylate (59.5 mg, 0.1 mmol) and 1-(2-mercaptoethyl)-4-phenylpyridinium nitrate (33.4 mg, 0.12 mmol) in anhydrous dimethylacetamide (1.0 ml) was cooled to ca. -20°C under a nitrogen atmosphere and treated dropwise with N,N-diisopropylethylamine (0.0227 ml, 0.13 mmol). After stirring at ca. -20°C for 30 minutes, the reaction mixture was diluted with n-butanol (5 ml),

ethyl acetate (2.5 ml), water (5 ml) and 0.5 M pH 6.8 N-methylmorpholine-hydrochloric acid buffer (2.5 ml), treated with 20% palladium hydroxide on carbon (30 mg), and hydrogenated on a Parr shaker at 45 psi for 60 minutes. The mixture was filtered through a celite pad to remove the catalyst which was washed with water. The aqueous portion of the filtrate was separated, washed three times with methylene chloride, concentrated under vaccum to ca. 2 ml, and added to a column of Dowex 50W-X4 resin (sodium form, 200-400 mesh, 1.5 x 30 cm) which was eluted with water and aqueous tetrahydrofuran in a cold room. Fractions containing 350 drops of eluant were collected. After 8 fractions, the solvent was changed from water to 5% tetrahydrofuran in water; and after 25 fractions, the solvent was changed to 10% tetrahydrofuran in water. The product containing fractions (30-56) were located by UV, combined, concentrated under vaccum to ca. 10 ml, filtered through a Gelman CR acrodisc, and lyophilized to provide the title compound (17.9 mg) as a pale yellow, amorphous solid.

IR (Nujol) $\nu$ max 3370 (br), 1748, 1637, 1590 $cm^{-1}$;
UV (0.05 M pH 7.0 buffer) $\lambda$ max 294 nm ($\epsilon$ 21,020);
UV (buffer + $NH_2OH.HCl$) $\lambda$ max 297 nm ($\epsilon$ 17,030) and extinguished $\lambda$ max 289 nm ($\epsilon$ 4,400);
NMR ($D_2O$) $\delta$ 1.10 (d, J=7.2 Hz, C$\underline{H}_3$CH), 1.18 (d, J=6.2 Hz, C$\underline{H}_3$CHOH), 3.11 (m, $CH_3C\underline{H}$), 3.37 and 3.56 (two m, $SCH_2$), 3.34 (dd, J=2.8 and 6.1 Hz, H6), 3.74 (dd, J=2.8 and 9.3 Hz, H5), 4.06 (p, J=6.2 Hz, $CH_3C\underline{H}OH$), 7.7 (m, 2 phenyl H), 8.0 (m, 3 phenyl H), 8.33 (d, J=6.9 Hz, 2 pyridyl H), 8.84 (d, J=6.9 Hz, 2 pyridyl H), ($NCH_2$ obscured by HOD peak).

EXAMPLE 5

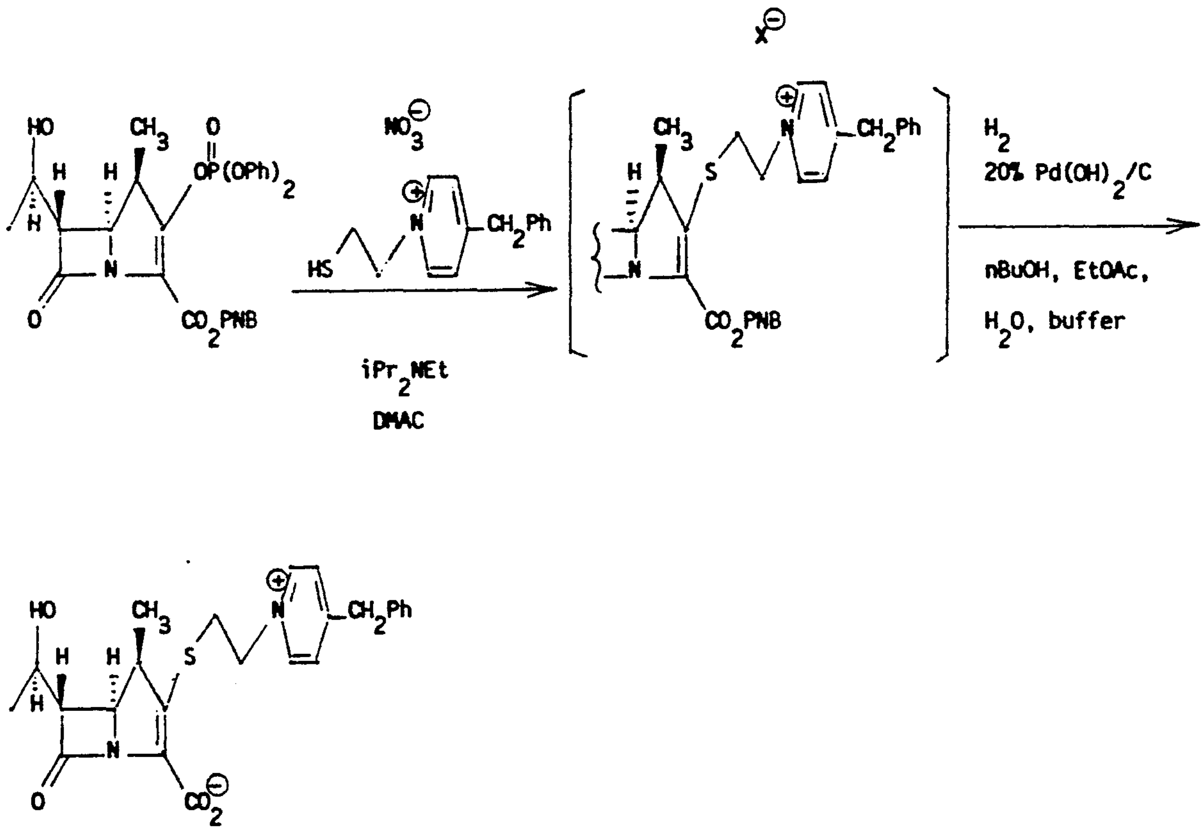


(5S,6S)-2-[2-(4-Benzyl-1-pyridinium)ethylthio]-6-[(1R)-hydroxyethyl]-1(R)-methylcarbapen-2-em-3-carboxylate

A solution of p-nitrobenzyl (5S,6S)-2-(diphenylphosphono)oxy-6-[(1R)-hydroxyethyl]-1(R)-methylcarbapen-2-em-3-carboxylate (59.5 mg, 0.1 mmol) and crude 4-benzyl-1-(2-mercaptoethyl)pyridinium nitrate (38 mg, 0.13 mmol) in anhydrous dimethylacetamide (1.0 ml) was cooled to -20°C under a nitrogen atmosphere and treated with N,N-diisopropylethylamine (0.026 ml, 0.15 mmol). The reaction mixture was stirred at -20°C for 30 minutes, then diluted with n-butanol (5 ml), ethyl acetate (2.5

ml), water (5 ml), and 0.5 M pH 6.8 N-methylmorpholine-hydrochloric acid buffer (2.5 ml), treated with 20% palladium hydroxide on carbon (30 mg), and hydrogenated in a Parr apparatus at 45 psi for 80 minutes. The mixture was filtered through a celite pad to remove the catalyst. The aqueous portion of the filtrate was washed three times with methylene chloride, concentrated under vacuum to ca. 2.5 ml, and added to a column of Dowex 50W-X4 resin (sodium form, 200-400 mesh, 1.5 X 35 cm). The column was eluted in a cold room with water (25 x 20 ml fractions) followed by 5% tetrahydrofuran in water (20 ml fractions). The product containing fractions (27-55) were located by UV, combined, concentrated under vacuum, filtered through a Gelman CR acrodisc, and lyophilized to give the title compound (24.4 mg) as an amorphous, yellow solid.

IR (Nujol) $\nu$ max 3320 (br), 1745, 1592 $cm^{-1}$;

UV (0.05M pH 7.0 buffer) $\lambda$ max 293 nm ($\epsilon$8110), 256 nm ($\epsilon$7440);

UV (buffer + $NH_2OH.HCl$) $\lambda$ max 253 nm ($\epsilon$6180) and extinguished max 298 nm ( 6760);

NMR ($D_2O$) $\delta$ 1.09 (d, J=7.2 Hz, $C\underline{H}_3CH$), 1.28 (d, J=6.4 Hz, $C\underline{H}_3CHOH$), 3.13 (m, $CH_3C\underline{H}$), 3.2-3.6 (m, $SCH_2$), 3.37 (dd, J=2.8 and 6.4 Hz, H6), 3.77 (dd, J=2.8 and 9.5 Hz, H5), 4.16 (p, J=6.4 Hz, $CH_3C\underline{H}OH$), 4.36 (s, $CH_2Ph$), 7.48 (m, 5 phenyl H), 7.87 (d, J=6.7 Hz, 2 pyridyl H), 8.69 (d, J=6.7 Hz, 2 pyridyl H), ($NCH_2$ obscured by HOD peak).

EXAMPLE 6

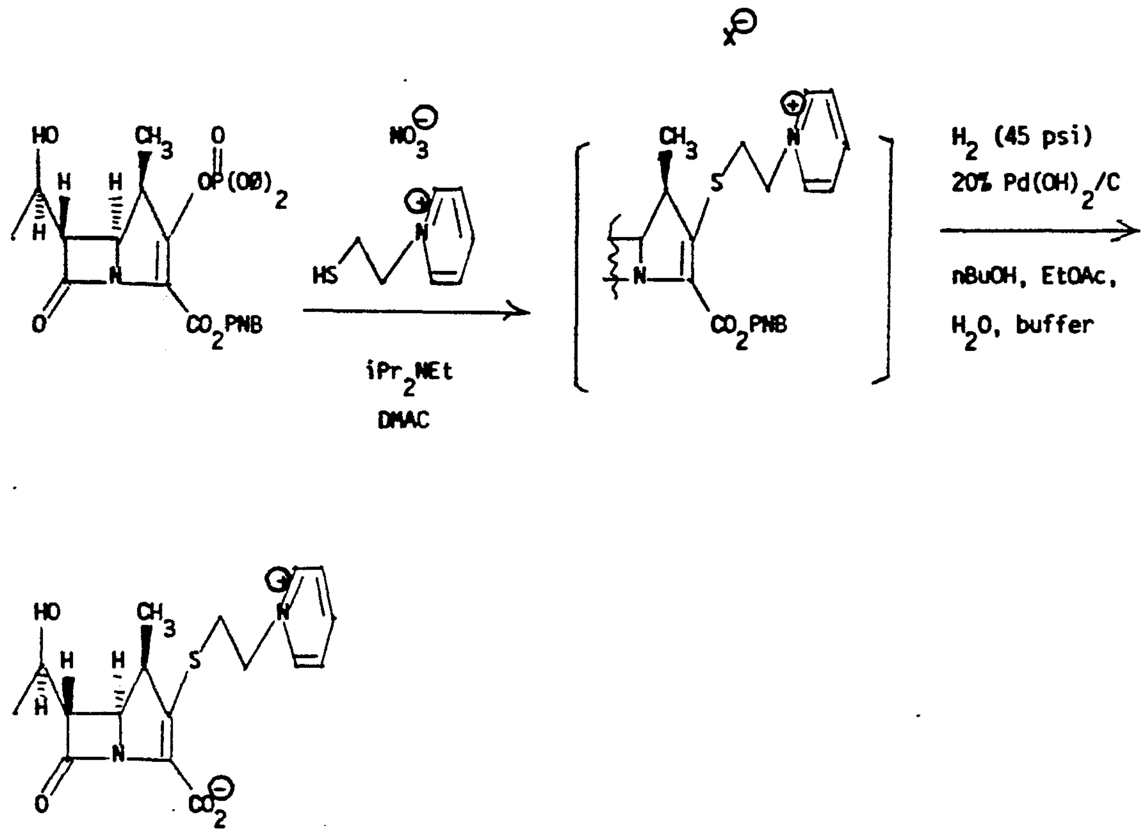


(5S,6S)-6-[1(R)-hydroxyethyl]-1(R)-methyl-2-(2-pyridiniumethylthio)-carbapen-2-em-3-carboxylate

A solution of p-nitrobenzyl(5S,6S)-2-diphenylphosphono)oxy-6-[1(R)-hydroxyethyl]-1(R)methyl-carbapen-2-em-3-carboxylate (125 mg, 0.21 mmol) and crude 1-(2-mercaptoethyl)pyridinium nitrate (49 mg, ca. 0.24 mmol) in anhydrous dimethylacetamide (2.1 ml) under a nitrogen atmosphere was cooled in an ice-water bath and treated with N,N-diisopropylethylamine (0.081 ml, 0.47 mmol). After stirring at ice bath temperature for 15 minutes, the solution was diluted with n-butanol (4.2 ml) ethyl acetate (2.1 ml), water (4.2 ml), and 0.5 M pH 6.8 N-methyl-

morpholine-hydrochloric acid buffer (2.1 ml), treated with 20% palladium hydroxide on carbon (60 mg), and hydrogenated on a Parr shaker at 45 psi for 60 minutes. The catalyst was removed by centrifugation and the supernatant was washed with methylene chloride. The aqueous phase was concentrated under vacuum to ca. 4 ml and charged onto a column of Dowex 50-X4 (sodium form, 200-400 mesh, 1.5 x 32 cm). The column was eluted with water in a cold room (4°) at 6.0 ml fractions/2.0 minutes. Fractions 13 through 24, which contained most of the product by UV, were combined and concentrated under vacuum to ca. 10 ml volume and lyophilized to afford the title compound (33.6 mg) as an amorphous yellow powder.

IR (Nujol) $\nu$ max 3300 (br), 1758, 1605 $cm^{-1}$;

UV (water) $\lambda$ max 294 nm ($\epsilon$8,290);

UV (water + $NH_2OH.HCl$) extinguished $\lambda$ max 296 nm ($\epsilon$ 7,590);

NMR ($D_2O$) $\delta$ 1.14 (d, J=7.3 Hz, $C\underline{H}_3CH$), 1.28 (d, J=6.3 Hz, $C\underline{H}_3CHOH$), 3.23 (qd, J=7.3, 9.5 Hz, $CH_3C\underline{H}$), 3.29 (dt, J=5.7, 15.6 Hz, $SC\underline{H}_AH_B$), 3.42 (dd, J=2.8, 6.0 Hz, H6), 3.56 (ddd, J=5.2, 7.2, 15.6 Hz $SCH_A\underline{H}_B$), 3.95 (dd, J=2.8, 9.5 Hz, H5), 4.23 (p, J 6.3 Hz, $CH_3C\underline{H}OH$), 8.10 (dd, J=5.7, 7.8 Hz, 2ArH), 8.63 (t, J=7.8 Hz, ArH), 8.86 (d, J=5.7 Hz, 2ArH).

EXAMPLE 7

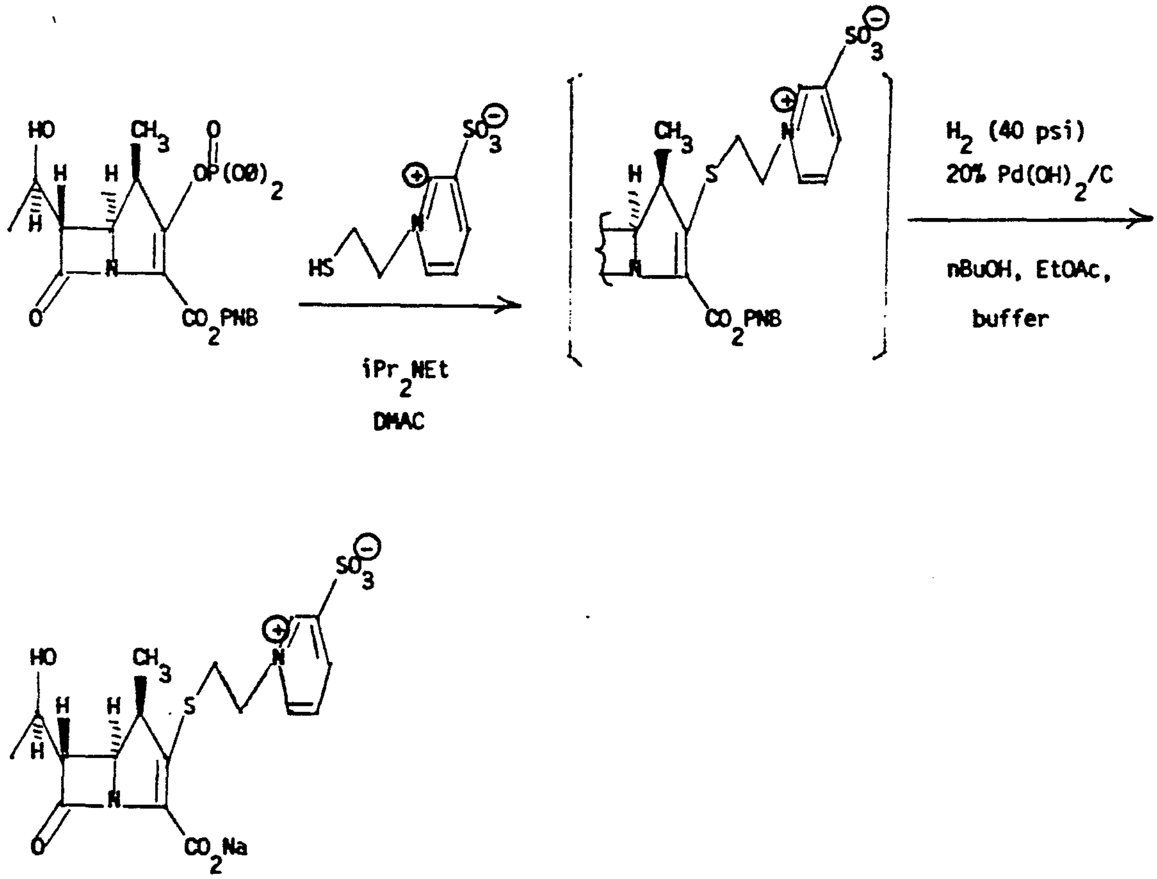


Sodium [5S,6S)-6-[1(R)-hydroxyethyl]-1(R)-methyl-2-[2-(3-sulfopyridinium)ethylthio]-carbapen-2-em-3-carboxylate

A solution of p-nitrobenzyl (5S,6S)-2-(diphenylphosphono)oxy-6-[1(R)-hydroxyethyl]-1(R)-methyl-carbapen-2-em-3-carboxylate (30 mg, 0.051 mmol) and crude 1-(2-mercaptoethyl)pyridinium-3-sulfonate (16 mg, ca. 0.073 mmol) in anhydrous dimethylacetamide (0.5 ml) under a nitrogen atmosphere was cooled in an ice-water bath and treated with N,N-diisopropylethylamine (0.020 ml, 0.11 mmol).

After stirring at 0° for 20 minutes, the reaction solution was diluted with $Et_2O$ (10 ml) and the precipitated solid recovered by filtration. The solid was dissolved in n-butanol (1 ml), ethyl acetate (0.5 ml), water (1 ml), and 0.5 M pH 6.8 N-methylmorpholine-hydrochloric acid buffer (0.5 ml), treated with 20% palladium hydroxide on carbon (34 mg) and hydrogenated on a Parr shaker at 40 psi for 60 minutes. The catalyst was removed by centrifugation and the supernatant was washed with methylene chloride. The aqueous phase was concentrated under vacuum to ca. 4 ml and charged onto a column of Dowex 50W-X4 (sodium form, 200-400 mesh, 1.5 x 24 cm). The column was eluted with water in a cold room (4°) at 4.5 ml fractions/2.0 minutes. Fractions 3 through 5, which contained most of the product by UV, were combined and concentrated under vacuum to ca. 1.5 ml. This solution was chromatographed on two 1.0 mm x 20 x 20 cm Analtech RPS-F plates using 1.5% ethanol/water as developing solvent in a cold room. The major UV visible bands at $R_f$ 0.4-0.6 were removed and eluted with 4:1 acetonitrile-water (100 ml). The eluant was washed with hexane, concentrated under vacuum to ca. 13 ml volume, passed through a Gelman CR acrodisc (0.45 μ) and lyophilized to afford the title compound (10.7 mg) as an amorphous white powder.

IR (Nujol) $\nu$ max 3400 (br), 1750, 1592 $cm^{-1}$;

UV (water) $\lambda$ max 293 nm ($\epsilon$6450).

UV (water + $NH_2OH.HCl$) extinguished $\lambda$ max 295 nm ($\epsilon$5620).

NMR ($D_2O$) $\delta$ 1.12 (d, J=7.2 Hz, $C\underline{H}_3CH$), 1.27 (d, J=6.4 Hz, $C\underline{H}_3CHOH$), 3.17 (dd, J=7.2, 9.4 Hz,

$CH_3\underline{CH}$), 3.36 (td, J=5.1, 15.2 Hz, $SC\underline{H}_aH_b$), 3.41 (dd, J=2.5, 6.2 Hz, H6), 3.58 (ddd, J=5.0, 8.4, 15.2 Hz, $SCH_a\underline{H}_b$), 3.98 (dd, J=2.5, 9.4 Hz, H5), 4.20 (p, J~6.2 Hz, $CH_3C\underline{H}OH$), 8.17 (dd, J=6.1, 8.0 Hz, ArH), 8.92-9.01 (m, 2ArH), 9.40 (s, ArH).

## EXAMPLE 8

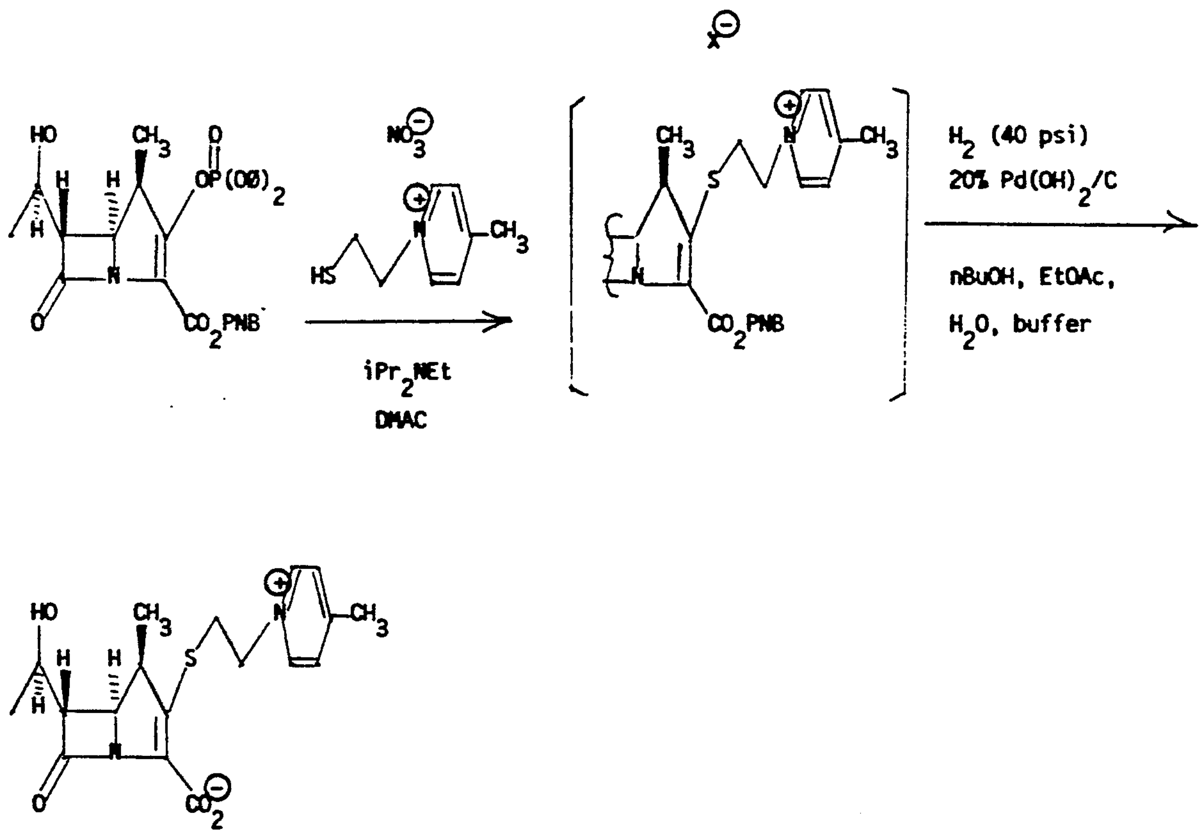


(5S,6S)-6-[1(R)-hydroxyethyl]-1(R)-methyl-2-[2-(4-methylpyridinium)ethylthio]-carbapen-2-em-3-carboxylate

A solution of p-nitrobenzyl (5S,6S)-2-(diphenylphosphono)oxy-6-[1(R)-hydroxyethyl]-1(R)-methyl-carbapen-2-em-3-carboxylate (50 mg, 0.084 mmol) and crude 4-methyl-1-(2-mercaptoethyl)pyridinium

nitrate (20 mg, ca. 0.093 mmol) in anhydrous dimethylacetamide (0.84 ml) under a nitrogen atmosphere was cooled in an ice-water bath and treated with N,N-diisopropylethylamine (0.032 ml, 0.19 mmol). After stirring at ice bath temperature for 15 minutes, the reaction solution was diluted with n-butanol (1.7 ml), ethyl acetate (0.8 ml), water (1.7 ml), and 0.5 M pH 6.8 N-methylmorpholine-hydrochloric acid buffer (0.8 ml), treated with 20% palladium hydroxide on carbon (20 mg), and hydrogenated or a Parr shaker at 40 psi for 60 minutes. The catalyst was removed by centrifugation and the supernatant was washed with methylene chloride. The aqueous phase was concentrated under vacuum to ca. 4 ml and charged onto a column of Dowex 50-X4 (sodium form, 200-400 mesh, 1.5 x 30 cm). The column was eluted with water in a cold room (4°) at 7.0 ml fractions/2.0 min. Fractions 12 through 25 which contained most of the product by UV were concentrated under vacuum to ca. 11 ml volume and lyophilized to afford the title compound (12 mg) as an amorphous white powder.

IR (Nujol) $\nu$max 3300 (br), 1758, 1648, 1595 $cm^{-1}$;
UV (water) $\lambda$ max 294 nm ($\epsilon$8,170);
UV (water + $NH_2OH.HCl$) extinguished $\lambda$max 296 nm ($\epsilon$7,550);
NMR ($D_2O$) $\delta$ 1.11 (d, J=7.3 Hz, $C\underline{H}_3CH$), 1.27 (d, J=7.3 Hz, $C\underline{H}_3CHOH$), 2.68 (s, $ArCH_3$), 3.18 (qd, J=7.3, 9.5 Hz, $CH_3C\underline{H}$), 3.27 (dt, J=5.3, 15.5 Hz, $SC\underline{H}_AH_B$), 3.40 (dd, J=2.7, 5.9 Hz, H6), 3.51 (ddd, J=4.8, 5.0, 15.5 Hz, $SCH_A\underline{H}_B$), 3.89 (dd, J=2.7, 9.5 Hz H5), 4.23 (p, J~6.3 Hz, $CH_3C\underline{H}OH$), 7.88 (d, J=6.6 Hz, 2ArH), 8.64 (d, J=6.6 Hz, 2ArH).

EXAMPLE 9

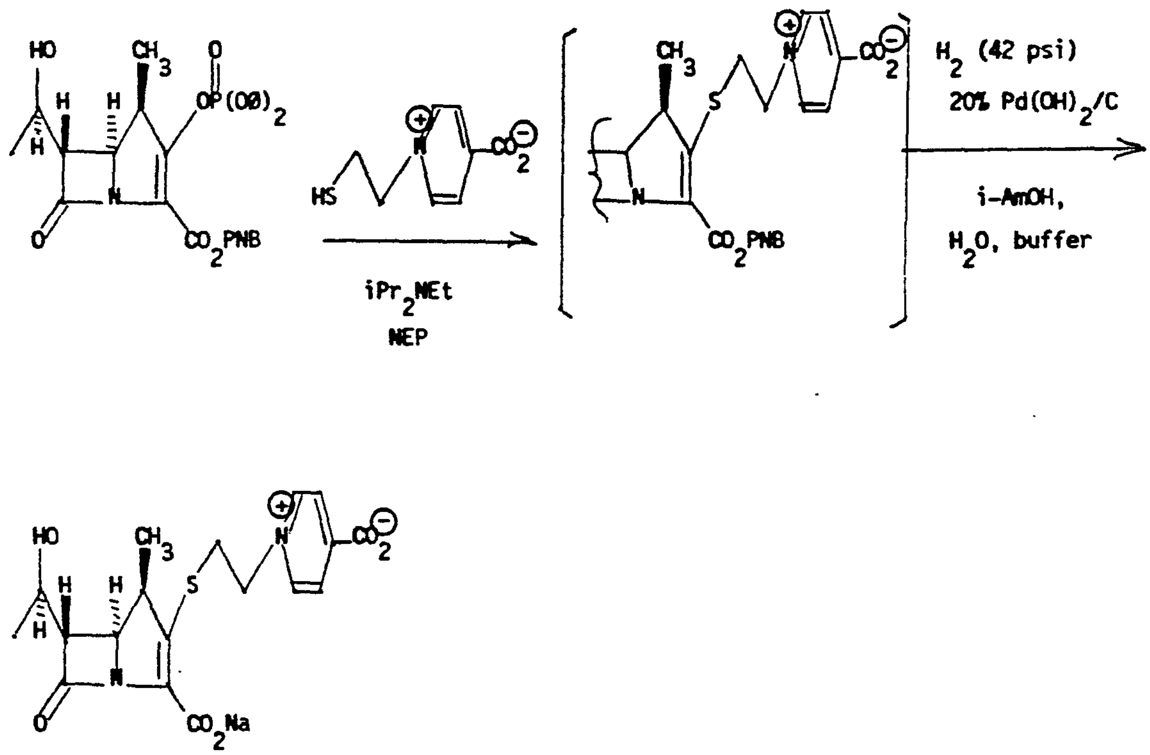


Sodium (5S,6S)-6[1(R)-hydroxyethyl]-1(R)-methyl-2-[2-(4-carboxypyridinium)ethylthio]-carbapen-2-em-3-carboxylate

A solution of p-nitrobenzyl (5S,6S)-2-(diphenylphosphono)oxy-6-[1(R)-hydroxyethyl]-1(R)-methyl-carbapen-2-em-3-carboxylate (75 mg, 0.13 mmol) and crude 1-(2-mercaptoethyl)pyridinium-4-carboxylate (26 mg, ca. 0.14 mmol) in anhydrous 1-ethyl-2-pyrrolidinone (0.50 ml) under a nitrogen atmosphere was cooled in an ice-water bath and treated with N,N-diisopropylethylamine (0.036 ml, 0.21 mmol). After stirring at 0° for 25 minutes, the reaction solution was diluted with water (1.3 ml), isoamyl

alcohol (1.8 ml), and 0.5 M pH 6.8 N-methylmorpholine-hydrochloric acid buffer (1.0 ml), treated with 20% palladium hydroxide on carbon (34 mg), and hydrogenated on a Parr shaker at 42 psi for 60 minutes. The catalyst was removed by filtration through a celite pad, and the filtrate was washed with methylene chloride. The aqueous phase was concentrated under vacuum to ca. 4 ml and charged onto a column of Dowex 50-X4 (sodium form, 200-400 mesh, 1.5 x 29 cm). The column was eluted with water in a cold room (4°) at 6.4 ml fractions/2.0 minutes. Fractions 4 through 9, which contained product by UV, were combined and concentrated under vacuum to ca. 1.5 ml. This solution was chromatographed on three 1.0 mm x 20 x 20 cm Analtech RPS-F plates using 15% EtOH/water as developing solvent in a cold room. The major UV visible bands of $R_f$ 0.2-0.4 were removed and eluted with 4:1 acetonitrile-water (100 ml). The eluate was washed with hexanes, concentrated under vacuum to ca. 16 ml volume, passed through a Gelman CR acrodisc (0.45μ), and lyophilized to afford the title compound (21.2 mg) as an amorphous white powder.
IR (Nujol) $\nu$ max 3350 (br), 1755, 1625, 1570 $cm^{-1}$;
UV (water) $\lambda$ max 293 nm ($\epsilon$7,370);
UV (water + $NH_2OH.HCl$) extinguished $\lambda$ max 298 nm ($\epsilon$ 6,280);
NMR ($D_2O$) $\delta$ 1.15 (d, J=7.2 Hz, $C\underline{H}_3CH$), 1.28 (d, J=6.4 Hz, $C\underline{H}_3CHOH$), 3.2-3.3 (m, $CH_3C\underline{H}$), 3.29 (td, J=6.3, 15.3 Hz, $SC\underline{H}_aH_b$), 3.42 (dd, J=2.7, 6.3 Hz, H6), 3.57 (td, J=5.8, 15.3 Hz, $SCH_a\underline{H}_b$), 3.97 (dd, J=2.7, 9.4 Hz, H5), 4.22 (p, J~6.4 Hz, $CH_3C\underline{H}OH$), 8.30 (d, J=6.7 Hz, 2ArH), 8.91 (d, J=6.7 Hz, 2ArH).

<u>EXAMPLE 10</u>

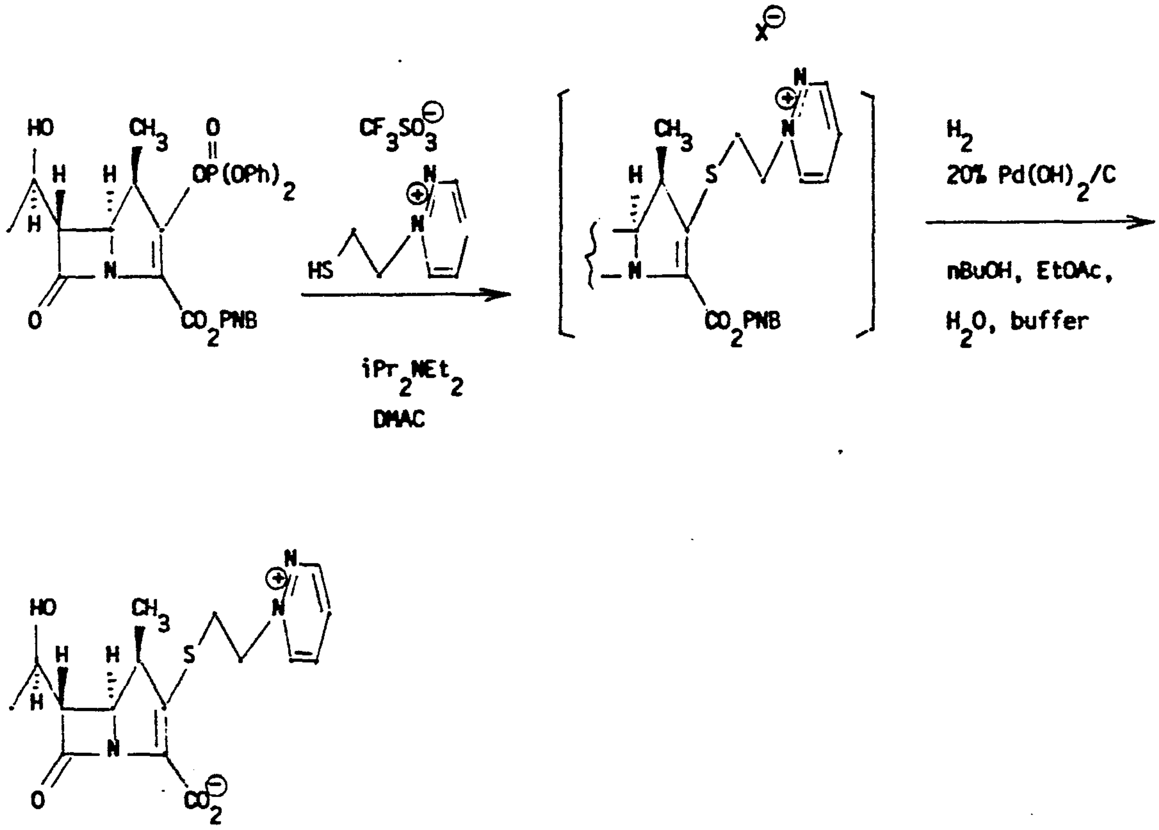


<u>(5S,6S)-6-[1(R)-hydroxyethyl]-2-[2-(1-pyridazinium) ethylthio]-1(R)-methylcarbapen-2-em-3-carboxylate</u>

A solution of p-nitrobenzyl (5S,6S)-2-(diphenylphosphono)oxy-6-[1(R)-hydroxyethyl]-1(R)-methylcarbapen-2-em-3-carboxylate (300 mg, 0.51 mmol) in anhydrous dimethylacetamide (1.65 ml) was cooled to -20°C and treated with 1-(2-mercaptoethyl)-pyridazinium triflate (163 mg, 0.57 mmol) and N,N-diisopropylethylamine (100 1, 0.57 mmol). After stirring at -20°C to -10°C for 40 minutes, the reaction mixture was diluted with n-butanol (10 ml), ethyl acetate (5.0 ml), D.I. water (10 ml), 0.5 <u>M</u> pH 6.8 N-methyl-morpholine-hydrochloride buffer (5.0 ml),

treated with 20% $Pd(OH)_2/C$, and hydrogenated on a Parr shaker at 45 psi for 2 hours. The catalyst was removed by filtrating through Prep-Disc filters (from Bio-Rad), and the filtrate was washed with methylene chloride. The aqueous layer was separated, concentrated to 2 ml, and charged onto a column of Dowex 50W-X4 (sodium form, 200-400 mesh, 2.4 X 20 cm). The column was eluted with water to give the title compound which was lyophilized to a pale-yellow solid powder.

UV ($H_2O$) $\lambda$ max 292 nm;

NMR ($D_2O$) $\delta$ 1.15 (d, C$\underline{H}_3$CH), 1.24 (d, C$\underline{H}_3$CHOH), 3.20-3.80 (m, $CH_3$C$\underline{H}$, H6, $SCH_2$), 4.12 (dd, H5), 4.25 (p, $CH_3$C$\underline{H}$OH), 5.10 (t, $NCH_2$), 8.60 and 9.65 (two m, pyridazinium-H).

EXAMPLE 11

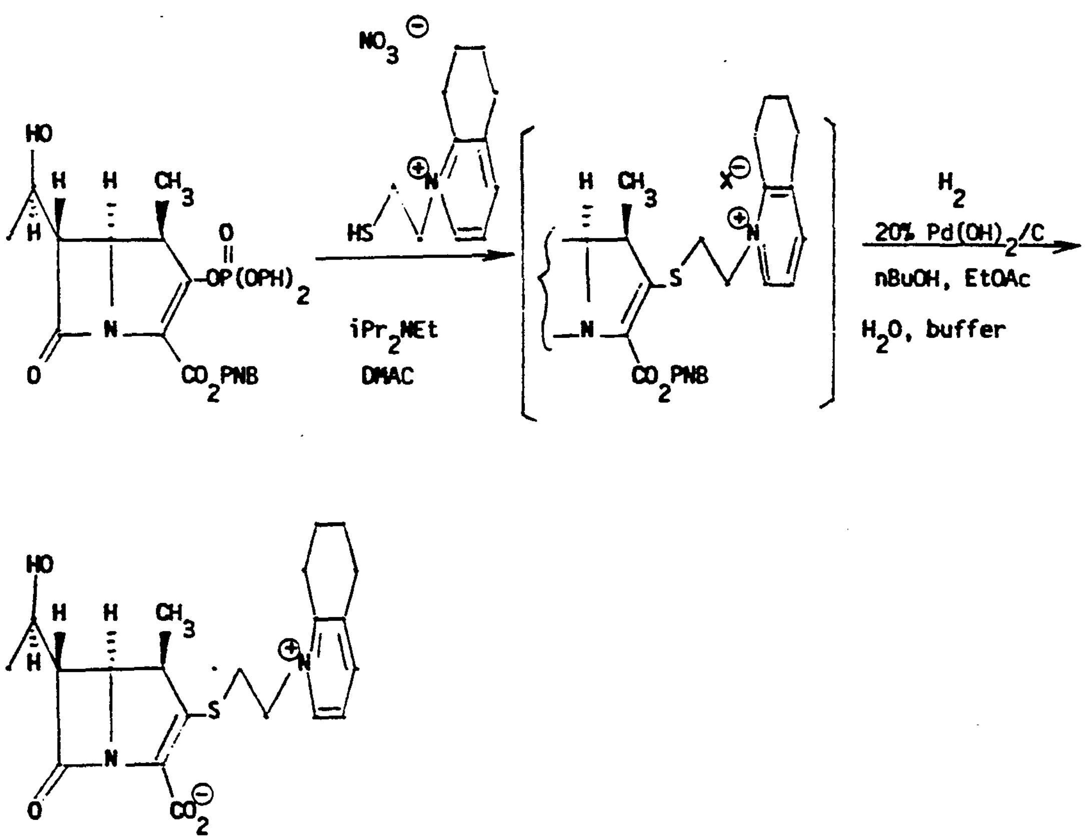

(5S,6S)-2-[2-(2,3-Cyclohexeno-1-pyridinium)ethylthio]-6-[(1R)-hydroxyethyl]-(1R)-methylcarbapen-2-em-3-carboxylate

A solution of p-nitrobenzyl (5S,6S)-2-(diphenylphosphono)oxy-6-[(1R)-hydroxyethyl]-1(R)-methylcarbapen-2-em-3-carboxylate (59.5 mg, 0.1 mmol and 2,3-cyclohexeno-1-(2-mercaptoethyl)pyridinium nitrate (28.2 mg, 0.11 mmol) in anhydrous dimethylacetamide (1.0 ml) was cooled to -20°C under a nitrogen atmosphere and treated with N,N-diisopropylethylamine (0.0209 ml, 0.12 mmol). The resulting solution was stirred at -20°C for 30 minutes, then diluted with n-butanol (5 ml), ethylacetate (2.5 ml), water (5 ml) and 0.5M pH 6,8 N-methylmorpholine-hydrochloric acid buffer, treated with 20% palladium hydroxide on carbon (30 mg), and hydrogenated at 47 psi for one hour. The mixture was filtered through celite with the aid of additional water. The aqueous portion of the filtrate was washed with methylene chloride (3X), concentrated under vacuum to ca. 2 ml, and added to a column of Dowex 50W-X4 resin (sodium form, 200-400 mesh, 1.5 x 35 cm). The column was eluted with deionized water in a cold room at the rate of 20 ml fractions every 7 minutes. The product containing fractions (6-9) were located by UV, combined, concentrated under vacuum to ca. 20 ml, filtered through a Gelman CR acrodisc, and lyophilized to give the title compound (22.4 mg) as a pale yellow, amorphous solid.

IR (Nujol) $\nu$ max 1747, 1599 $cm^{-1}$;

UV (0.05 M pH 7.0 buffer) $\lambda$max 300 nm(sh, $\epsilon$ 8680), 282 nm ($\epsilon$11,290);

UV (buffer + $NH_2OH$ HCl) $\lambda$max 275 nm ($\epsilon$6160) and extinguished $\lambda$ max 298 nm ($\epsilon$7730);

NMR ($D_2O$) $\delta$ 1.13(d, J=7.2 Hz, C$\underline{H}_3$CH), 1.27 (d, J=6.4z, C$\underline{H}_3$CHOH), 1.76-2.08 (m, $CH_2CH_2$), 2.96-3.43 (m, $CH_3$C$\underline{H}$, $SCH_2$, and 2 aryl $CH_2$), 3.41 (dd, J=2.7 and 5.9 Hz, H6), 3.85 (dd, J=2.7 and 9.5 Hz, H5), 4.22 (~p, J=6.2 Hz, $CH_3$C$\underline{H}$OH), 7.74 (~dd, pyridyl H), 8.26 (~d, pyridyl H), 8.59 (~d, pyridyl H), ($NCH_2$ obscured by HOD peak).

## EXAMPLE 12

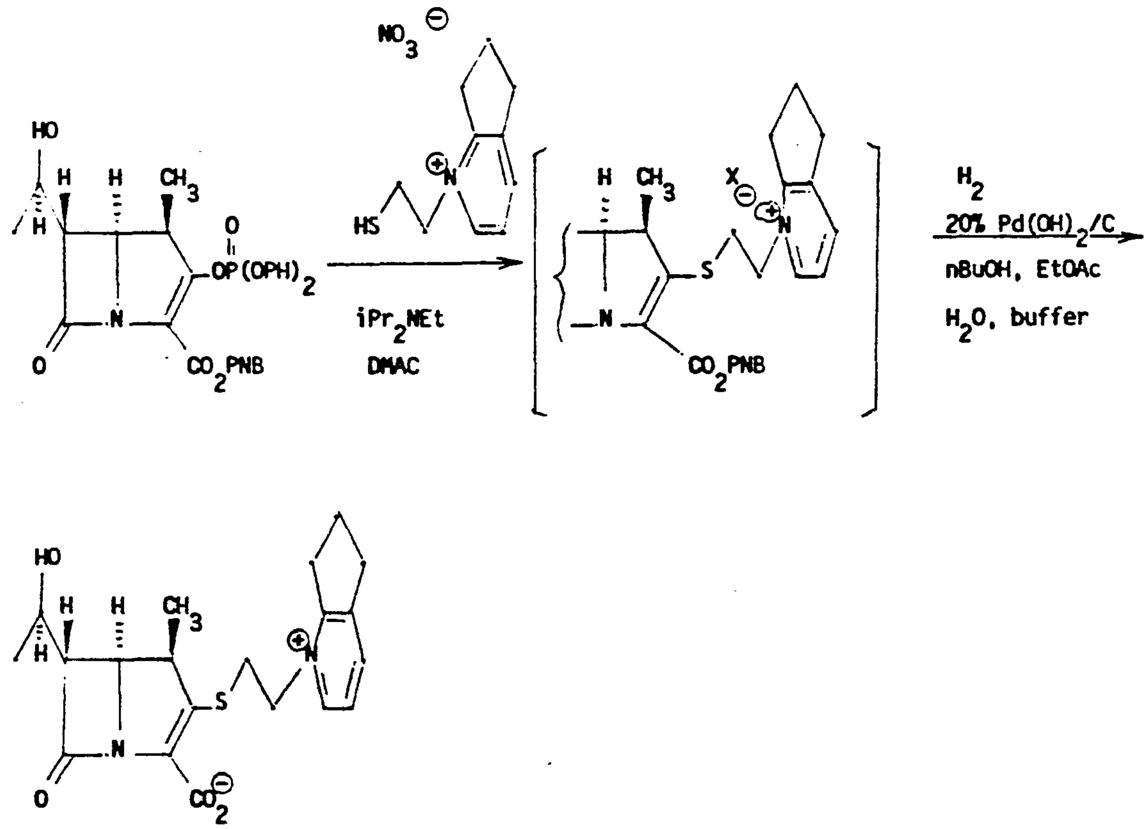


(5S,6S)-2-[2-(2,3-cyclopenteno-1-pyridinium)ethylthio]-6-[(1R)-hydroxyethyl]-1(R)-methylcarbapen-2-em-3-carboxylate

A solution of p-nitrobenzyl (5S,6S)-2-(diphenylphosphono)oxy-6-[1(R)-hydroxyethyl]-1(R)-methylcarbapen-2-em-3-carboxylate (59.5 mg, 0.1 mmol) in anhydrous dimethylacetamide (0.7 ml) was cooled to ca. -20°C and stirred under a nitrogen atmosphere while a solution of 2,3-cyclopenteno-1-(2-mercapto-ethyl)pyridinium nitrate (29 mg, 0.12 mmol) in dimethylacetamide (0.3 ml) was added dropwise. The resulting solution was treated with N,N-diisopropylethylamine (0.023 mL, 0.13 mmol) and stirred at -20°C for 30 minutes. The mixture was diluted with n-butanol (5 ml), ethyl acetate (2.5 ml), water (5 ml) and 0.5M pH 6.8 N-methylmorpholine-hydrochloric acid buffer (2.5 ml), treated with 20% palladium hydroxide on carbon (30 mg), and hydrogenated at 45 psi for 60 minutes. The mixture was filtered through a pad of celite, and the aqueous portion of the filtrate was washed with methylene chloride (3x), concentrated under vacuum to ca. 2 ml, and added to a column of Dower 50W-X4 resin (sodium form, 200-400 mesh, 1.5 x 38 cm). The column was eluted with deionized water in a cold room at a rate of 20 ml fractions every 7 minutes. The product containing fractions (5-9) were located by UV, combined, concentrated under vacuum and lyophilized to give the title compound (25.6 mg) as an amorphous, off-white solid.

IR (Nujol) $\nu$ max 3400 (br), 1745, 1595 $cm^{-1}$;
UV (0.05 M pH 7.0 buffer) $\lambda$ max 300 nm (sh, $\epsilon$ 7620), 283nm ($\epsilon$ 10,820);
UV (buffer + $NH_2OH\cdot HCl$) $\lambda$max 278 nm ($\epsilon$5870) and extinguished $\lambda$ max 299 nm ($\epsilon$6780);
NMR ($D_2O$) $\delta$ 1.13(d, J=7.2 Hz, C$\underline{H}_3$CH), 1.27 (d, J=6.3z, C$\underline{H}_3$CHOH), 2.32 (m, $CH_2$), 3.07-3.67 (m, $CH_3C\underline{H}$, $SCH_2$, 2 pyridyl-$CH_2$, H6), 3.86 (dd,

J=2.6 and 9.4 Hz, H5), 4.22 (p, J=6.3 Hz, $CH_3C\underline{H}OH$), 7.80 (dd, J=6.4 and 7.6 Hz, pyridyl-H), 8.33 (d, J=7.6 Hz, pyridyl-H), 8.53 (d, J=6.4 Hz, pyridyl-H), ($NCH_2$ obscured by HOD peak).

## EXAMPLE 13

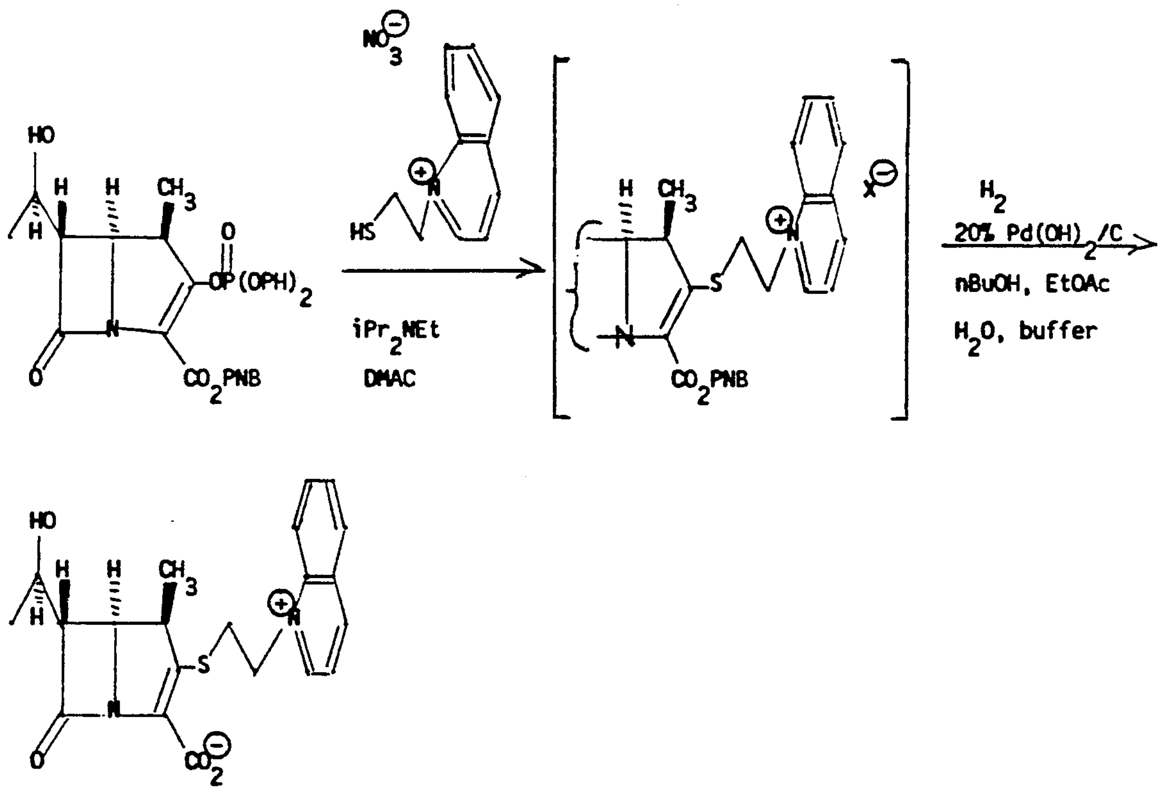


<u>(5S,6S)-6-[1(R)-hydroxyethyl]-2-[2-(1-quinolinium)-ethylthio]-1(R)-methylcarbapen-2-em-3-carboxylate</u>

A solution of 1-(2-mercaptoethyl)quinolinium nitrate (30.1 mg, 0.12 mmol) in dimethylacetamide (0.3 ml) was added dropwise over 5 minutes to a solution of p-nitrobenzyl (5S,6S)-2-(diphenylphosphono)oxy-6-[1(R)-hydroxyethyl]-1(R)-methylcarbapen-2-em-3-carboxylate (59.5 mg, 0.1 mmol) and N,N-diisopropylethylamine (0.023 ml, 0.13 mmol) in dimethylacetamide (0.7 ml) which was cooled to -20°C and stirred under a nitrogen atmosphere. After aging 45 minutes at -20°C, the reaction mixture was diluted with n-butanol (5 ml), ethylacetate (2.5 ml), water (5 ml) and 0.5M pH 6.8 N-methyl-morpholine-hydrochloric and buffer (2.5 ml), treated with 20% palladium hydroxide on carbon (30 mg), and hydrogenated at 45 psi for 60 minutes on a Parr shaker. The mixture was diluted with water and filtered to remove the catalyst. The filtrate was washed with methylene chloride (3x), concentrated under vacuum to ca. 2 ml, and added to a column of Dowex 50W-X4 (sodium form, 200-400 mesh, 1.5 x 36 cm.) which was eluted with deionized water in a cold room at a rate of 10 ml fractions every 3.5 minutes. Fractions 18-36, which contained 3.4 mg of the title compound by UV analysis, were combined, concentrated under vacuum, and lyophilized to afford a light brown solid. This material was combined with the product (2.5 mg by UV analysis) derived from a similar experiment and chromatographed on a 0.25 mm x 20 x 20 cm Analtech RPS-F plate which was developed with water in a cold room. The UV visible band at $R_f$ 0.07-0.18 was removed and eluted with 4:1 acetonitrile-water. The eluant was diluted with water, washed with petroleum ether, concentrated under vacuum, filtered through a Gelman CR acrodisc, and lyophilized to afford the title compound (2.3 mg) as a yellow, amorphous solid.

UV ($H_2O$) $\lambda$max 302 nm;

UV ($H_2O$ + $NH_2OH$) $\lambda$max 317 nm and extinguished $\lambda$max 297 nm;

NMR ($D_2O$) $\delta$ 0.90 (d, J=7.1 Hz, $C\underline{H}_3CH$), 1.13 (d, J=6.4 Hz, $C\underline{H}_3CHOH$), 2.41(dq, J=9.3 and 7.1 Hz, $CH_3C\underline{H}$), 2.66 (dd, J=2.6 and 9.3 Hz, H5), 3.17 (dd, J=2.6 and 5.7 Hz, H6), 3.44 (td, J=4 and 15.5 Hz, $SC\underline{H}aHb$), 3.70 (ddd, J=4.3, 10.4, and 15.5 Hz, $SCHa\underline{H}b$), 4.06 (p, J $\sim$6Hz, $CH_3C\underline{H}OH$), 5.03 (m, $NC\underline{H}aHb$), 5.66 (td, J=4 and 14,5 Hz, $NCHa\underline{H}b$), 7.97, 8.12, 8.34, 8.46, 8.56, 9.18, and 9.24 (seven m's, 7 quinolinium-H).

The following examples illustrate preparation of useful intermediates for the preparation of Formula I compounds.

## EXAMPLE 14

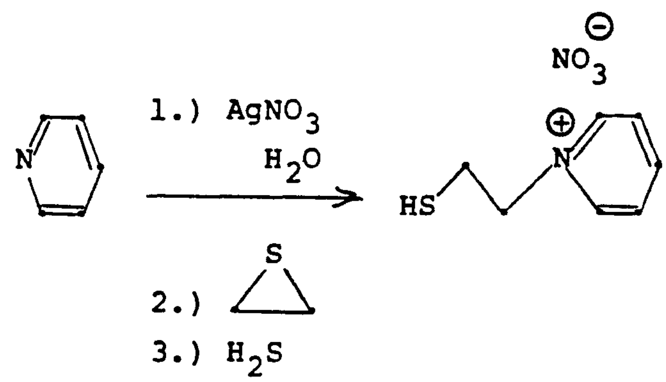


1-(2-Mercaptoethyl)pyridinium nitrate

A solution of silver nitrate (42.81 g, 0.252 mol) in water (100 ml) was added to a stirring solution of pyridine (20.4 ml, 0.252 mol) in water (100 ml). A white solid precipitated. The mixture was diluted with water (50 ml), cooled in an ice-bath, stirred, and treated dropwise with ethylene sulfide (15.0 ml, 0.252 mol). The resulting solution

was stirred at room temperature for 2 hours. Hydrogen sulfide was bubbled through the reaction mixture for 15 minutes to give a suspension which was filtered through a celite pad. The filtrate was evaporated under vacuum to give the title compound (33.2 g) as a crystalline mass.

NMR ($D_2O$) $\delta$ 3.23 (t, J=7 Hz, $SCH_2$), 4.87 (t, J=7 Hz, $NCH_2$), 8.0-9.0 (m, ArH).

## EXAMPLE 15

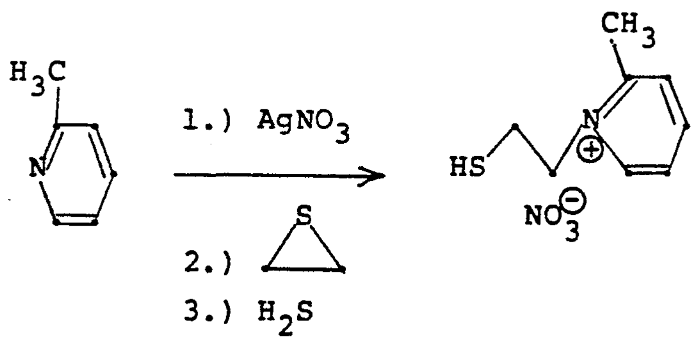


1-(2-Mercaptoethyl)-2-methylpyridinium nitrate

A solution of silver nitrate (11.41 g, 0.067 mol) in water (27 ml) was added to an ice-cold, stirring solution of 2-picoline (6.6 ml, 0.067 mol) in water (27 ml). The resulting solution gave way to a pale grey suspension. This was stirred and treated dropwise with ethylene sulfide (4.0 ml, 0.067 mol). After ca. 1 hour, a solution developed which slowly reverted to a milky white suspension. After 2 hours, the solid was filtered off, washed with water, suspended in $H_2O$ (50 ml), and treated with excess hydrogen sulfide. The resulting black mixture was centrifuged and filtered to remove the silver

sulfide. The water-white filtrate was evaporated under vacuum to provide the title compound (2.15 g) as an oil.
NMR ($D_2O$) δ 2.93 (s, $CH_3$), 3.18 (t, J=7 Hz, $SCH_2$), 4.83 (t, J=7 Hz, $NCH_2$), 7.8-8.9 (m, 4 ArH).

EXAMPLE 16

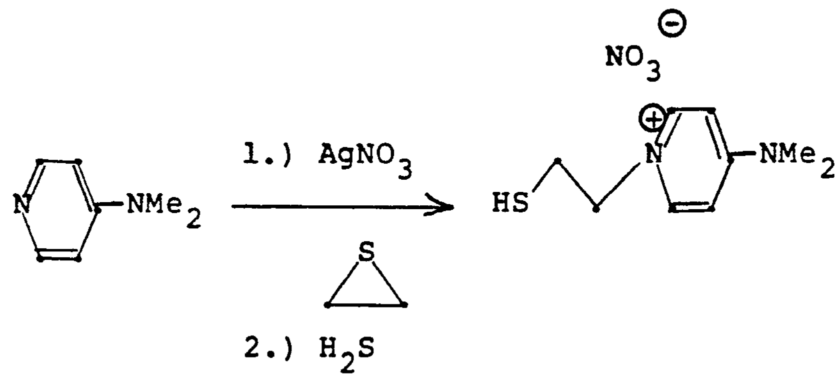


4-(Dimethylamino)-1-(2-mercaptoethyl)pyridinium nitrate

Ethylene sulfide (5.0 ml, 0.084 mol) and a solution of silver nitrate (14.27 g, 0.084 mol) in water (75 ml) were added slowly and concurrently to an ice-cold, stirring solution of 4-(dimethylamino) pyridine (10.26 g, 0.084 mol) in water (150 ml). The resulting mixture was stirred at room temperature for 2 hours, then treated with excess hydrogen sulfide. The silver sulfide was removed by filtration through a pad of celite. The filtrate was evaporated under vacuum to an oil which deposited crystals on standing overnight. The crystals (4-dimethylaminopyridinium nitrate by NMR) were filtered off with the aid of acetone and the filtrate was evaporated under vacuum to provide the title compound (11.88 g) as a yellow solid.

NMR ($D_2O$) $\delta$ 3.07 (t, J=7 Hz, $SCH_2$), 3.23 (s, $N(CH_3)_2$), 4.37 (t, J=7 Hz, $NCH_2$), 6.95 (d, J=8 Hz, 2 ArH), 8.10 (d, J=8 Hz, 2 ArH).

## EXAMPLE 17

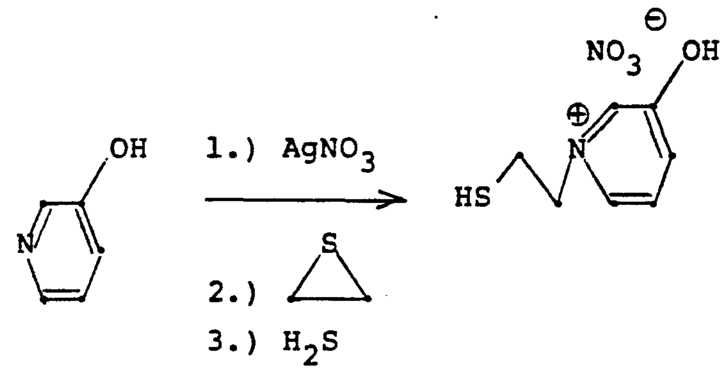


3-Hydroxy-1-(2-mercaptoethyl)pyridinium nitrate

A solution of silver nitrate (1.70 g, 10 mmol) in water (5 ml) was added dropwise over 3 minutes to an ice-cold, stirring suspension of 3-hydroxypyridine (0.95 g, 10 mmol) in water (25 ml). After sitrring an additional 5 minutes at 5°C, the mixture was treated dropwise over 3 minutes with ethylene sulfide (0.60 ml, 10 mmol). The resulting suspension was stirred in the cold and under a nitrogen atmosphere for 5.5 hours. The solid portion was collected by filtration, washed with water and acetone, and dried under vacuum to give a pale yellow powder (1.83 g).

The powder was suspended in water (25 ml), cooled in an ice-bath, and stirred rapidly while hydrogen sulfide was bubbled in over 15 minutes. After stirring an additional 5 minutes, the mixture was filtered through a celite pad to remove silver sulfide and the filtrate was evaporated under vacuum to provide the title compound (0.85 g) as a pale yellow oil.

NMR ($D_2O$) δ 3.15 (t, J=6.4 Hz, $SCH_2$), 4.72 (t, J=6.4 Hz, $NCH_2$), 7.94 and 8.42 (two m, ArH); NMR ($D_2O$ + $NaHCO_3$) δ 3.11 (t, J=6.6 Hz, $SCH_2$), 4.52 (t, J=6.6 Hz, $NCH_2$), 7.54 and 7.82 (two m, ArH).

EXAMPLE 18

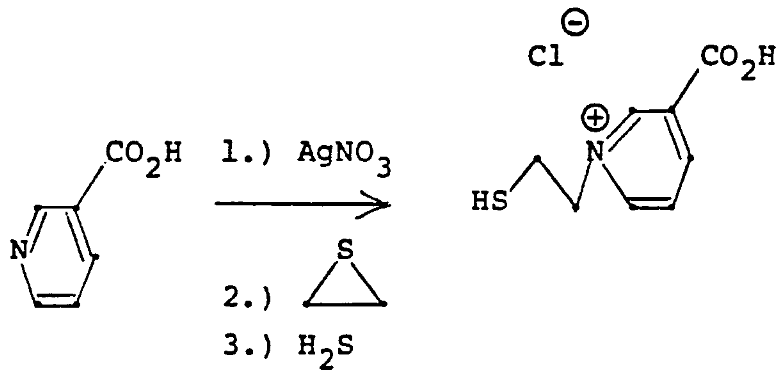


3-Carboxy-1-(2-mercaptoethyl)pyridinium chloride

Silver nitrate (4.14 g, 24.4 mmol) was added to a solution of nicotinic acid (3.00 g, 24.4 mmol) in water (200 ml) to give a white precipitate. The mixture was stirred and treated dropwise with ethylene sulfide (1.45 ml, 24.4 mmol). The resulting yellow mixture was stirred at room temperature for 65 hours, then filtered to remove the solid portion which was washed with water (~15 ml), ethanol (~20 ml) and diethyl ether, and dried under vacuum to provide the intermediate silver mercaptide (4.60 g).

A portion (2.14 g) of the silver salt was suspended in water (25 ml) and the mixture was stirred rapidly with ice-bath cooling while hydrogen sulfide was bubbled in over 5 minutes. After stirring an additional 10 minutes at 0°, the black

mixture was filtered through a celite pad to remove the silver sulfide. The colorless filtrate was concentrated under vacuum to ca. 9 ml and added to a column of Dowex 50W-X4 resin (hydrogen form) which was eluted with water, 1N hydrochloric acid, and 6N hydrochloric acid in a cold room. The product containing fractions were located in the 6N HCl eluant by UV, combined, and evaporated under vacuum to an oil containing crystals. This material was triturated with acetone to provide the title compound (153 mg) as a white solid.

NMR ($D_2C$) $\delta$ 3.04 (t, J=6.4 Hz, $SCH_2$), 4.72 (t, J=6.4 Hz, $NCH_2$), 8.88 (m, ArH), 8.91 (m, 2ArH), 9.28 (s, ArH).

EXAMPLE 19

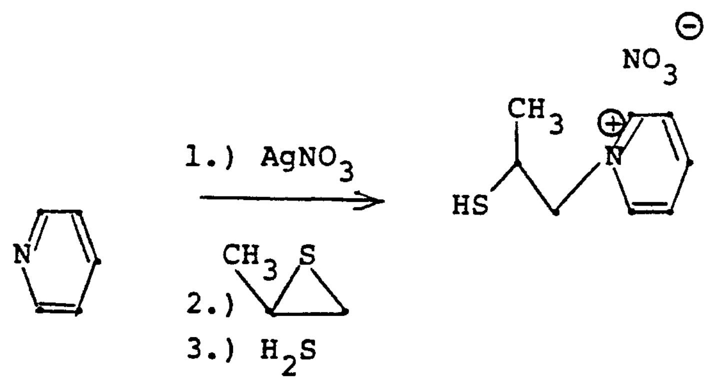


1-(2-Mercaptopropyl)pyridinium nitrate

A solution of silver nitrate (34.36 g, 0.202 mol) in water (80 ml) was added to a solution of pyridine (16.8 g, 0.212 mol) in water (60 ml). A white precipitate formed. The mixture was diluted with water to ca. 600 ml, stirred, cooled in an ice-bath, and treated with propylene sulfide (15.00

g, 0.202 mol) and additional pyridine (50.4 g, 0.636 mol). The resulting solution was stirred at room temperature for 3 hours then evaporated under vacuum to an oil. The oil was dissolved in water (100 ml) and treated with excess hydrogen sulfide. The resulting black mixture was centrifuged and filtered to remove the silver sulfide, and the filtrate was evaporated under vacuum to afford the crude product as a pale yellow oil (25.6 g). A portion of the crude product was dissolved in methanol, dried with calciuim sulfate, filtered, and evaporated under vacuum to afford the title compound as a clear, colorless oil.

NMR (DMSO-$d_6$) $\delta$ 1.36 (d, J=7 Hz, $C\underline{H}_3CH$), 2.88 (d, J=9 Hz, SH), 3.4-3.9 (m, CH), 4.57 (dd, J=8 and 13 Hz, NC$\underline{Ha}$Hb), 4.93 (dd, J=6 and 13 Hz, NCHa$\underline{Hb}$), 8.0-9.3 (m, ArH).

EXAMPLE 20

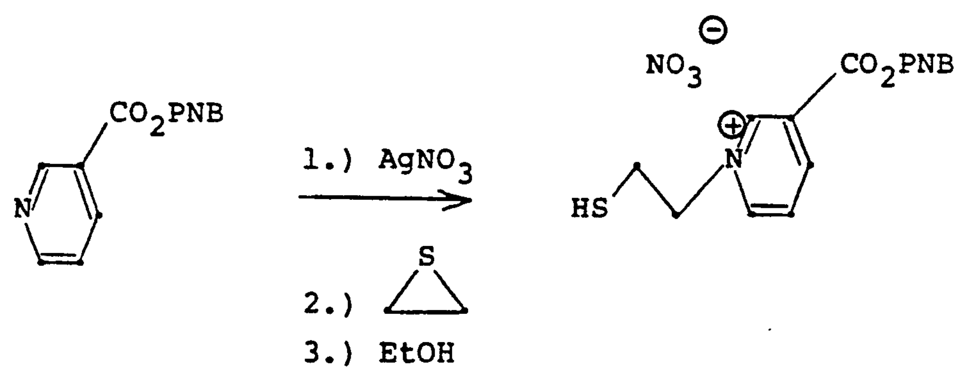


1-(2-Mercaptoethyl)-3-(p-nitrobenzyloxycarbonyl) pyridinium nitrate

A solution of silver nitrate (1.39 g, 8.19 mmol) in acetonitrile (10 ml) was added to a solution

of p-nitrobenzyl nicotinate (2.00 g, 8.19 mmol) in acetonitrile (50 ml). A tan precipitate formed. The mixture was stirred at room temperature and treated with ethylene sulfide (0.49 ml, 8.19 mmol). After 1.5 hours, the mixture was filtered to remove the precipitate which dried under vacuum to an off-white solid. This material was suspended in ethanol (75 ml), treated with hydrogen sulfide over 5 minutes, and stirred an additional 5 minutes at room temperature. The mixture was centrifuged and filtered to remove the silver sulfide. The filtrate, on standing overnight at room temperature, desposited the title compound (0.30 g) as fine white needles. NMR (DMSO-$d_6$)$\delta$ 2.72 (t, J=7 Hz, SH), 3.15 (~q, J= 7 Hz, $SCH_2$), 4.90 (t, J=7 Hz, $NCH_2$), 5.66 (s, $CH_2Ar$), 7.87 (d, J=8 Hz, 2 ArH), 8.33 (d, J=8 Hz, ArH), 8.38 (m, ArH), 9.14 (d, J=8 Hz, ArH), 9.31 (d, J=6 Hz, ArH), 9.70 (s, ArH).

## EXAMPLE 21

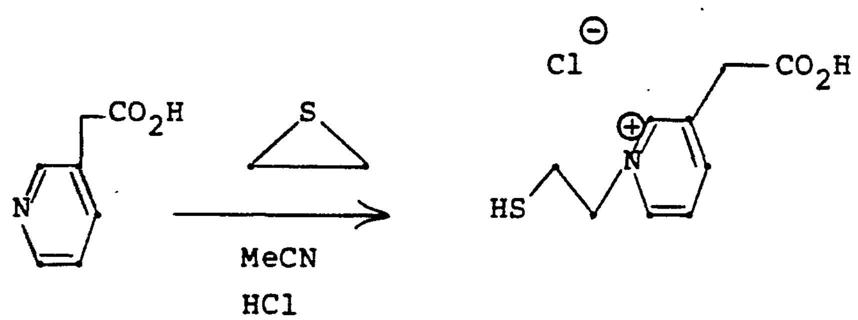


3-Carboxymethyl-1-(2-mercaptoethyl)pyridinium chloride

A mixture of 3-pyridylacetic acid (1.00 g, 7.3 mmol), 3-pyridylacetic acid hydrochloride (0.63 g, 3.65 mmol), ethylene sulfide (0.44 ml, 7.4 mmol)

and acetonitrile (14 ml) was heated at reflux overnight. After cooling to room temperature, the lower, oily phase of the biphasic reaction mixture was separated and lyophilized from water (10 ml) to give the title compound (0.44 g) as an oil. NMR ($D_2O$) $\delta$ 3.18 (m, $SCH_2$), 3.96 (s, $CH_2CO_2$), 4.80 (m, $NCH_2$), 8.08 (m, ArH), 8.54 (m, ArH), 8.84 (m, 2 ArH).

EXAMPLE 22

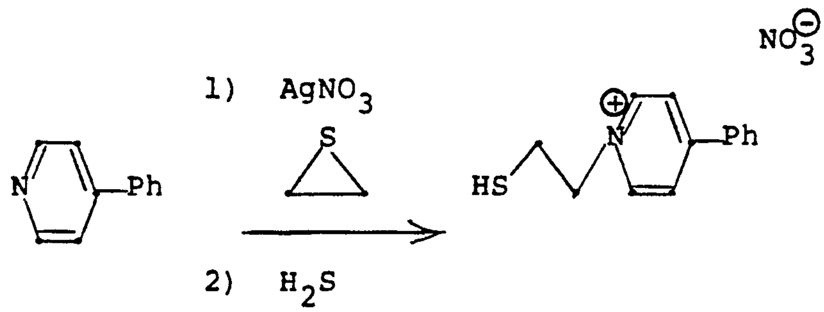


1-(2-Mercaptoethyl)-4-phenylpyridinium nitrate

4-Phenylpyridine (776 mg, 5 mmol) was added at once to a solution of silver nitrate (850 mg, 5 mmol) in acetonitrile (5 ml). The resulting suspension was stirred at room temperature under a nitrogen atmosphere while ethylene sulfide (0.357 ml, 5 mmol) was added dropwise over 6 minutes. The mixture became warm, yellowed, and turned to a thick paste. After stirring an additional one hour at room temperature, the mixture was centrifuged to remove the solid which was washed with acetonitrile and ethyl ether and dried under vacuum to a yellow powder.

A suspension of the powder in water (25 ml) was stirred vigorously and treated with hydrogen sulfide over 5 minutes. After stirring an additional 5 minutes, the mixture was filtered through a celite pad to remove silver sulfide and the filtrate was evaporated under vacuum to a pale yellow gum. The gum was triturated with ethyl ether, dissolved in isopropanol and precipitated with ether, and dissolved in ethanol and evaporated under vacuum to give the title compound (983 mg) as a pale tan solid. IR (Nujol) $\nu$ max 3415 (br), 1635, 1350 (br) $cm^{-1}$; UV ($H_2O$) $\lambda$ max 296 nm ($\epsilon$19,890); NMR ($D_2O$) $\delta$ 3.19 (t, J=6.3 Hz, $SCH_2$), 4.76 (t, J=6.3 Hz, $NCH_2$), 7.65 (m, 2 phenyl H), 7.9 (m, 3 phenyl H), 8.25 (d, J=7.0 Hz, 2 pyridyl H), 8.81 (d, J=7.0 Hz, 2 pyridyl H).

EXAMPLE 23

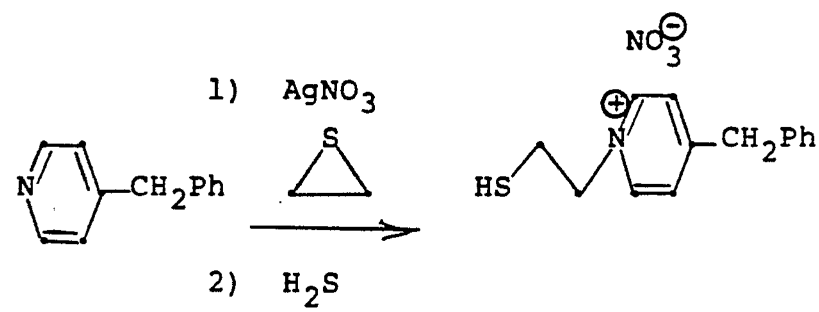


4-Benzyl-1-(2-mercaptoethyl)pyridinium nitrate

A solution of 4-benzylpyridine (846 mg, 5 mmol) in acetonitrile (1 ml) was added to a solution of silver nitrate (850 mg, 5 mmol) in acetonitrile (4 ml). The resulting solution was stirred under a nitrogen atmosphere at room temperature while

ethylene sulfide (0.357 ml, 5 mmol) was added dropwise over 5 minutes. The mixture warmed, turned pale yellow, and deposited a precipitate. After stirring an additional 60 minutes at room temperature, the mixture was separated into a solvent phase and an insoluble gum. The gum was triturated twice with acetonitrile and twice with ethanol to provide a pale yellow solid. This material was taken up in water (25 ml) to give a gummy mixture which was treated with excess hydrogen sulfide while scratching vigorously with a spatula. After several minutes, a black, stirrable solid formed. The mixture was filtered through a celite pad and the filtrate was evaporated under vaccum to a clear gum. This material was triturated with ethyl ether, dissolved in isopropanol and precipitated with ether, and twice dissolved in anhydrous ethanol and evaporated under vacuum to afford the title compound (479 mg) as a clear gum.

IR (Nujol) $\nu$ max 3440 (br), 1635, 1345 (br) $cm^{-1}$;
UV ($H_2O$) $\lambda$ max 255 nm ($\epsilon$5370);
NMR ($D_2O$) $\delta$ 3.12 (t, J=6.4 Hz, $SCH_2$), 4.34 (s, $CH_2Ph$), 4.71 (t, J=6.4 Hz, $NCH_2$), 7.40 (m, 5 phenyl H), 7.91 (d, J=6.8 Hz, 2 pyridyl H), 8.71 (d, J=6.8 Hz, 2 pyridyl H).

EXAMPLE 24

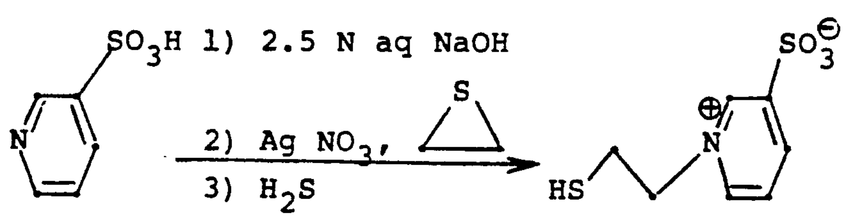

1-(2-Mercaptoethyl)pyridinium-3-sulfonate

A stirred solution of 3-pyridinesulfonic acid (2.00 g, 12.6 mmol) in water (20 ml) cooled in an ice-water bath was treated with a solution of 2.5 N sodium hydroxide (4.0 ml, 10 mmol) to give a solution of pH 5. The cold solution was treated with a solution of silver nitrate (2.13 g, 12.6 mmol) in water (5 ml) followed by ethylene sulfide (0.86 ml, 14.5 mmol). The resulting suspension was stirred at ice bath temperature 1.5 hour. The solid portion was collected by filtration, washed with water, and dried under vacuum to give a pale yellow powder (6.46 g).

The powder was suspended in water (20 ml) and stirred rapidly while hydrogen sulfide was bubbled in over 5 minutes. After stirring an additional 10 minutes, the mixture was filtered through a celite pad to remove silver sulfide and the filtrate was evaporated under vacuum to provide the title compound (1.78 g) as a clear colorless oil. NMR ($D_2O$) $\delta$ 3.22 (t, J=6 Hz, $SCH_2$), 4.90 (t, J=6 Hz, $NCH_2$), 8.26 (dd, J=7, 8 Hz, ArH), 8.90 (d, J=8 Hz, ArH), 9.05 (d, J=7 Hz, ArH), 9.31 (s, ArH).

EXAMPLE 25

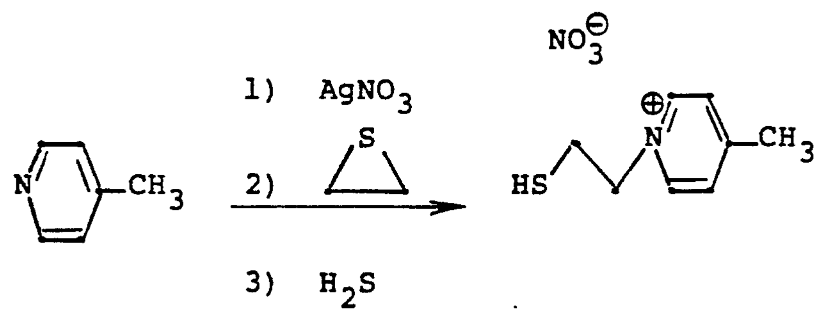

4-Methyl-1-(2-mercaptoethyl)pyridinium nitrate

A solution of silver nitrate (3.49 g, 20.6 mmol) in water (20 ml) was added to a stirred solution of 4-picoline (2.0 ml, 20.6 mmol) in water (20 ml). The resulting white suspension was cooled in an ice-water bath and treated with ethylene sulfide (1.5 ml, 25 mmol). The reaction was stirred at ice bath temperature for 1.8 hour. The resulting solution was rapidly stirred while hydrogen sulfide was bubbled in over 5 minutes. After stirring an additional 5 minutes, the mixture was filtered through a celite pad to remove silver sulfide and the filtrate evaporated under vacuum to provide the title compound (3.41 g) as a colorless oil.
NMR ($D_2O$) $\delta$ 2.67 (s, $CH_3$), 3.14 (t, J=6.4 Hz, $SCH_2$), 4.71 (t, J=6.4 Hz, $NCH_2$), 7.91 (d, J=6.4 Hz, 2ArH), 8.68 (d, J=6.4 Hz, 2-ArH).

EXAMPLE 26

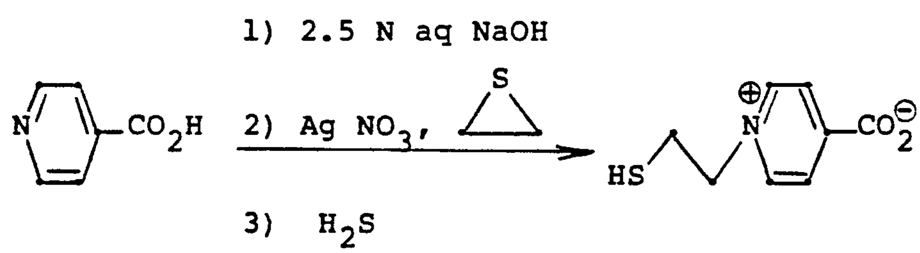


1-(2-Mercaptoethyl)pyridinium-4-carboxylate

A stirred suspension of isonicotinic acid (2.00 g, 16.2 mmol) in water (40 ml) was treated with a solution of 2.5N sodium hydroxide (4.9 ml, 12 mmol) to give a solution of pH 6. The solution was cooled in an ice bath and treated with a solution of silver nitrate (2.76 g, 16.2 mmol) in water (5 ml). The

resulting white suspension was treated with ethylene sulfide (1.06 ml, 17.8 mmol) added dropwise over 3 minutes. The resulting yellow suspension was stirred at ice bath temperature 1 hour. The solid portion was collected by filtration and washed with water to give a pale yellow cake.

The cake was suspended in water (25 ml) and stirred rapidly while hydrogen sulfide was bubbled in over 5 minutes. After stirring an additional 5 minutes, the mixture was filtered through a celite pad to remove silver sulfide and the filtrate evaporated under vacuum to provide the title compound (1.57 g) as a pale yellow solid.
NMR ($D_2O$) $\delta$ 3.20 (t, J=6 Hz, $SCH_2$), 4.86 (t, J=6 Hz, $NCH_2$), 8.37 (d, J=8 Hz, 2ArH), 9.00 (d, J=8 Hz, 2ArH).

EXAMPLE 27

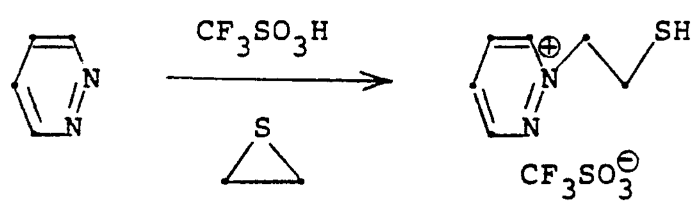


1-(2-Mercaptoethyl)pyridazinium triflate

At 0°C, under $N_2$ atmosphere, pyridazine (0.73 ml, 10 mmol) was treated with $CF_3SO_3H$ (0.44 ml, 5 mmol) and ethylene sulfide (0.30 ml, 5 mmol). The suspension was then heated at 55°C overnight. After cooling to room temperature, the mixture was extracted with 3 X 10 ml ether. The oil residue was dissolved in acetone and filtered from insoluble material. The filtrate was evaporated *in vacuo* to give product as deep orange oil.

NMR (acetone-$d_6$) $\delta$ 2.1-3.8 (m, $SCH_2$), 5.2 (t, $NCH_2$) 8.45 (t, pyridazinyl-H), 8.6 (m, pyridazinyl-H), 9.36 (dq, pyridazimyl-H), 9.97 (dt, pyridazinyl-H).

EXAMPLE 28

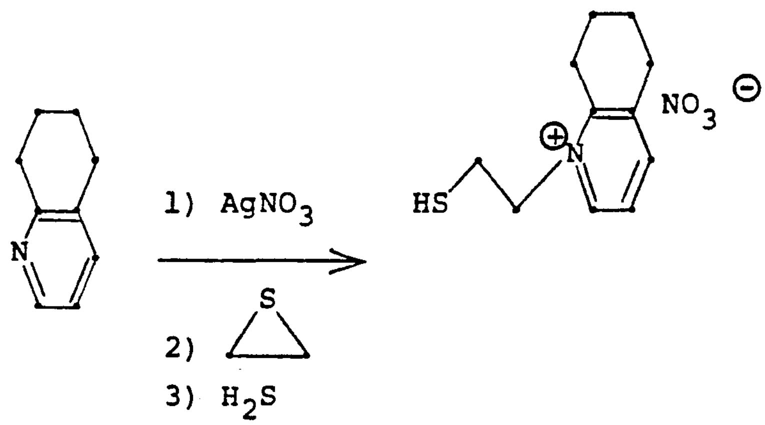


2,3-Cyclohexeno-1-(2-mercaptoethyl)pyridinium nitrate

2,3-Cyclohexenopyridine (0.65 ml, 5 mmol) was added to a solution of silver nitrate (850 mg, 5 mmol) in anhydrous aretonitrite (5 ml). The resulting solution was stirred at room temperature under a nitrogen atmosphere while ethylene sulfide (0.357 ml, 5 mmol) was added dropwise over 4 minutes. The mixture became cloudy and warmed during the addition. Further stirring at room temperature gave a clear solution. After 2 hours, the solution was evaporated under vacuum to a yellow foam which was triturated with ethyl ether to give a yellow powder.

The powder was suspended in water (25 ml) and stirred while hydrogen sulfide was bubbled in over 10 minutes. After aging for 5 minutes, the

mixture was filtered through a celite pad to remove the silver sulfide. The filtrate was evaporated under vacuum to a semi-solid which was triturated with several portions of isopropanol and dried under vacuum to afford the title compound (233 mg) as a white solid.

IR(Nujol) $\nu$max 3420 (br), 1612, 1360 (br), 1330 (br);
UV ($H_2O$) $\lambda$max 277 nm ($\epsilon$ 6530);
NMR ($D_2O$) $\delta$ 1.78-2.10 (m, $CH_2CH_2$), 3.01 (t, J=6.4 Hz, pyridyl-$CH_2$, 3.10 (t, J=6.9 Hz, $SCH_2$), 3.16 (t, J=6.4 Hz, pyridyl-$CH_2$), 4.72 (t, J=6.9 Hz, $NCH_2$), 7.76 (dd, J=6 and 8 Hz, pyridyl-H), 8.23 (d, J=8 Hz, pyridyl-H), 8.62 (d, J=8 Hz, pyridyl-H).

## EXAMPLE 29

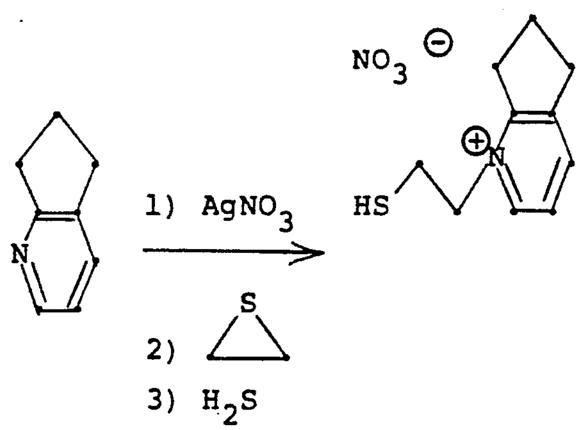


2,3-Cyclopenteno-1-(2-mercaptoethyl) pyridinium nitrate

Ethylene sulfide (0.060 ml, 1 mmol) was added dropwise to a strring solution of 2,3-cyclopentenopyridine (0.117 ml, 1 mmol) and silver nitrate (170 mg, 1 mmol) in anhydrous

acetonitrite (1.0 ml) at room temperature and under a nitrogen atmosphere. A creamy, pale yellow gum separated during the addition. The mixture was stirred at room temperature for 60 minutes to give a fine, cream colored solid which was collected by centrifugation, washed with acetonitrite, and dried under vaccum.

The solid was taken up in water (5 ml) and stirred rapidly while hydrogen sulfide was bubbled in over 5 minutes. After 5 more minutes, the mixture was filtered through a celite pad and the filtrate was evaporated under vacuum to provide the title compound (172 mg) as a white semi-solid.

IR(Nujol)$\nu$max 1621, 1345(br)Cm$^{-1}$;
UV ($H_2O$)$\lambda$max 277 nm ($\epsilon$ 6560);
NMR ($D_2O$)$\delta$ 2.33 (p, J=7.7 Hz,$CH_2$), 3.12 (t, J=6.6 Hz, $SCH_2$), 3.20 (t, J=7.7 Hz, pyridyl-$CH_2$), 3.41 (t, J=7.7 Hz, pyridyl-$CH_2$), 4.68 (t, J=6.6 Hz, $NCH_2$), 7.77 (dd, J=6 and 8 Hz, pyridyl-H), 8.30 (d, J=8 Hz, pyridyl-H), 8.53(d, J=6 Hz, pyridyl-H).

EXAMPLE 30

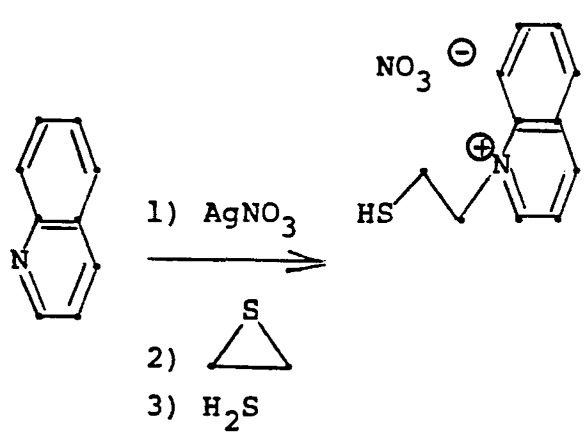

1-(2-Mercaptoethyl)quinolinium nitrate

Ethylene sulfide (0.357 ml, 5 mmol) was added dropwise over 6 minutes to a mixture of quinoline (0.591 ml, 5 mmol) and silver nitrate (850 mg, 5 mmol) in acetonitrile (5 ml) which was stirred at room temperature under a nitrogen atmosphere. After stirring for 60 minutes, the mixture was centrifuged to remove the yellow precipitate which was washed with acetointrile and dried under vacuum to give a yellow powder.

A suspension of the powder in water (25 ml) was stirred rapidly at room temperature while hydrogen sulfide was bubbled in for 10 minutes. After aging 5 minutes, the mixture was filtered and the filtrate was evaporated under vacuum to a pale yellow oil. This material was triturated with isopropanol and dried under vacuum to give the title compound (326 mg) as a white powder.

IR(Nujol) $\nu$max 1524, 1343 $cm^{-1}$;
UV ($H_2O$) $\lambda$max 317 nm ($\epsilon$ 7740);
NMR ($D_2O$) $\delta$ 3.27 (t, J=6.4 Hz, $SCH_2$), 5.24 (t, J=6.4 Hz, $NCH_2$), 8.5 (m, 2 quinolinium-H), 8.27 (m, quinolinium-H), 8.42 (m, 2 quinolinium-H, 9.17 (m, quinolinium-H, 9.31 (m, quinolinium-H).

EXAMPLE 31

Step A:

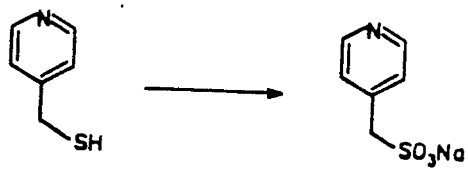

Sodium 4-pyridinemethanesulfonate

A solution of 4-mercaptomethylpyridine (2.20 g, 17.6 mmol) in acetonitrile (8.0 ml) was added to a stirred solution of 40% peracetic acid-acetic acid (25 ml) at ice bath temperature. After addition the solution was removed from the ice bath and stirred at room temperature overnight. The solution was concentrated under vacuum to a white solid which was dissolved in water (1.5 ml) and adjusted to pH 9 with 2.5N aqueous sodium hydroxide. The resulting white suspension was filtered and the filtrate evaporated under vacuum to afford the title compound as a white solid (2.32 g).

NMR ($D_2O$) $\delta$ 4.24 (s, $CH_2$), 7.50 (d, J-5.4 Hz, ArH), 8.53 (d, J=5.4 Hz, ArH).

Step B:

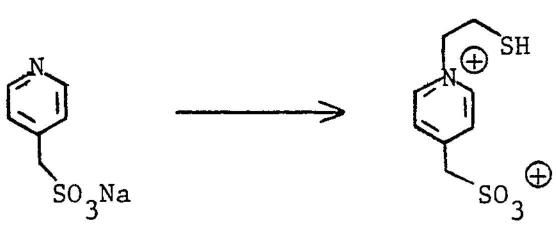

[1-(2-Mercaptoethyl)-4-pyridinio]methylsulfonate

A solution of silver nitrate (1.31 g, 7.68 mmol) in water (4 ml) was added to a stirred solution of sodium 4-pyridylmethylsulfonate (1.50 g, 7.68 mmol) in water (15 ml). The resulting hazy solution was treated with ethylene sulfide (0.51 ml, 8.58 mmol) added dropwise over 2 minutes. The resulting gummy mixture was let stand at room temperature 30 minutes and the supernatant decanted. The residue was mixed with water (10 ml) and vigorously bubbled with hydrogen sulfide for 10 minutes. The mixture was vigorously stirred an additional 1 hour and the silver sulfide precipitate removed by centrifugation. The supernatant was concentrated under vacuum to ca. 5 ml and charged onto a column of Dowex 50W-X4 (hydrogen form, 200-400 mesh, 1.5 x 33 cm). The column was eluted with water at 6.0 ml fractions/2.0 minutes. Fractions 19 to 27 combined and evaporated under vacuum to an off-white solid. The solid was mixed with a few mls of methanol and filtered to afford the title compound as an off-white solid (303 mg).

NMR ($D_2O$) $\delta$ 3.16 (t, J=6.4 Hz, $SCH_2$), 4.52 (s, $CH_2SO_3^-$), 4.79 (t, J=6.4 Hz, $NCH_2$), 8.12 (d, J=6.2 Hz, ArH), 8.86 (d, J=6.2 Hz, ArH).

Anal. Calc'd for $C_8H_{11}NO_3S_2$:
C, 41.18; H, 4.75; N, 6.00; S, 27.48

Found: C, 41.32; H, 4.77; N, 6.07; S, 27.17.

Step C:

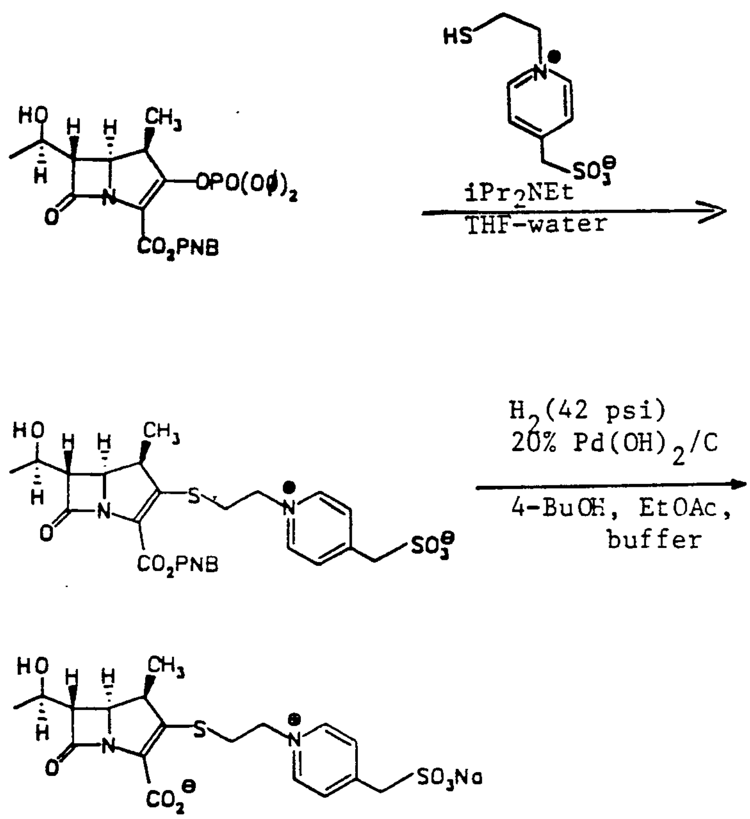


Sodium (5R,6S)-6-[(1R)-hydroxyethyl]-2-(2-(4-sulfonatomethyl)-1-pyridinium)ethyl)thio-1(R)-methyl-carbapen-2-em-3-carboxylate

A solution of p-nitrobenzyl (5R,6S)-2-(diphenylphosphono)oxy-6-[(1R)-hydroxyethyl]-1(R)-methyl-carba-2-em-3-carboxylate (200 mg, 0.336 mmol) and [1-(2-mercaptoethyl-4-pyridinio]methylsulfonate (82 mg, 0.353 mmol) in tetrahydrofuran (1.5 ml) and water (0.5 ml) was treated at room temperature with N,N-diisopropylethylamine (0.062 ml, 0.353 mmol). After stirring 11 minutes, the reaction solution was diluted with n-butanol (6.7 ml), ethyl acetate (3.4 ml), water (6.7 ml), and 0.5M pH 6.8 N-methylmorpholine-hydrochloric acid buffer (3.4 ml), mixed

with 20% palladium hydroxide on carbon (75 mg), and hydrogenated on a Parr shaker at 42 psi for 75 minutes. The catalyst was removed by filtration through a celite pad, and the filtrate washed with methylene chloride. The aqueous phase was concentrated under vacuum to ca. 10 ml and charged onto a column of Dowex 50W-X4 (sodium form, 200-400 mesh, 1.5 x 33 cm). The column was eluted with water in the cold room (4°) at 4.0 ml fractions/2.0 minutes. Fractions 4 to 10 were combined and concentrated under vacuum to ca. 8 ml and lyophilized to powder. This material was chromatographed on four 1.0 mm 20 x 20 cm Analtech RPS-F plates using 2% ethanol-water as a developing solvent in a cold room (4°). The UV visible band on each plate at Rf 0.4-0.5 was removed and eluted with 4:1 acetonitrile-water. The eluant was washed with hexane, concentrated under vacuum to ca. 4 ml, filtered through an Acrodisc (Gelman, 0.45 micron CR) and lyophilized to afford the title compound (35.3 mg) as an amorphous white powder.

IR (Nujol) 3400 (br), 1740, 1640, 1593 $cm^{-1}$

UV (water) $\lambda$max 298 nm ($\epsilon$ 6,940), $\lambda$ max 260 nm, ($\epsilon$ 6,020), $\lambda$max 229 nm ($\epsilon$ 11,600).

NMR ($D_2O$) $\delta$ 1.14 (d, J=7.0 Hz, $C\underline{H}_3CH$), 1.28 (d, J=6.4 Hz, $C\underline{H}_3CHOH$), 3.22 (dq, J=7.0, 9.5 Hz, $CH_3C\underline{H}$), 3.29 (td, J=6.0, 15.2 Hz, $SC\underline{H}_aH_b$), 3.41 (dd, J=2.5, 6.4 Hz, H6), 3.56 (td, J=6.0, 15.2 Hz, $SCH_a\underline{H}_b$), 3.99 (dd, J=2.5, 9.5 Hz, H5), 4.29 ( p, J=6.4 Hz, $CH_3C\underline{H}OH$), 4.53 (s, $CH_2SO_3^-$), 3.8 (m, $NCH_2$), 8.11 (d, J=6.4 Hz, ArH), 8.83 (d, J=6.4 Hz, ArH).

EXAMPLE 32

Step A:

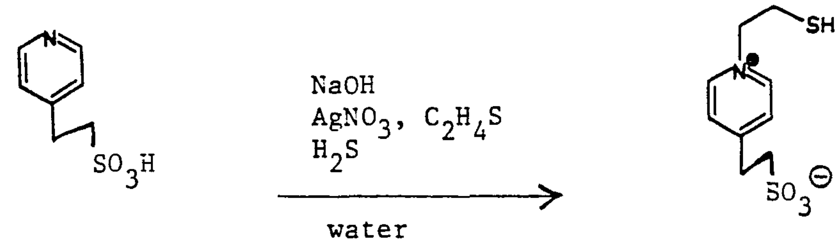


[1-(2-Mercaptoethyl)-4-pyridinio]-2-ethylsulfonate

A solution of silver nitrate (1.84 g, 10.6 mmol) in water (5 ml) was added to a stirred aqueous solution (20 ml) of 4-pyridine ethane sulfonic acid (2.00 g, 10.6 mmol) which had been adjusted to pH 7 by the addition of aqueous 2.5N sodium hydroxide (3.0 ml). The solution was cooled in an ice-water bath and ethylene sulfide (0.71 ml, 11.8 mmol) added. The resulting mixture was let stand 45 minutes at ice-bath temperature and the upper clear phase decanted. The gummy solid was mixed with water (20 ml) and vigorously bubbled with hydrogen sulfide for 15 minutes. After stirring an additional 20 minutes, the mixture was filtered through a celite pad to remove silver sulfide and the filtrate was evaporated under vacuum to provide crude title compound as a white semi-solid (1.00 g).

Step B:

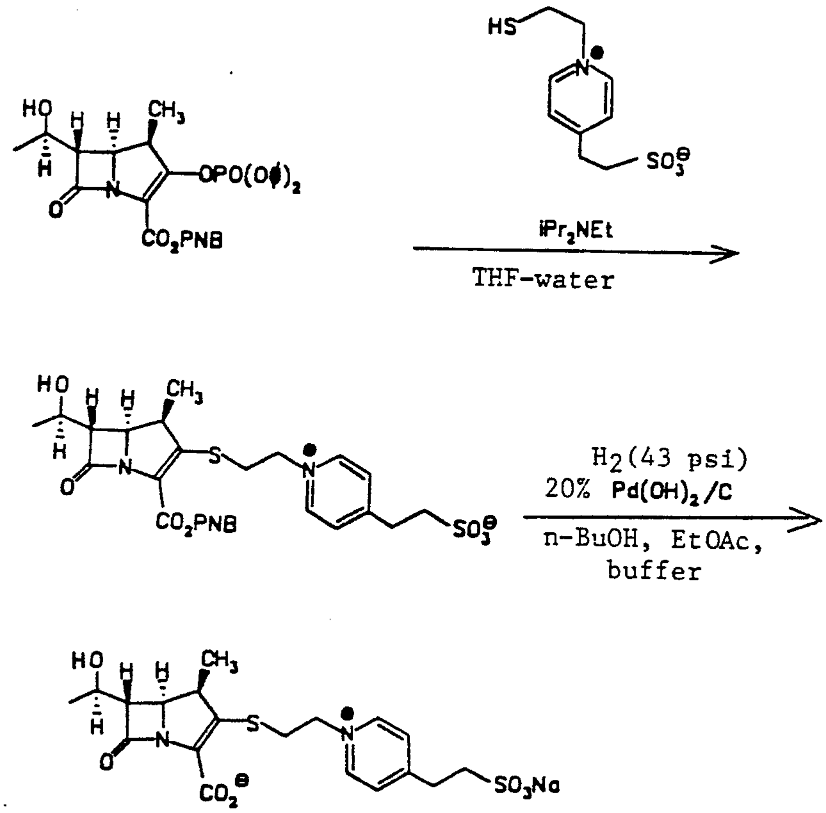


Sodium (5R,6S)-6-[(1R)-hydroxyethyl]-2-(2-(4-(2-sulfonatoethyl)-1-pyridinium)ethyl)thio-1(R)-methylcarbapen-2-em-3-carboxylate

A solution of p-nitrobenzyl (5R,6S)-2-(diphenylphosphono)oxy-6-[(1R)-hydroxyethyl]-carba-2-em-3-carboxylate (200 mg, 0.344 mmol) and [1-(2-mercaptoethyl)-4-pyridinio]-2-ethylsulfonate (83 mg, 0.336 mmol) in tetrahydrofuran (2.0 ml) and water (1.0 ml) was treated at room temperature with N,N-diisopropylethylamine (0.062 ml, 0.353 mmol). After stirring 15 minutes, the reaction solution was diluted with n-butanol (6.7 ml), ethyl acetate (3.4 ml), water (6.7 ml), and 0.5M pH 6.8 N-methylmorpholine-hydrochloric acid buffer (3.4 ml), mixed with 20% palladium hydroxide on carbon (75 mg), and

hydrogenated on a Parr shaker at 43 psi for 75 minutes. The catalyst was removed by filtration through a celite pad, and the filtrate washed with methylene chloride. The aqueous phase was concentrated under vacuum to ca. 8 ml and charged onto a column of Dowex 50W-X4 (sodium form, 200-400 mesh, 1.5 x 33 cm). The column was eluted with water in the cold room (4°) at 5.0 ml fractions/2.0 minutes. Fractions 4 to 10 were combined and concentrated under vacuum to ca. 13 ml and lyophilized to powder. This material was chromatographed on five 0.5 mm 20 x 20 cm Analtech RPS-F plates using 1% ethanol-water as a developing solvent in a cold room (4°). The major UV visible band on each plate at Rf 0.3-0.5 was removed and eluted with 4:1 acetonitrile-water. The eluant was washed with hexane, concentrated under vacuum to ca. 6 ml, filtered through an Acrodisc (Gelman, 0.45 micron CR) and lyophilized to afford the title compound (82.1 mg) as an amorphous white powder.

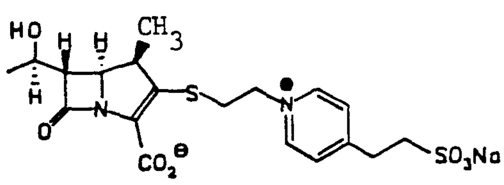

IR (Nujol) 3400 (br), 1740, 1635, 1590 $cm^{-1}$

UV (water) $\lambda$ max 294 nm ($\epsilon$ 7,200), $\lambda$ max 256 nm ($\epsilon$ 5,330), $\lambda$ max 224 ($\epsilon$ 10,400)

UV (water + $NH_2OH.HCl$ + $K_2HPO_4$) extinguished max 297 nm ($\epsilon$ 6,630)

NMR ($D_2O$) δ 1.13 (d, J=7.3 Hz, C$\underline{H}_3$CH), 1.28 (d, J=6.3 Hz, C$\underline{H}_3$CHOH), 3.1-3.7 (m, $SCH_2$, $CH_3C\underline{H}$, H6), 3.40 (s, $ArC\underline{H}_2C\underline{H}_2SO_3^-$), 3.94 (dd, J=2.4, 9.5 Hz, H5), 4.23 (~p, J=6.3 Hz, $CH_3C\underline{H}OH$), 8.01 (d, J=6.4 Hz, ArH), 8.73 (d, J=6.4 Hz, ArH).

The crude product was dissolved in water (ca. 3 ml) and charged onto a column of Dowex 50W-X4 (hydrogen form, 200-400 mesh, 1.5 x 30 cm). The column was eluted with water at room temperature at 3.5 ml fractions/2.0 minutes. Fractions 15 to 49 were combined and evaporated under vacuum to provide the title compound as a white solid (0.49 g).

NMR ($D_2O$) δ 3.15 (t, J=6.4 Hz, $SC\underline{H}_2$), 3.39 (s, $C\underline{H}_2C\underline{H}_2SO_3^-$), 4.75 (t, J=6.4 Hz, $NC\underline{H}_2$), 8.04 (d, J=6.8 Hz, ArH), 8.77 (d, J=6.8 Hz, ArH).

Anal. Calcd. for $C_9H_{13}NO_3S_2$:
C, 43,71; H, 5.30; N, 5.66; S, 25.93
Found: C, 43.43; H, 5.32; N, 5.41; S, 26.08.

EXAMPLE 33

Step A:

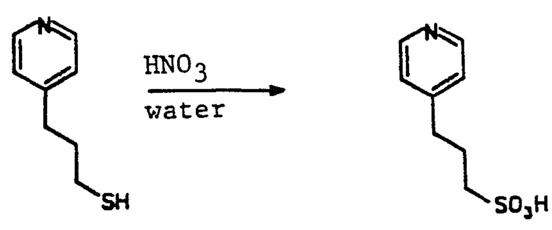


3-(4-Pyridyl)propanesulfonic acid

Concentrated nitric acid (6.0 ml, 96 mmol) was added dropwise to an ice-cold stirring solution of 3-(4-pyridyl)propanethiol (2.45 g, 16.0 mmol) in water (16 ml). After addition, the solution was allowed to warm to room temperature. The reaction flask was fitted with a reflux condenser and the solution heated to reflux. After ca. 10 minutes of

reflux rapid evolution of brown nitrogen oxides occurred. The solution was heated an additional 5 minutes and allowed to cool to room temperature. The solution was concentrated under water aspiration to ca. 3 ml and mixed with methanol (3 ml) followed by acetone (50 ml) to precipitate a white solid which was recovered by filtration. The recovered solid (1.95 g) was vacuum dried to afford the title compound.

NMR ($D_2O$) $\delta$ 2.19 (~p, J=7.5 Hz, $CH_2$-$C\underline{H}_2CH_2$), 2.96 (~t, J=7.7 Hz, $C\underline{H}_2SO_3$), 3.11 (~t, J=7.8 Hz, $C\underline{H}_2Ar$).

MS (FAB) $M^+$ 202, 155.

<u>Step B</u>:

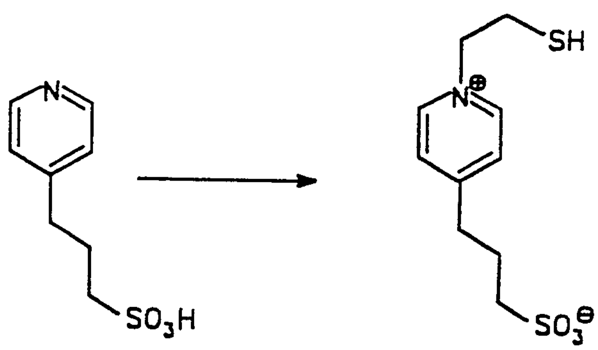

<u>[1-(2-Mercaptoethyl)-4-pyridinio]-3-propylsulfonate</u>

A solution of silver nitrate (633 mg, 3.73 mmol) in water (2.0 ml) was added to a stirred ice cold aqueous solution (8.4 ml) of 3-(4-pyridyl)-propanesulfonic acid previously neutralized to pH 7 with aqueous sodium hydroxide. The resulting hazy solution was treated with ethylene sulfide. A thick precipitate formed and after 20 minutes the upper clear phase was decanted and the gummy precipitate water washed. The gum was mixed with water (10 ml)

and vigorously bubbled with hydrogen sulfide for 5 minutes. After stirring an additional 50 minutes the mixture was centrifuged to remove silver sulfide and the supernatant adjusted to pH 7 with aqueous sodium hydroxide and concentrated to an oil. The crude product was dissolved in water (3 ml) and charged onto a column of Dowex 50W-X4 (hydrogen form, 200-400 mesh, 1.5 x 20 cm). The column was eluted with water at 3.5 ml fractions 11.5 minutes. Fractions 40 to 80 were concentrated under vacuum to afford the title compound (79 mg) as an oil.

NMR ($D_2O$) $\delta$ 2.14-2.29 (m, $CH_2C\underline{H}_2CH_2$), 2.95-3.02 (m, $C\underline{H}_2Ar$), 3.10-3.18 (m, $CH_2SO_3^{\ominus}$), 3.16 (t, J=6.4 Hz, $SCH_2$), 4.75 (t, J=6.4 Hz, $\overset{\oplus}{N}CH_2$), 8.00 (d, J=6.4 Hz, ArH), 8.76 (d, J=6.4 Hz, ArH).

Step C:

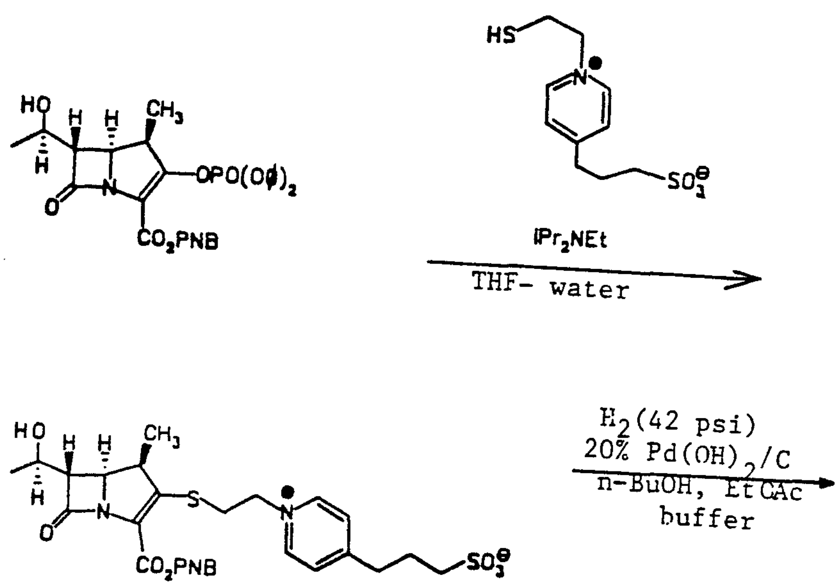

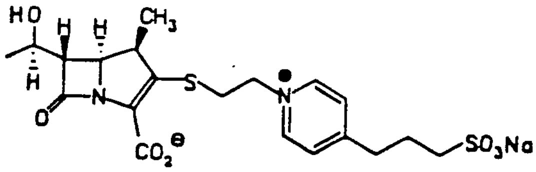

Sodium (5R,6S)-6-[(1R)-hydroxyethyl]-2-(2-(4-(3-sulfonatopropyl)-1-pyridinium)ethyl)thio-1(R)-methyl-carbapen-2-em-3-carboxylate

A solution of p-nitrobenzyl (5R,6S)-2-diphenylphosphono)oxy-6-[(1R)-hydroxyethyl]-1(R)-methyl-carba-2-em-3-carboxylate (61 mg, 0.101 mmol) and [1-(2-mercaptoethyl)-4-pyridinio]-3-propylsulfonate (29 mg, 0.111 mmol) in tetrahydrofuran (0.60 ml) and water (0.2 ml) was treated at room temperature with N,N-diisopropylethylamine (0.019 ml, 0.106 mmol). After stirring 10 minutes, the reaction solution was diluted with n-butanol (2.0 ml), ethyl acetate (1.0 ml), water (2.0 ml), and 0.5M pH 6.8 N-methylmorpholine-hydrochloric acid buffer (1.0 ml), mixed with 20% palladium hydroxide on carbon (25 mg), and hydrogenated on a Parr shaker at 42 psi for 75 minutes. The catalyst was removed by filtration through an Acrodisc (Gelman, 0.45 micron CR), and the filtrate washed with ethyl acetate and ether. The aqueous phase was concentrated under vacuum to ca. 5 ml and charged onto a column of Dowex 50W-X4 (sodium form, 200-400 mesh, 1.5 x 36 cm). The column was eluted with water in the cold room (4°) at 5.2 ml fractions/2.0 minutes. Fractions 5 to 10 were combined and concentrated under vacuum to ca. 5 ml and lyophilized to powder. This material was

chromatographed on two 1.0 mm 20 x 20 cm Analtech RPS-F plates using 3% ethanol-water as a developing solvent in a cold room (4°). The major UV visible band on each plate at Rf 0.3-0.4 was removed and eluted with 4:1 acetonitrile-water. The eluant was washed with hexane, concentrated under vacuum to ca. 4 ml, filtered through an Acrodisc (Gelman, 0.45 micron CR) and lyophilized to afford the title compound (21.8 mg) as an amorphous white powder.

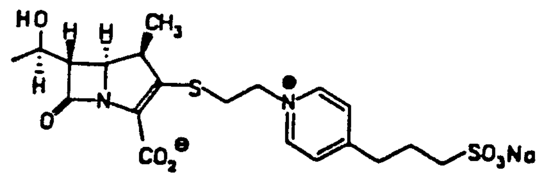

IR (Nujol) 3350, (br), 1742, 1628, 1592 $cm^{-1}$

UV (water) $\lambda$ max 296 nm ($\epsilon$ 6,720), $\lambda$ max 256 nm ($\epsilon$ 5,050), $\lambda$ max 225 ($\epsilon$ 10,000)

UV (water + $NH_2OH.HCl$ + $K_2HPO_4$) extinguished max 297 nm ($\epsilon$ 6,360)

NMR ($D_2O$) $\delta$ 1.12 (d, J=7.2 Hz, $C\underline{H}_3CH$), 1.28 (d, J=6.4 Hz, $C\underline{H}_3CHOH$), 2.21 (~p, J=8 Hz, $CH_2C\underline{H}_2CH_2$), 3.00 (~t, J=8 Hz, $ArC\underline{H}_2-$), 3.13 (~t, J=8 Hz, $CH_2SO_3^-$), 3.17 (dq, J=7.2, 9.5 Hz, $CH_3C\underline{H}$), 3.29 (td, J=5.2, 15.5 Hz, $SC\underline{H}_aH_b$), 3.39 (dd, J=2.7, 6.1 Hz, H6), 3.54 (ddd, J=4.9, 7.7, 15.5 Hz, $SCH_a\underline{H}_b$), 3.89 (dd, J=2.6, 9.5 Hz, H5), 4.23 (~p, J=6.4 Hz, $CH_3\underline{CH}OH$), 4.7 (m, $\overset{+}{N}CH_2$), 7.97 (d, J=6.6 Hz, ArH), 8.72 (d, J=6.6 Hz, ArH).

## EXAMPLE 34

By using the procedures of Examples 1-30, the following compounds are prepared:

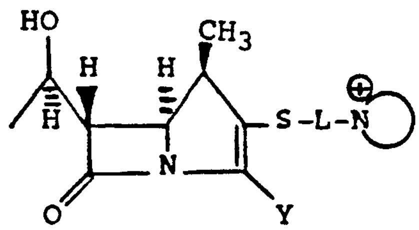

I

| Compound No. | L | 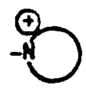 | Y |
|---|---|---|---|
| 1 | $-CH_2CH_2-$ | 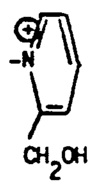 | $-CO_2^{\ominus}$ |
| 2 | $-CH_2CH_2-$ | 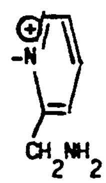 | $-CO_2^{\ominus}$ |

| Compound No. | L | 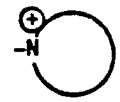 | Y |
|---|---|---|---|
| 3 | $-CH_2CH_2-$ | 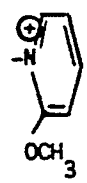 | $-CO_2^{\ominus}$ |
| 4 | $-CH_2CH_2-$ | 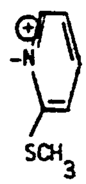 | $-CO_2^{\ominus}$ |
| 5 | $-CH_2CH_2-$ | 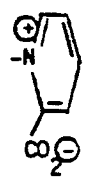 | $-CO_2Na$ |
| 6 | $-CH_2CH_2-$ | 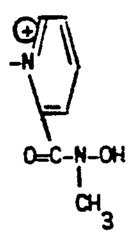 | $-CO_2^{\ominus}$ |
| 7 | $-CH_2CH_2-$ | 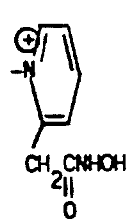 | $-CO_2^{\ominus}$ |

| Compound No. | L | 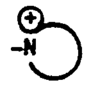 | Y |
| --- | --- | --- | --- |
| 8 | $-CH_2CH_2-$ | 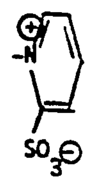 | $-CO_2Na$ |
| 9 | $-CH_2CH_2-$ | 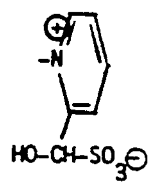 | $-CO_2Na$ |
| 10 | $-CH_2CH_2-$ | 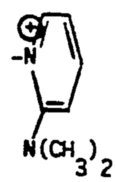 | $-CO_2^{\ominus}$ |
| 11 | $-CH_2CH_2-$ | 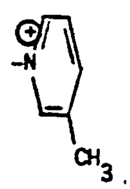 | $-CO_2^{\ominus}$ |
| 12 | $-CH_2CH_2-$ | 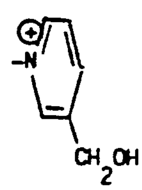 | $-CO_2^{\ominus}$ |
| 13 | $-CH_2CH_2-$ | 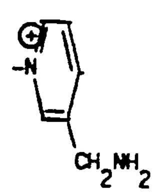 | $-CO_2^{\ominus}$ |

| Compound No. | L | 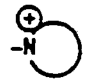 | Y |
|---|---|---|---|
| 14 | $-CH_2CH_2-$ | 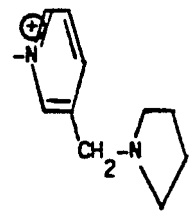 | $-CO_2^{\ominus}$ |
| 15 | $-CH_2CH_2-$ | 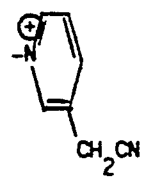 | $-CO_2^{\ominus}$ |
| 16 | $-CH(CH_3)CH_2-$ | 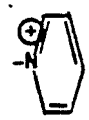 | $-CO_2^{\ominus}$ |
| 17 | $-CH_2CH_2-$ | 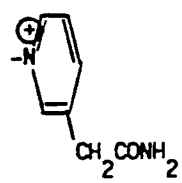 | $-CO_2^{\ominus}$ |
| 18 | $-CH_2CH_2-$ | 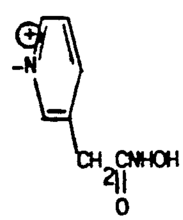 | $-CO_2^{\ominus}$ |
| 19 | $-CH_2CH_2-$ | 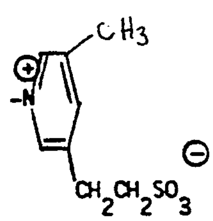 | $-CO_2Na$ |

| Compound No. | L | 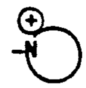 | Y |
|---|---|---|---|
| 20 | $-CH_2CH_2-$ | 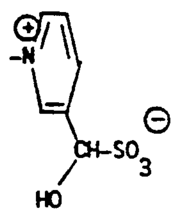 | $-CO_2Na$ |
| 21 | $-CH_2CH_2-$ | 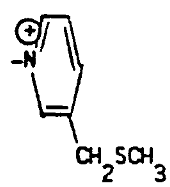 | $-CO_2^{\ominus}$ |
| 22 | $-CH_2CH_2-$ | 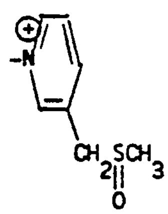 | $-CO_2^{\ominus}$ |
| 23 | $-CH_2CH_2-$ | 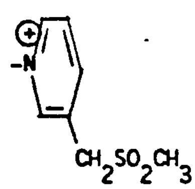 | $-CO_2^{\ominus}$ |
| 24 | $-CH_2CH_2-$ | 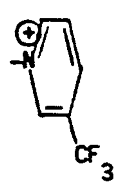 | $-CO_2^{\ominus}$ |
| 25 | $-CH_2CH_2-$ | 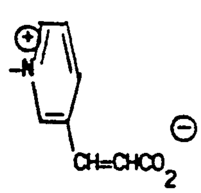 | $-CO_2Na$ |

| Compound No. | L | 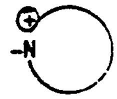 | Y |
|---|---|---|---|
| 26 | $-CH_2CH_2-$ | 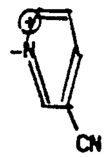 | $-CO_2^{\ominus}$ |
| 27 | $-CH_2CH_2-$ | 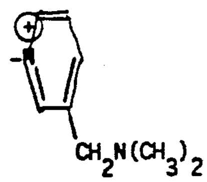 | $-CO_2^{\ominus}$ |
| 28 | $-CH_2CH_2-$ | 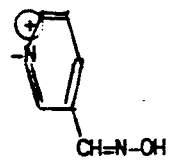 | $-CO_2^{\ominus}$ |
| 29 | $-CH_2CH_2-$ | 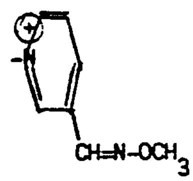 | $-CO_2^{\ominus}$ |
| 30 | $-CH_2CH_2-$ | 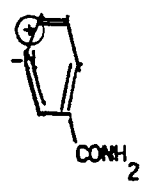 | $-CO_2^{\ominus}$ |
| 31 | $-CH_2CH_2-$ | 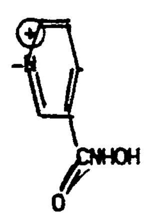 | $-CO_2^{\ominus}$ |

| Compound No. | L | 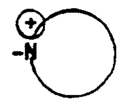 | Y |
|---|---|---|---|
| 32 | $-CH_2CH_2-$ | 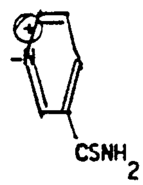 | $-CO_2^{\ominus}$ |
| 33 | $-CH_2CH_2-$ | 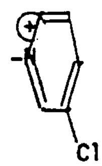 | $-CO_2^{\ominus}$ |
| 34 | $-CH_2CH_2-$ | 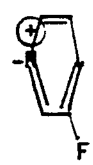 | $-CO_2^{\ominus}$ |
| 35 | $-CH_2CH_2-$ | 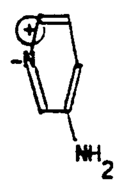 | $-CO_2^{\ominus}$ |
| 36 | $-CH_2CH_2-$ | 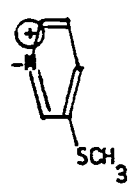 | $-CO_2^{\ominus}$ |
| 37 | $-CH_2CH_2-$ | 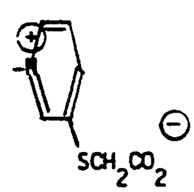 | $-CO_2Na$ |

| Compound No. | L | 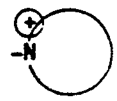 | Y |
|---|---|---|---|
| 38 | $-CH_2CH_2-$ | 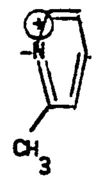 | $-CO_2^{\ominus}$ |
| 39 | $-CH_2CH_2-$ | 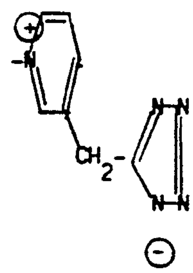 | $-CO_2Na$ |
| 40 | $-CH_2CH_2-$ | 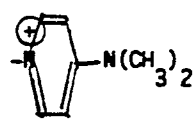 | $-CO_2^{\ominus}$ |
| 41 | $-CH_2CH_2-$ | 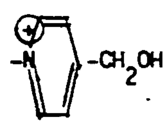 | $-CO_2^{\ominus}$ |
| 42 | $-CH_2CH_2-$ | 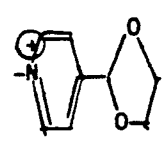 | $-CO_2^{\ominus}$ |
| 43 | $-CH_2CH_2-$ | 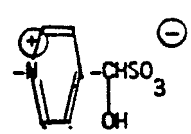 | $-CO_2Na$ |

| Compound No. | L | 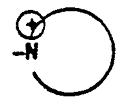 | Y |
|---|---|---|---|
| 44 | $-CH_2CH_2-$ | 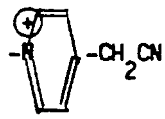 | $-CO_2^{\ominus}$ |
| 45 | $-CH_2CH_2-$ | 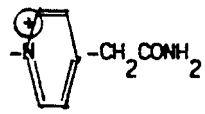 | $-CO_2^{\ominus}$ |
| 46 | $-CH_2CH_2-$ | 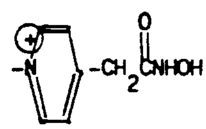 | $-CO_2^{\ominus}$ |
| 47 | $-CH_2CH_2-$ | 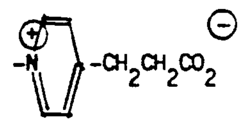 | $-CO_2Na$ |
| 48 | $-CH_2CH_2-$ | 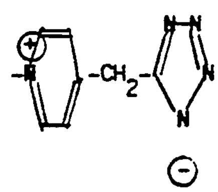 | $-CO_2Na$ |
| 49 | $-CH_2CH_2-$ | 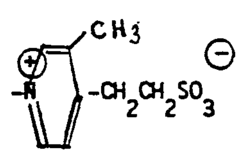 | $-CO_2Na$ |

| Compound No. | L | 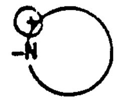 | Y |
|---|---|---|---|
| 50 | $-CH_2CH_2-$ | 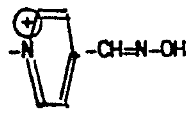 | $-CO_2^{\ominus}$ |
| 51 | $-CH_2CH_2-$ | 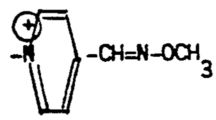 | $-CO_2^{\ominus}$ |
| 52 | $-CH_2$ | 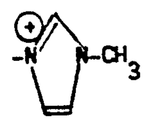 | $-CO_2^{\ominus}$ |
| 53 | $-CH_2CH_2-$ | 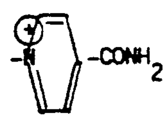 | $-CO_2^{\ominus}$ |
| 54 | $-CH_2CH_2-$ | 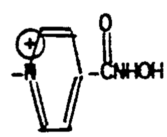 | $-CO_2^{\ominus}$ |
| 55 | $-CH_2CH_2-$ | 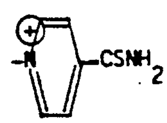 | $-CO_2^{\ominus}$ |

| Compound No. | L | 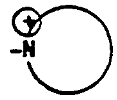 | Y |
|---|---|---|---|
| 56 | $-CH_2CH_2-$ | 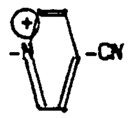 | $-CO_2^{\ominus}$ |
| 57 | $-CH_2CH_2-$ | 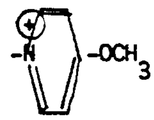 | $-CO_2^{\ominus}$ |
| 58 | $-CH_2CH_2-$ | 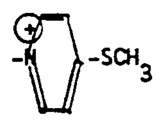 | $-CO_2^{\ominus}$ |
| 59 | $-CH_2CH_2-$ | 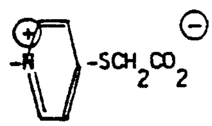 | $-CO_2Na$ |
| 60 | $-CH_2CH_2-$ | 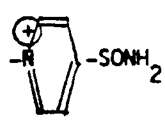 | $-CO_2^{\ominus}$ |
| 61 | $-CH_2CH_2-$ | 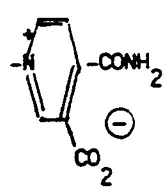 | $-CO_2Na$ |

| Compound No. | L | 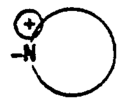 | Y |
| --- | --- | --- | --- |
| 62 | $-CH_2CH_2-$ | 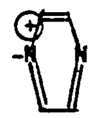 | $-CO_2^{\ominus}$ |
| 63 | $-CH_2CH_2-$ | 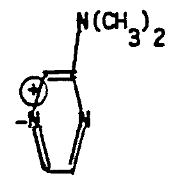 | $-CO_2^{\ominus}$ |
| 64 | $-CH_2CH_2-$ | 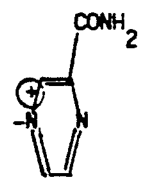 | $-CO_2^{\ominus}$ |
| 65 | $-CH_2CH_2-$ | 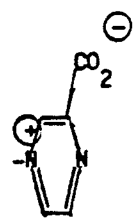 | $-CO_2Na$ |
| 66 | $-CH(CH_3)CH_2-$ | 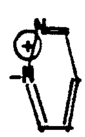 | $-CO_2^{\ominus}$ |
| 67 | $-CH_2CH_2-$ | 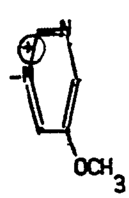 | $-CO_2^{\ominus}$ |

| Compound No. | L | 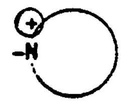 | Y |
|---|---|---|---|
| 68 | $-CH_2CH_2-$ | 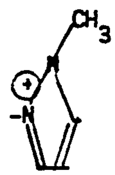 | $-CO_2^{\ominus}$ |
| 69 | $-CH_2CH_2-$ | 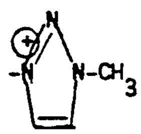 | $-CO_2^{\ominus}$ |
| 70 | $-CH_2CH_2-$ | 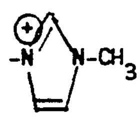 | $-CO_2^{\ominus}$ |
| 71 | $-CH_2CH_2-$ | 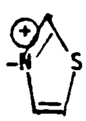 | $-CO_2^{\ominus}$ |
| 72 | $-CH(CH_3)CH_2-$ | 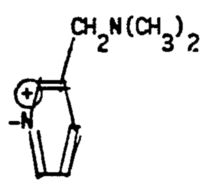 | $-CO_2^{\ominus}$ |
| 73 | $-CH(CH_3)CH_2-$ | 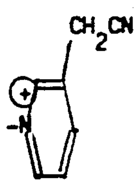 | $-CO_2^{\ominus}$ |

| Compound No. | L | 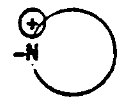 | Y |
|---|---|---|---|
| 74 | $-CH(CH_3)CH_2-$ | 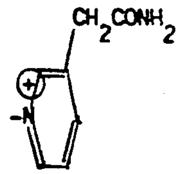 | $-CO_2^{\ominus}$ |
| 75 | $-CH(CH_3)CH_2-$ | 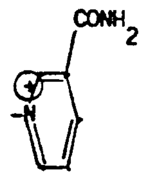 | $-CO_2^{\ominus}$ |
| 76 | $-CH(CH_3)CH_2-$ | 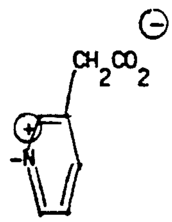 | $-CO_2Na$ |
| 77 | $-CH(CH_3)CH_2-$ | 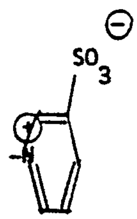 | $-CO_2Na$ |
| 78 | $-CH(CH_3)CH_2-$ | 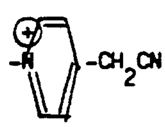 | $-CO_2^{\ominus}$ |
| 79 | $-CH(CH_3)CH_2-$ | 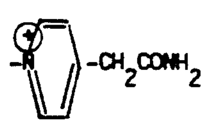 | $-CO_2^{\ominus}$ |

| Compound No. | L | 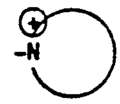 | Y |
| --- | --- | --- | --- |
| 80 | $-CH(CH_3)CH_2-$ | 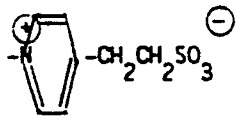 | $-CO_2Na$ |
| 81 | $-CH(CH_3)CH_2-$ | 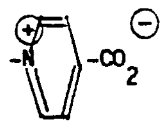 | $-CO_2Na$ |
| 82 | $-CH(CH_3)CH_2-$ | 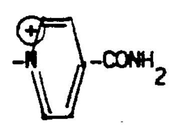 | $-CO_2^{\ominus}$ |
| 83 | $-CH_2$ | 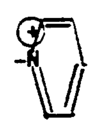 | $-CO_2^{\ominus}$ |
| 84 | $-CH_2$ | 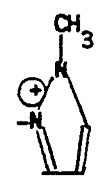 | $-CO_2^{\ominus}$ |
| 85 | $-CH_2$ | 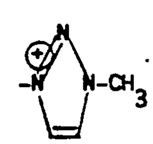 | $-CO_2^{\ominus}$ |

| Compound No. | L | 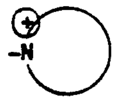 | Y |
|---|---|---|---|
| 86 | $-CH(CH_3)-$ | 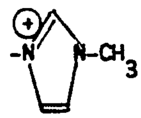 | $-CO_2^{\ominus}$ |
| 87 | $-CH_2CH_2CH_2-$ | 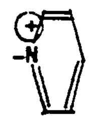 | $-CO_2^{\ominus}$ |
| 88 | $-CH_2CH_2-$ | 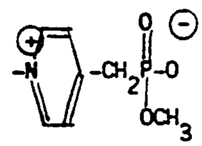 | $-CO_2Na$ |
| 89 | $-CH_2CH_2-$ | 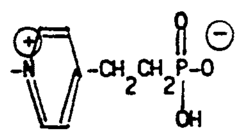 | $-CO_2Na$ |
| 90 | $-CH_2CH_2-$ | 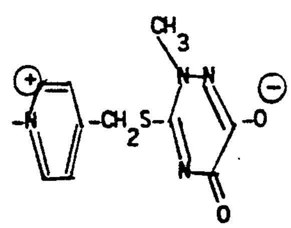 | $-CO_2Na$ |
| 91 | $-CH_2$ | 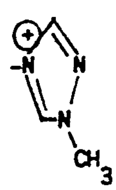 | $-CO_2^{\ominus}$ |

| Compound No. | L | 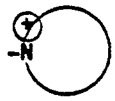 | Y |
|---|---|---|---|
| 92 | $-CH_2-$ | 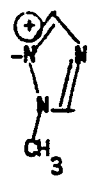 | $-CO_2^{\ominus}$ |
| 93 | $-CH_2-$ | 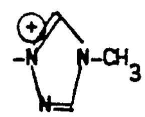 | $-CO_2^{\ominus}$ |
| 94 | $-CH_2-$ | 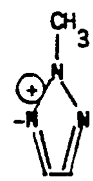 | $-CO_2^{\ominus}$ |
| 95 | $-CH_2-$ | 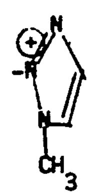 | $-CO_2^{\ominus}$ |
| 96 | $-CH_2-$ | 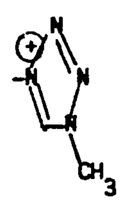 | $-CO_2^{\ominus}$ |
| 97 | $-CH_2-$ | 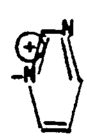 | $-CO_2^{\ominus}$ |
| 98 | $-CH_2-$ | 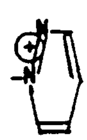 | $-CO_2^{\ominus}$ |

| Compound No. | L | 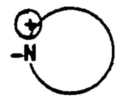 | Y |
|---|---|---|---|
| 99 | $-CH_2-$ | 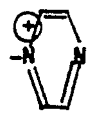 | $-CO_2^{\ominus}$ |
| 100 | $-CH_2-$ | 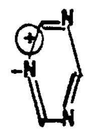 | $-CO_2^{\ominus}$ |
| 101 | $-CH_2-$ | 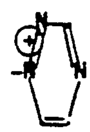 | $-CO_2^{\ominus}$ |
| 102 | $-CH_2-$ | 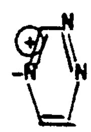 | $-CO_2^{\ominus}$ |
| 103 | $-CH_2-$ | 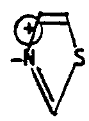 | $-CO_2^{\ominus}$ |
| 104 | $-CH_2-$ | 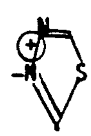 | $-CO_2^{\ominus}$ |
| 105 | $-CH_2-$ | 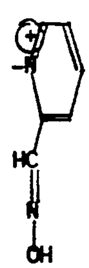 | $-CO_2^{\ominus}$ |

| Compound No. | L | 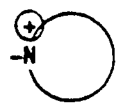 | Y |
|---|---|---|---|
| 106 | $-CH_2CH_2-$ | 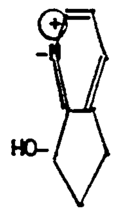 | $-CO_2^{\ominus}$ |
| 107 | $-CH_2CH_2-$ | 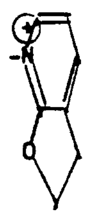 | $-CO_2^{\ominus}$ |
| 108 | $-CH_2CH_2-$ | 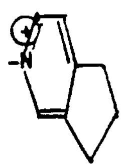 | $-CO_2^{\ominus}$ |
| 109 | $-CH_2CH_2-$ | 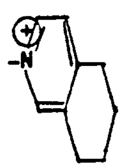 | $-CO_2^{\ominus}$ |
| 110 | $-CH_2CH_2-$ | 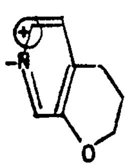 | $-CO_2^{\ominus}$ |

| Compound No. | L | 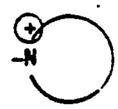 | Y |
|---|---|---|---|
| 111 | $-CH_2CH_2-$ | 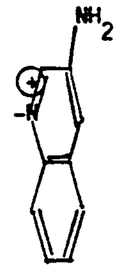 | $-CO_2^{\ominus}$ |
| 112 | $-CH_2CH_2-$ | 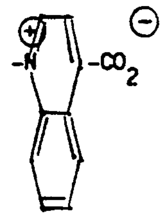 | $-CO_2Na$ |
| 113 | $-CH_2CH_2-$ | 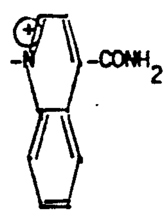 | $-CO_2^{\ominus}$ |
| 114 | $-CH_2CH_2-$ | 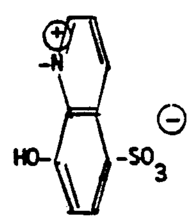 | $-CO_2Na$ |
| 115 | $-CH_2CH_2-$ | 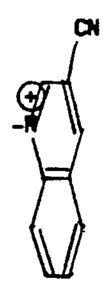 | $-CO_2^{\ominus}$ |

| Compound No. | L | 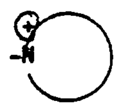 | Y |
|---|---|---|---|
| 116 | $-CH_2CH_2-$ | 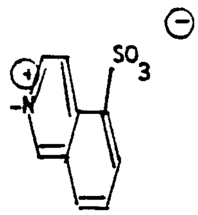 | $-CO_2Na$ |
| 117 | $-CH_2CH_2-$ | 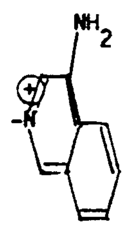 | $-CO_2^{\ominus}$ |
| 118 | $-CH_2CH_2-$ | 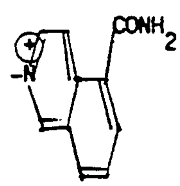 | $-CO_2^{\ominus}$ |
| 119 | $-CH_2CH_2-$ | 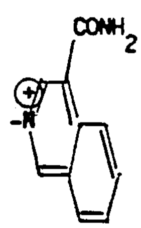 | $-CO_2^{\ominus}$ |
| 120 | $-CH_2CH_2-$ | 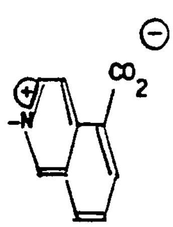 | $-CO_2Na$ |

| Compound No. | L | 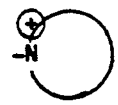 | Y |
|---|---|---|---|
| 121 | $-CH_2CH_2-$ | 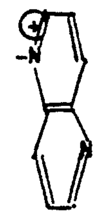 | $-CO_2^{\ominus}$ |
| 122 | $-CH_2CH_2-$ | 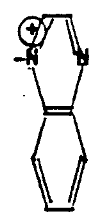 | $-CO_2^{\ominus}$ |
| 123 | $-CH_2CH_2-$ | 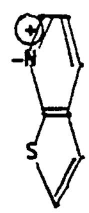 | $-CO_2^{\ominus}$ |
| 124 | $-CH_2CH_2-$ | 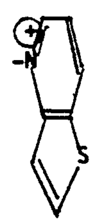 | $-CO_2^{\ominus}$ |
| 125 | $-CH_2CH_2-$ | 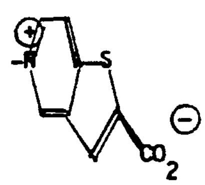 | $-CO_2Na$ |

| Compound No. | L | 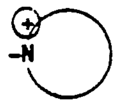 | Y |
|---|---|---|---|
| 126 | $-CH_2CH_2-$ | 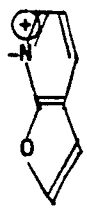 | $-CO_2^{\ominus}$ |
| 127 | $-CH_2CH_2-$ | 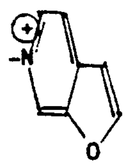 | $-CO_2^{\ominus}$ |
| 128 | $-CH_2CH_2-$ | 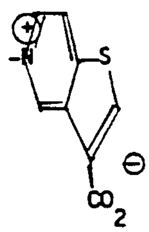 | $-CO_2Na$ |
| 129 | $-CH_2CH_2-$ | 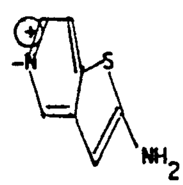 | $-CO_2^{\ominus}$ |
| 130 | $-CH_2CH_2-$ | 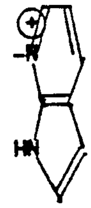 | $-CO_2^{\ominus}$ |

| Compound No. | L | 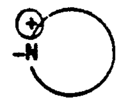 | Y |
|---|---|---|---|
| 131 | $-CH_2CH_2-$ | 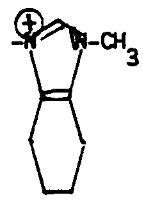 | $-CO_2^{\ominus}$ |
| 132 | $-CH_2CH_2-$ | 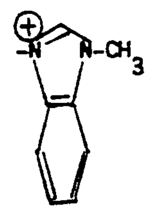 | $-CO_2^{\ominus}$ |
| 133 | $-CH_2CH_2-$ | 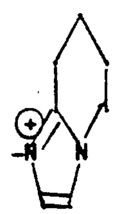 | $-CO_2^{\ominus}$ |
| 134 | $-CH_2CH_2-$ | 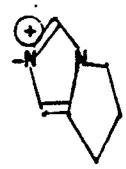 | $-CO_2^{\ominus}$ |
| 135 | $-CH_2CH_2-$ | 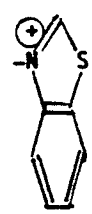 | $-CO_2Na$ |

| Compound No. | L | 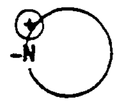 | Y |
|---|---|---|---|
| 136 | $-CH_2CH_2-$ | 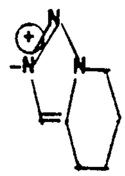 | $-CO_2^{\ominus}$ |
| 137 | $-CH_2CH_2-$ | 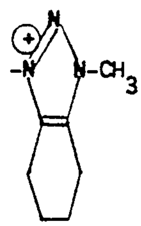 | $-CO_2^{\ominus}$ |
| 138 | $-CH_2CH_2-$ | 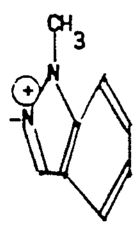 | $-CO_2^{\ominus}$ |
| 139 | $-CH_2CH_2-$ | 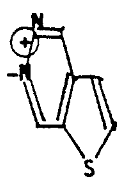 | $-CO_2^{\ominus}$ |
| 140 | $-CH_2CH_2-$ | 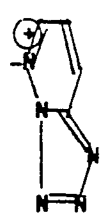 | $-CO_2Na$ |

| Compound No. | L | 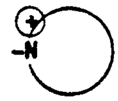 | Y |
|---|---|---|---|
| 141 | $-CH_2CH_2-$ | 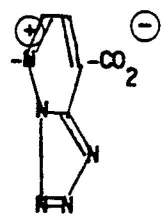 | $-CO_2Na$ |
| 142 | $-CH_2CH_2-$ | 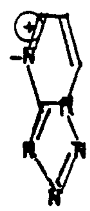 | $-CO_2^{\ominus}$ |
| 143 | $-CH_2CH_2-$ | 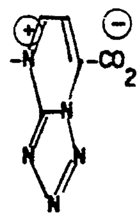 | $-CO_2Na$ |
| 144 | $-CH_2CH_2-$ | 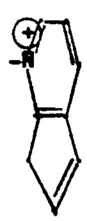 | $-CO_2^{\ominus}$ |
| 145 | $-CH_2CH_2-$ | 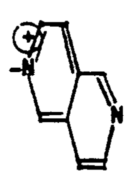 | $-CO_2^{\ominus}$ |

# 0168707



| Compound No. | L | 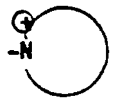 | Y |
|---|---|---|---|
| 146 | 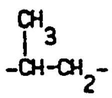 | 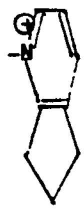 | $-CO_2^{\ominus}$ |
| 147 | $-CH_2-$ | 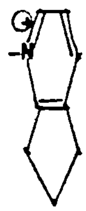 | $-CO_2^{\ominus}$ |
| 148 | $-CH_2-$ | 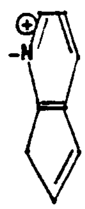 | $-CO_2^{\ominus}$ |
| 149 | $-CH_2-$ | 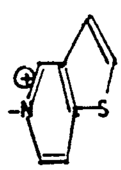 | $-CO_2^{\ominus}$ |
| 150 | $-CH_2-$ | 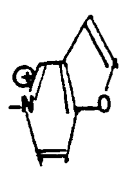 | $-CO_2^{\ominus}$ |

| Compound No. | L | 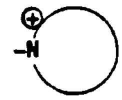 | Y |
|---|---|---|---|
| 151 | $-CH_2-$ | 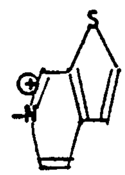 | $-CO_2^{\ominus}$ |
| 152 | $-CH_2-$ | 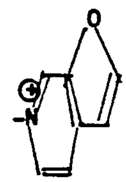 | $-CO_2^{\ominus}$ |
| 153 | $-CH_2-$ | 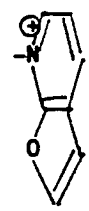 | $-CO_2^{\ominus}$ |
| 154 | $-CH_2-$ | 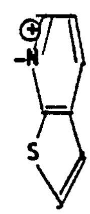 | $-CO_2^{\ominus}$ |
| 155 | $-CH_2-$ | 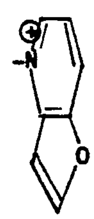 | $-CO_2^{\ominus}$ |

| Compound No. | L | 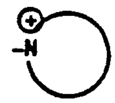 | Y |
|---|---|---|---|
| 156 | $-CH_2-$ | 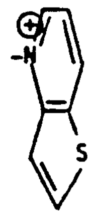 | $-CO_2^{\ominus}$ |
| 157 | $-CH_2-$ | 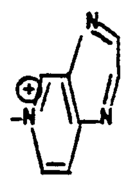 | $-CO_2^{\ominus}$ |
| 158 | $-CH_2-$ | 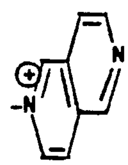 | $-CO_2^{\ominus}$ |
| 159 | $-CH_2-$ | 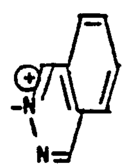 | $-CO_2^{\ominus}$ |
| 160 | $-CH_2-$ | 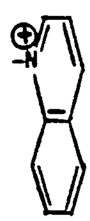 | $-CO_2^{\ominus}$ |
| 161 | $-CH_2CH_2-$ | 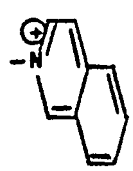 | $-CO_2^{\ominus}$ |

| Compound No. | L | 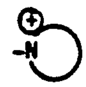 | Y |
|---|---|---|---|
| 162 | $-CH_2-$ | 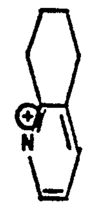 | $-CO_2^{\ominus}$ |
| 163 | $-CH_2CH_2-$ | 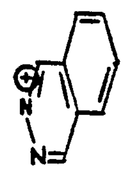 | $-CO_2^{\ominus}$ |
| 164 | $-CH_2CH_2-$ | 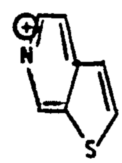 | $-CO_2^{\ominus}$ |
| 165 | $-CH_2CH_2-$ | 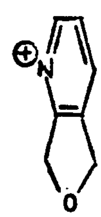 | $-CO_2^{\ominus}$ |
| 166 | $-CH_2CH_2-$ | 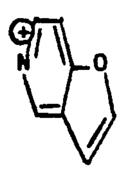 | $-CO_2^{\ominus}$ |

| Compound No. | L | 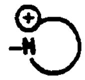 | Y |
|---|---|---|---|
| 167 | $-CH(CH_3)CH_2-$ | 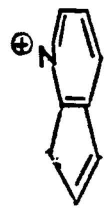 | $-CO_2^{\ominus}$ |
| 168 | $-CH_2CH_2-$ | 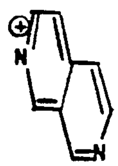 | $-CO_2^{\ominus}$ |
| 169 | $-CH_2CH_2-$ | 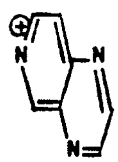 | $-CO_2^{\ominus}$ |
| 170 | $-CH_2CH_2-$ | 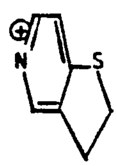 | $-CO_2^{\ominus}$ |
| 171 | $-CH_2CH_2-$ | 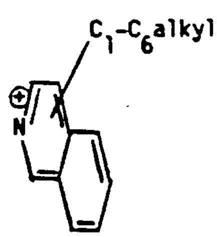 | $-CO_2^{\ominus}$ |
| 172 | $-CH_2CH_2-$ | 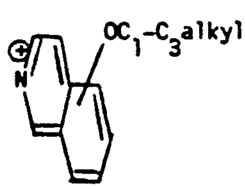 | $-CO_2^{\ominus}$ |

| Compound No. | L | 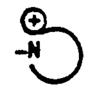 | Y |
|---|---|---|---|
| 173 | $-CH_2CH_2-$ | 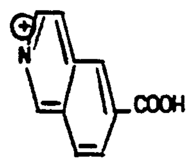 | $-CO_2Na$ |
| 174 | $-CH_2CH_2-$ | 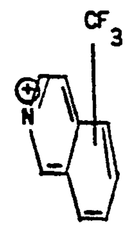 | $-CO_2^{\ominus}$ |
| 175 | $-CH_2CH_2-$ | 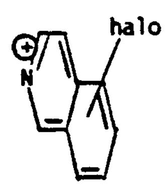 | $-CO_2^{\ominus}$ |
| 176 | $-CH(CH_3)CH_2-$ | 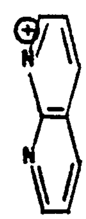 | $-CO_2^{\ominus}$ |
| 177 | $-CH(CH_3)CH_2-$ | 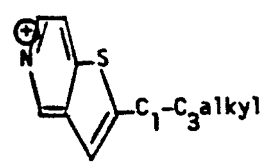 | $-CO_2^{\ominus}$ |

| Compound No. | L | 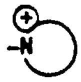 | Y |
|---|---|---|---|
| 178 | $-CH(CH_3)CH_2-$ | 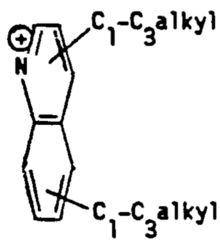 | $-CO_2^{\ominus}$ |
| 179 | $-CH(CH_3)CH_2-$ | 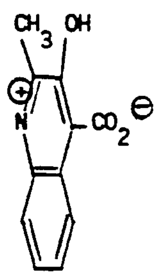 | $-CO_2Na$ |
| 180 | $-CH(CH_3)CH_2-$ | 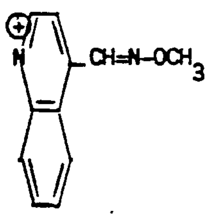 | $-CO_2^{\ominus}$ |
| 181 | $-CH(CH_3)CH_2-$ | 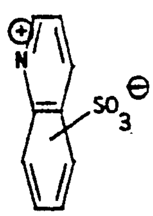 | $-CO_2Na$ |
| 182 | $-CH(CH_3)CH_2-$ | 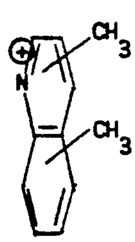 | $-CO_2^{\ominus}$ |

| Compound No. | L | 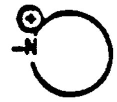 | Y |
|---|---|---|---|
| 183 | $-CH_2CH_2-$ | 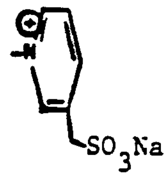 | $-CO_2^{\ominus}$ |
| 184 | $-CH_2CH_2-$ | 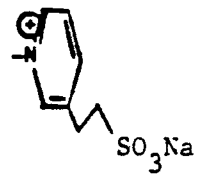 | $-CO_2^{\ominus}$ |
| 185 | $-CH_2CH_2-$ | 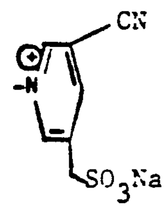 | $-CO_2^{\ominus}$ |
| 186 | $-CH_2CH_2-$ | 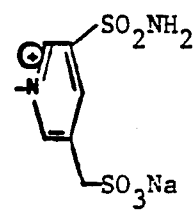 | $-CO_2^{\ominus}$ |
| 187 | $-CH_2CH_2-$ | 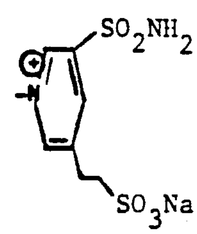 | $-CO_2^{\ominus}$ |

| Compound No | L | 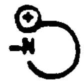 | Y |
|---|---|---|---|
| 188 | $-CH_2CH_2-$ | 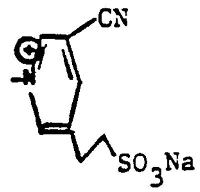 | $-CO_2^{\ominus}$ |
| 189 | $-CH_2CH_2-$ | 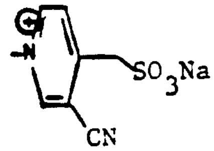 | $-CO_2^{\ominus}$ |
| 190 | $-CH_2CH_2-$ | 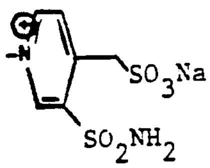 | $-CO_2^{\ominus}$ |
| 191 | $-CH_2CH_2-$ | 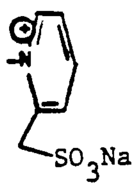 | $-CO_2^{\ominus}$ |
| 192 | $-CH_2CH_2-$ | 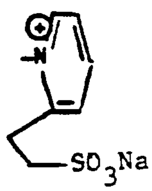 | $-CO_2^{\ominus}$ |
| 193 | $-CH_2CH_2-$ | 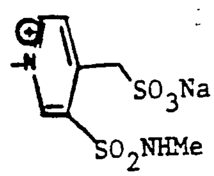 | $-CO_2^{\ominus}$ |

| Compound No. | L | 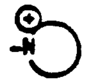 | Y |
| --- | --- | --- | --- |
| 194 | $-CH_2CH_2-$ | 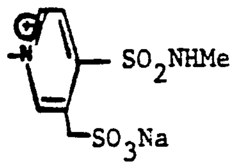 | $-CO_2^{\ominus}$ |
| 195 | $-CH_2CH_2-$ | 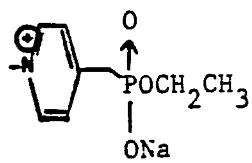 | $-CO_2^{\ominus}$ |
| 196 | $-CH_2CH_2-$ | 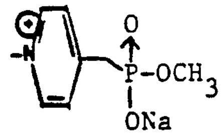 | $-CO_2^{\ominus}$ |
| 197 | $-CH_2CH_2-$ | 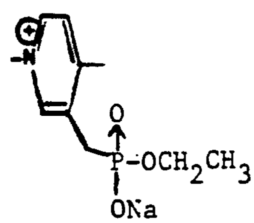 | $-CO_2^{\ominus}$ |
| 198 | $-CH_2CH_2-$ | 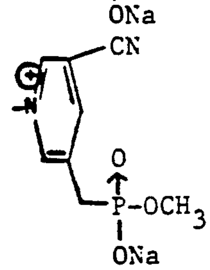 | $-CO_2^{\ominus}$ |
| 199 | $-CH_2CH_2-$ | 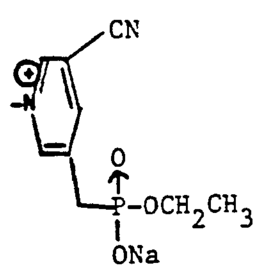 | $-CO_2^{\ominus}$ |

| Compound No. | L | 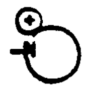 | Y |
|---|---|---|---|
| 200 | $-CH_2CH_2-$ | 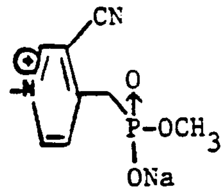 | $-CO_2^{\ominus}$ |
| 201 | $-CH_2CH_2-$ | 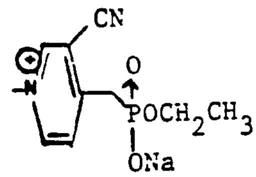 | $-CO_2^{\ominus}$ |
| 202 | $-CH_2CH_2-$ | 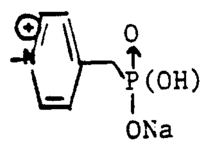 | $-CO_2^{\ominus}$ |
| 203 | $-CH_2CH_2-$ | 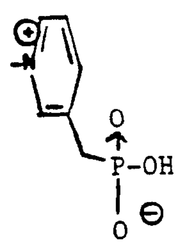 | $-CO_2^{\ominus}$ |
| 204 | $-CH_2CH_2-$ | 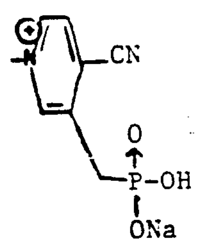 | $-CO_2^{\ominus}$ |
| 205 | $-CH_2CH_2-$ | 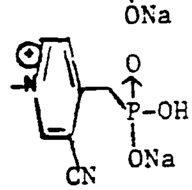 | $-CO_2^{\ominus}$ |

| Compound No. | L | 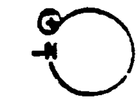 | Y |
| --- | --- | --- | --- |
| 206 | $-CH_2CH_2-$ | 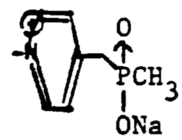 | $-CO_2^{\ominus}$ |
| 207 | $-CH_2CH_2-$ | 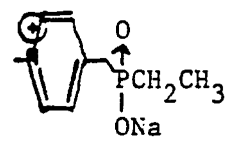 | $-CO_2^{\ominus}$ |
| 208 | $-CH_2CH_2-$ | 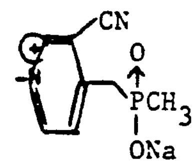 | $-CO_2^{\ominus}$ |
| 209 | $-CH_2CH_2-$ | 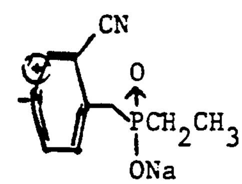 | $-CO_2^{\ominus}$ |
| 210 | $-CH_2CH_2-$ | 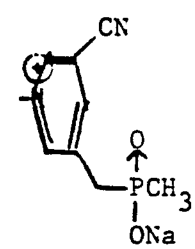 | $-CO_2^{\ominus}$ |
| 211 | $-CH_2CH_2-$ | 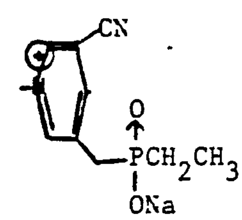 | $-CO_2^{\ominus}$ |

| Compound No. | L | 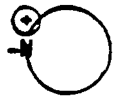 | Y |
|---|---|---|---|
| 212 | $-CH_2CH_2-$ | 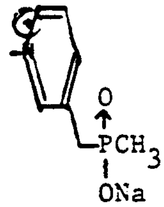 | $-CO_2^{\ominus}$ |
| 213 | $-CH_2CH_2-$ | 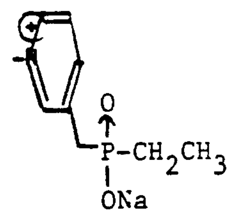 | $-CO_2^{\ominus}$ |
| 214 | $-CH_2CH_2-$ | 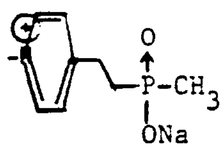 | $-CO_2^{\ominus}$ |
| 215 | $-CH_2CH_2-$ | 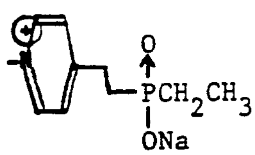 | $-CO_2^{\ominus}$ |
| 216 | $-CH_2CH_2-$ | 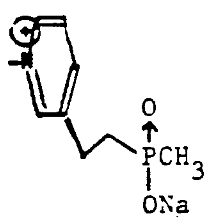 | $-CO_2^{\ominus}$ |
| 217 | $-CH_2CH_2-$ | 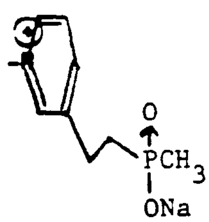 | $-CO_2^{\ominus}$ |

| Compound No. | L | 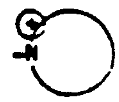 | Y |
|---|---|---|---|
| 218 | $-CH_2CH_2-$ | 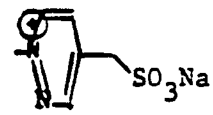 | $-CO_2^{\ominus}$ |
| 219 | $-CH_2CH_2-$ | 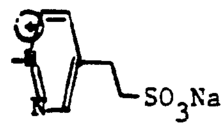 | $-CO_2^{\ominus}$ |
| 220 | $-CH_2CH_2-$ | 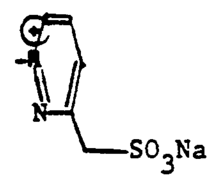 | $-CO_2$ |
| 221 | $-CH_2CH_2-$ | 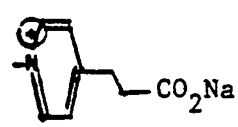 | $-CO_2^{\ominus}$ |
| 222 | $-CH_2CH_2-$ | 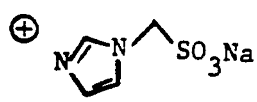 | $-CO_2^{\ominus}$ |
| 223 | $-CH_2CH_2-$ | 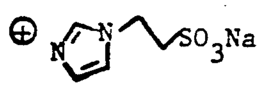 | $-CO_2^{\ominus}$ |

0168707

16622IG

| Compound No. | L | 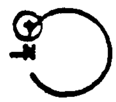 | Y |
|---|---|---|---|
| 224 | $-CH_2CH_2-$ | 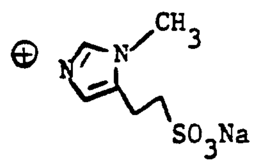 | $-CO_2^{\ominus}$ |
| 225 | $-CH_2CH_2-$ | 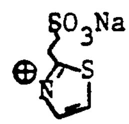 | $-CO_2^{\ominus}$ |
| 226 | $-CH_2CH_2-$ | 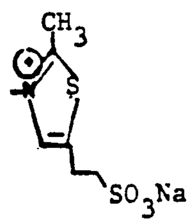 | $-CO_2^{\ominus}$ |
| 227 | $-CH_2CH_2-$ | 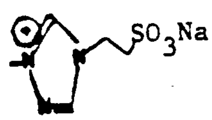 | $-CO_2^{\ominus}$ |
| 228 | $-CH_2CH_2-$ | 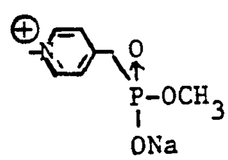 | $-CO_2^{\ominus}$ |

EXAMPLE 35

By using the procedures of Examples 1-30, the following compounds are prepared:

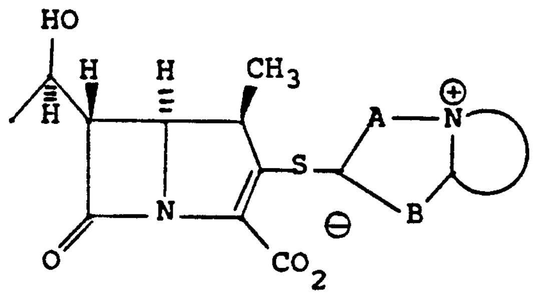 where 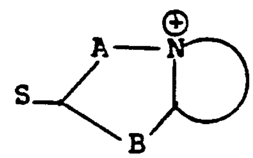 is

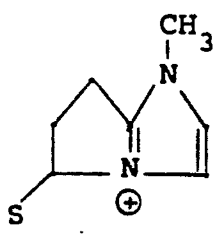 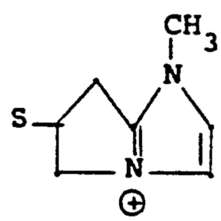 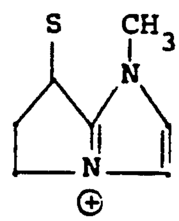

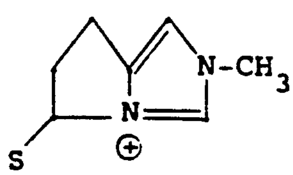 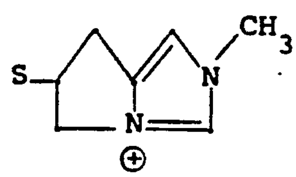 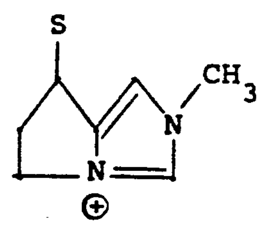

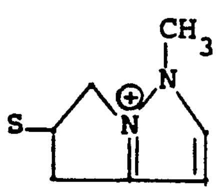 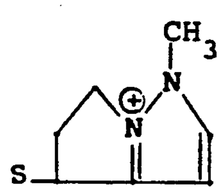 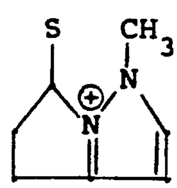

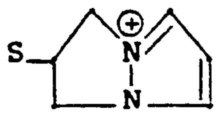
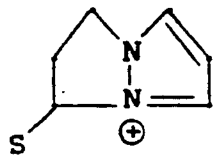
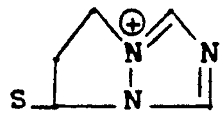

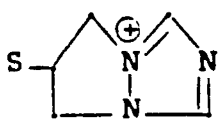
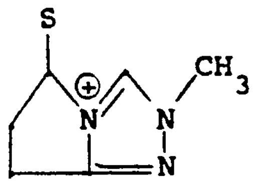
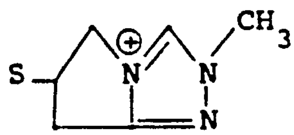
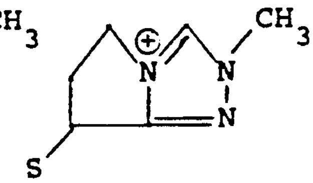

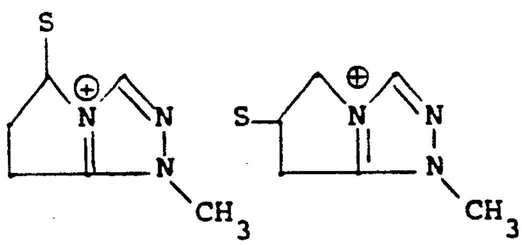
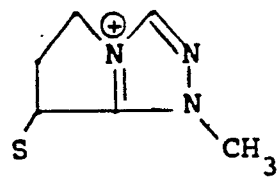

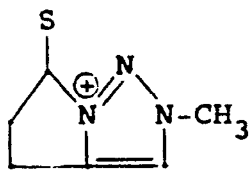
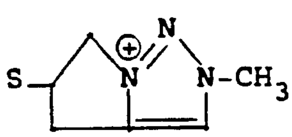
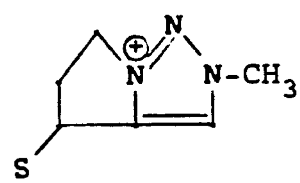

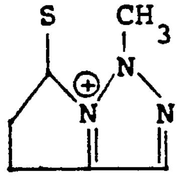
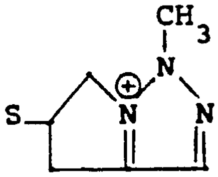
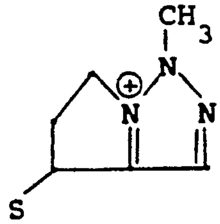

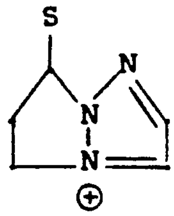 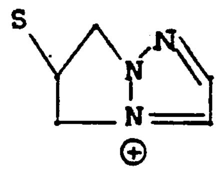 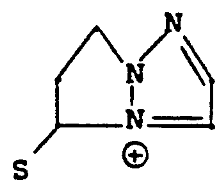

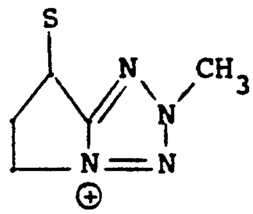 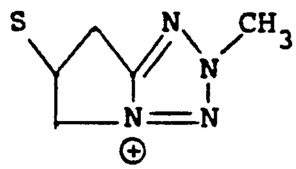 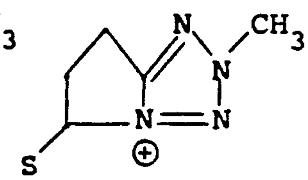

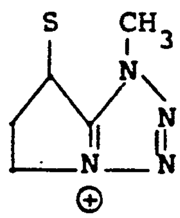 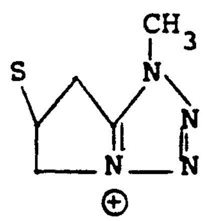 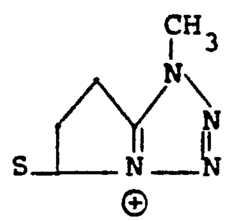

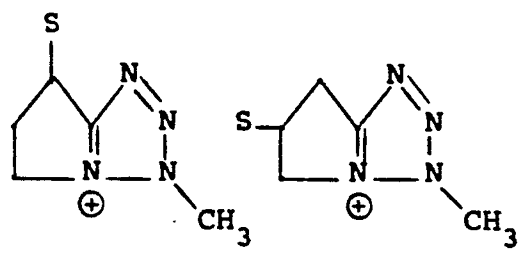 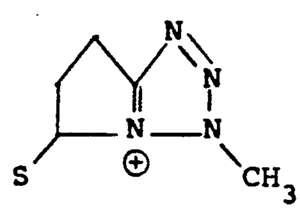

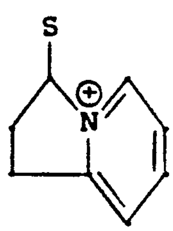 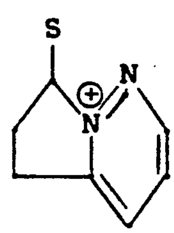 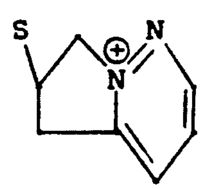

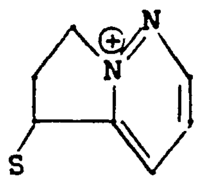
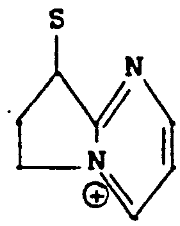
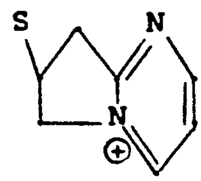

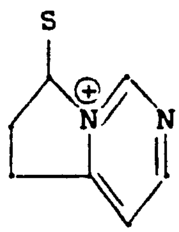
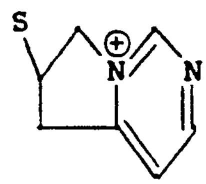
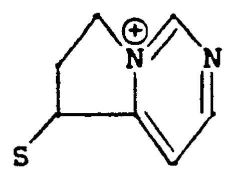

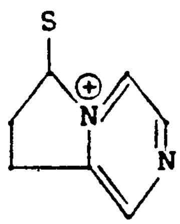
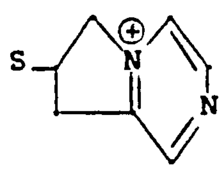
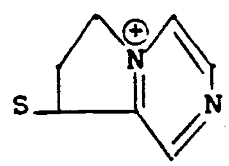

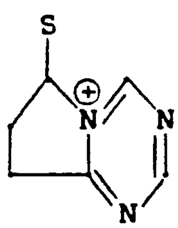
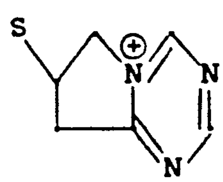
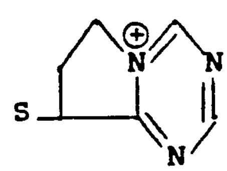

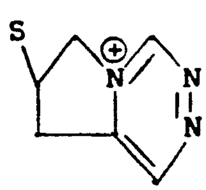
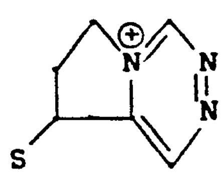
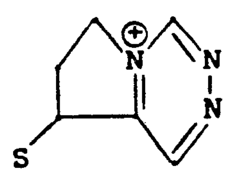

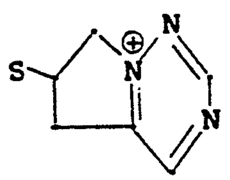

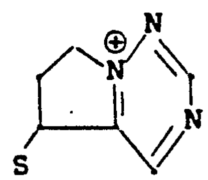

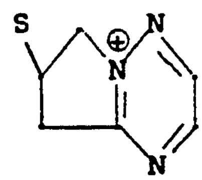

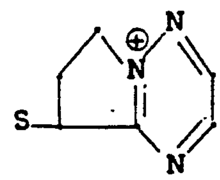

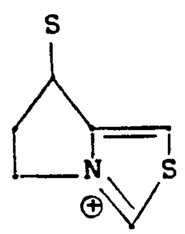

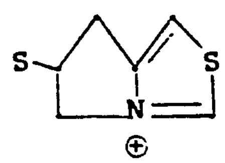

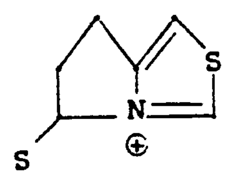

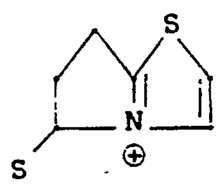

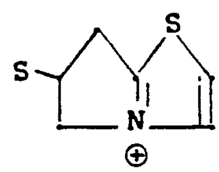

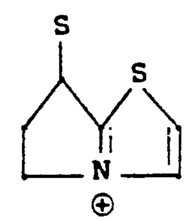

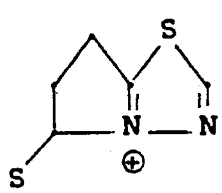

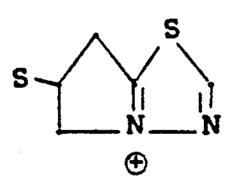

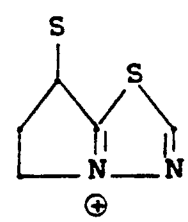

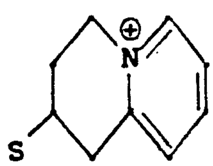

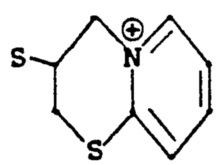

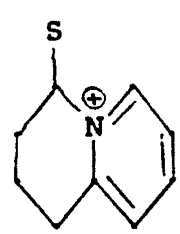

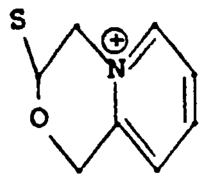
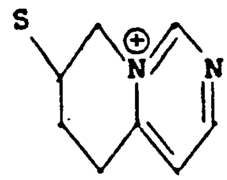
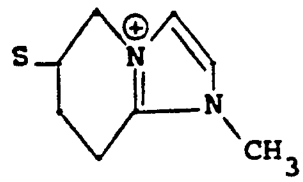

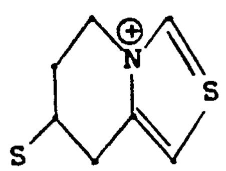
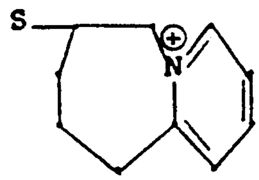
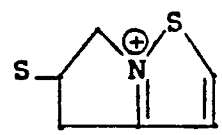

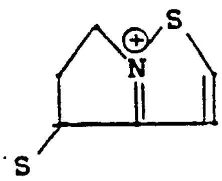
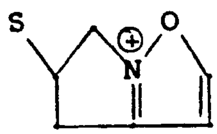
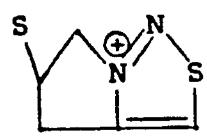

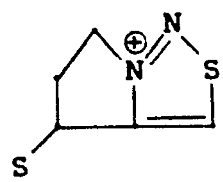
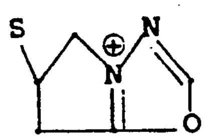
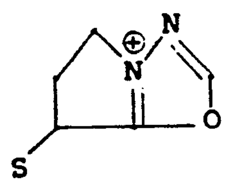

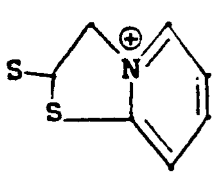
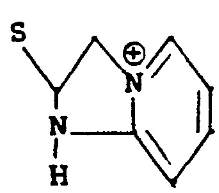
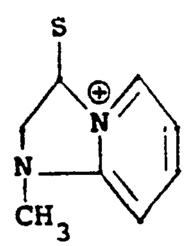

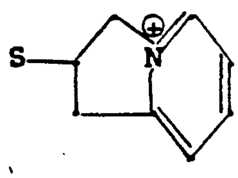

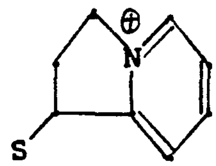

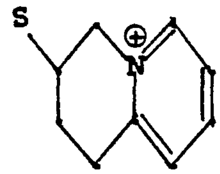

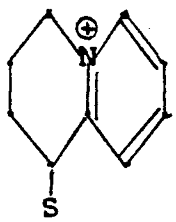

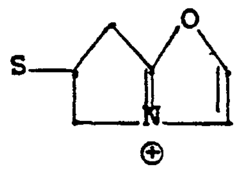

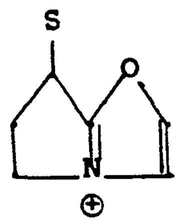

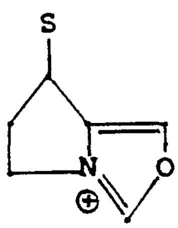

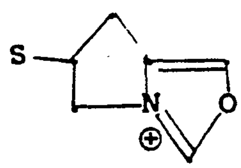

Claims to the invention follow.

WHAT IS CLAIMED IS:

1. A compound having the formula:

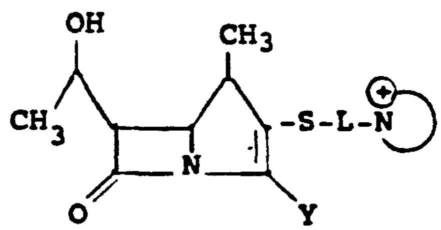

wherein

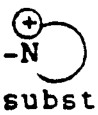 is a quaternary, monocyclic or bicyclic, substituted or unsubstituted heteroaryl group containing (a) when monocyclic, up to 3 heteroatoms and up to 6 total ring atoms or (b) when bicyclic up to 5 heteroatoms and 9-10 ring atoms, which is optionally substituted by one or more of the groups independently selected from

1) a substituted or unsubstituted $C_1$-$C_6$ alkyl, $C_2$-$C_6$ alkenyl, $C_5$-$C_7$ cycloalkenyl, $C_3$-$C_7$ cycloalkyl, or ($C_3$-$C_7$ cycloalkyl) methyl;
2) a substituted or unsubstituted $C_3$-$C_7$ heterocycloalkyl or ($C_3$-$C_7$ heterocycloalkyl) methyl having up to 3 hetero ring atoms;
3) an unsubstituted or substituted phenyl or heteroaryl radical;
4) an unsubstituted or substituted phenyl ($C_1$-$C_4$ alkyl) or heteroaryl ($C_1$-$C_4$ alkyl) radical;
5) a trifluoromethyl or pentafluoroethyl group;

6) a halogen atom;

7) an unsubstituted or substituted $C_1$-$C_4$ alkoxy radical;

8) a hydroxyl group;

9) an unsubstituted or substituted ($C_1$-$C_6$ alkyl) carbonyloxy radical;

10) a carbamoyloxy radical which is unsubstituted, monosubstituted or disubstituted on the nitrogen with a $C_1$-$C_4$ alkyl group;

11) a $C_1$-$C_6$ alkylthio radical, $C_1$-$C_6$ alkylsulfinyl radical or a $C_1$-$C_6$ alkylsulfonyl radical each of which is unsubstituted or substituted in the alkyl group;

12 ) a sulfamoyl group which is unsubstituted, monosubstituted, or disubstituted on nitrogen with a $C_1$-$C_4$ alkyl group,

13) an amino group;

14) a mono ($C_1$-$C_4$ alkyl) amino or di ($C_1$-$C_4$ alkyl) amino radical each of which is unsubstituted or substituted in the alkyl group;

15 ) a formylamino group;

16 ) an unsubstituted or substituted ($C_1$-$C_6$ alkyl) carbonylamino radical;

17 ) a ($C_1$-$C_4$ alkoxy) carbonylamino radical;

18 ) a ureido group in which the terminal nitrogen atom is unsubstituted or monosubstituted with a $C_1$-$C_6$ alkyl group;

19 ) a ($C_1$-$C_6$ alkyl) sulfonamido group;

20 ) a cyano group;

21 ) a formyl or acetalized formyl radical;

22) an unsubstituted or substituted ($C_1$-$C_6$ alkyl) carbonyl radical wherein the carbonyl group is free or acetalized;

23) an unsubstituted or substituted phenylcarbonyl or heteroarylcarbonyl radical;

24) a hydroxyiminomethyl radical in which the oxygen or carbon atom is optionally substituted by a $C_1$-$C_4$ alkyl group;

25) a ($C_1$-$C_6$ alkoxy) carbonyl radical;

26) a carbamoyl radical which is unsubstituted, monosubstituted, or disubstituted on the nitrogen atom with a $C_1$-$C_4$ alkyl group;

27) a N-hydroxy carbamoyl or N-($C_1$-$C_4$ alkoxy) carbamoyl radical in which the nitrogen atom may be additionally substituted by a $C_1$-$C_4$ alkyl group;

28) a thiocarbamoyl group;

29) an amidino group 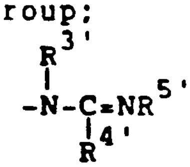 or

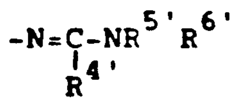

wherein $R^{3'}$, $R^{4'}$, $R^{5'}$, $R^{6'}$ are hydrogen, $C_1$-$C_4$ alkyl, or wherein two of the groups together form a $C_3$-$C_6$ alkylidine radical optionally interupted by a heteroatom and joined to either one or two nitrogen atoms to form a ring;

30) a guanidino group in which $R^{4'}$ above is $NR^{5'}R^{6'}$;

31) a carbamimidoyl group (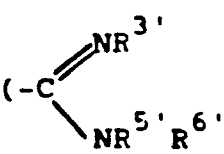) wherein $R^{3'}$, $R^{5'}$ and $R^{6'}$ are as defined above;

32) **a cyano ($C_1$-$C_4$ alkyl) radical;**

33) a carbamoyl ($C_1$-$C_4$ alkyl) radical;

34) **a hydroxy ($C_1$-$C_4$ alkyl) radical;**

35) **an amino ($C_1$-$C_6$ alkyl) radical which is unsubstituted, monosubstituted, or disubstituted on the nitrogen atom with $C_1$-$C_4$ alkyl groups, and**

36) an acidic side-chain of the structure -A or $-(CH_2)_n-X-(CH_2)_m-Y-A$ where:

n = 0-4

m = 0-4

X = $CHR^s$, CH=CH, phenylene ($-C_6H_4-$), NH, N(C1-C4 alkyl), O, S, S=O, C=O, $SO_2$, $SO_2NH$, $CO_2$, CONH,$OCO_2$, OC=O, NHC=O;

$R^s$ = H, O(C1-C4 alkyl), $NH_2$, NH(C1-C4 alkyl), N(C1-C4 alkyl)$_2$, CN, $CONH_2$, CON(C1-C4 alkyl)$_2$, $CO_2H$, $SO_2NH_2$, $SO_2$NH(C1-C4 alkyl);

Y = single bond, NH, N(C1-C4 alkyl), O, S;

A = an acidic function

**wherein the substituents in groups 7), 9), 11), 14), 16), 22), and 23) are selected from hydroxy, $C_1$-$C_4$alkoxy, mercapto, amino, mono- or di($C_1$-$C_4$alkyl)amino, cyano, halo, $CF_3$, COOH, sulfo, carbamoyl, and sulfamoyl, and wherein the substituents in grous 1)-4) are selected from those defined in groups 5)- 31).**

**L is a substituted or unsubstituted, straight or branched chain, bivalent $C_1$-$C_6$ alkyl, $C_2$-$C_6$ alkenyl or $C_3$-$C_6$ cycloalkyl, or $-C_1$-$C_4$ alkyl-X-$C_1$-$C_4$ alkyl wherein X is O, S, NH, or N($C_{1-4}$ alkyl), wherein the substituents are selected from groups 2) through 31);**

**and**

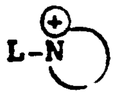 together, in addition to the individual definitions of L and 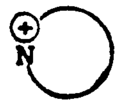 above, may be the group

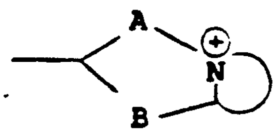

where

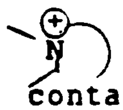 is a 5- to 6-membered heteroaryl group containing from one to three heteroatoms, which is optionally substituted by one or more members independently selected from the group consisting of the substituents defined as 1) - 40) under 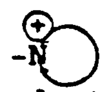 ; and

A and B are independently selected from a direct bond or a substituted or unsubstituted $C_1$-$C_4$alkylene group optionally interrupted by a heteroatom; provided that, A and B cannot both be a direct bond, and that the ring containing A and B contains from 5 to 8 atoms; and wherein the substituents on A and B are selected from hydroxyl, $C_1$-$C_3$alkoxy, amino, carboxyl, cyano, carbamoyl, trifluoromethyl, $C_1$-$C_4$alkyl, and di($C_1$-$C_4$alkyl)amino;

and

Y is i) -COOH, a pharmaceutically acceptable ester or salt thereof;

ii) COOR wherein R is a removeable carboxy protecting group;

iii) COOM wherein M is an alkali metal; or

iv) $COO^-$ provided that when Y is other than iv) a counterion $Z^{\ominus}$ is present.

2. A compound of Claim 1 wherein the compound is a member selected from the group consisting of:

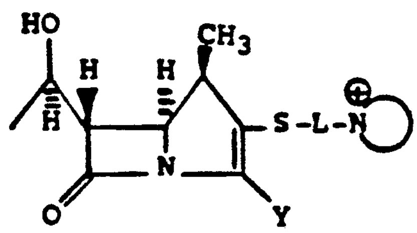

I

| Compound No. | L | 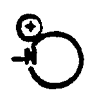 | Y |
|---|---|---|---|
| 1 | $-CH_2CH_2-$ | 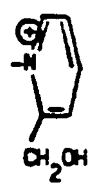 | $-CO_2^{\ominus}$ |
| 2 | $-CH_2CH_2-$ | 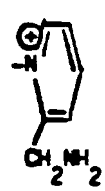 | $-CO_2^{\ominus}$ |

| Compound No. | L | 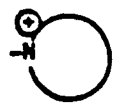 | Y |
|---|---|---|---|
| 3 | $-CH_2CH_2-$ | 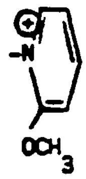 | $-CO_2^{\ominus}$ |
| 4 | $-CH_2CH_2-$ | 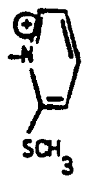 | $-CO_2^{\ominus}$ |
| 5 | $-CH_2CH_2-$ | 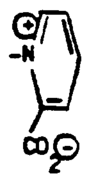 | $-CO_2Na$ |
| 6 | $-CH_2CH_2-$ | 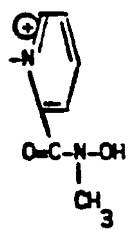 | $-CO_2^{\ominus}$ |
| 7 | $-CH_2CH_2-$ | 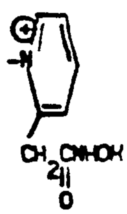 | $-CO_2^{\ominus}$ |

| Compound No. | L | 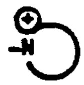 | Y |
|---|---|---|---|
| 8 | $-CH_2CH_2-$ | 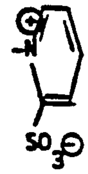 | $-CO_2Na$ |
| 9 | $-CH_2CH_2-$ | 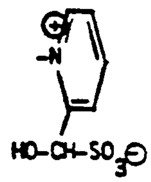 | $-CO_2Na$ |
| 10 | $-CH_2CH_2-$ | 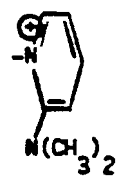 | $-CO_2^{\ominus}$ |
| 11 | $-CH_2CH_2-$ | 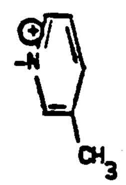 | $-CO_2^{\ominus}$ |
| 12 | $-CH_2CH_2-$ | 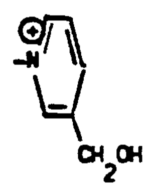 | $-CO_2^{\ominus}$ |
| 13 | $-CH_2CH_2-$ | 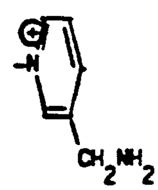 | $-CO_2^{\ominus}$ |

| Compound No. | L | 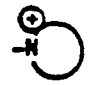 | Y |
|---|---|---|---|
| 14 | $-CH_2CH_2-$ | 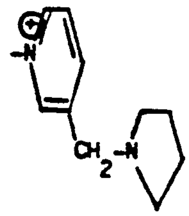 | $-CO_2^{\ominus}$ |
| 15 | $-CH_2CH_2-$ | 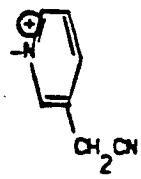 | $-CO_2^{\ominus}$ |
| 16 | $-CH(CH_3)CH_2-$ | 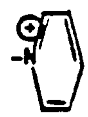 | $-CO_2^{\ominus}$ |
| 17 | $-CH_2CH_2-$ | 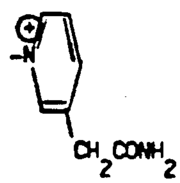 | $-CO_2^{\ominus}$ |
| 18 | $-CH_2CH_2-$ | 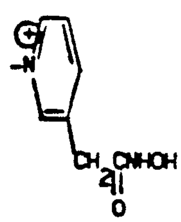 | $-CO_2^{\ominus}$ |
| 19 | $-CH_2CH_2-$ | 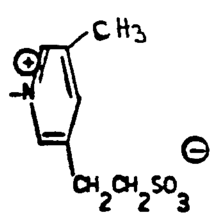 | $-CO_2Na$ |

| Compound No. | L | 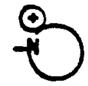 | Y |
|---|---|---|---|
| 20 | $-CH_2CH_2-$ | 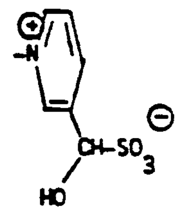 | $-CO_2Na$ |
| 21 | $-CH_2CH_2-$ | 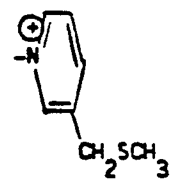 | $-CO_2^{\ominus}$ |
| 22 | $-CH_2CH_2-$ | 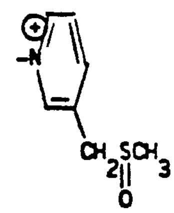 | $-CO_2^{\ominus}$ |
| 23 | $-CH_2CH_2-$ | 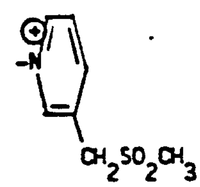 | $-CO_2^{\ominus}$ |
| 24 | $-CH_2CH_2-$ | 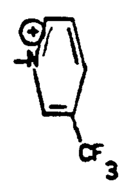 | $-CO_2^{\ominus}$ |
| 25 | $-CH_2CH_2-$ | 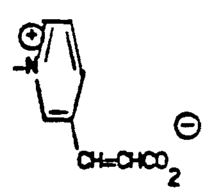 | $-CO_2Na$ |

| Compound No. | L | 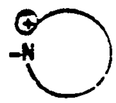 | Y |
|---|---|---|---|
| 26 | $-CH_2CH_2-$ | 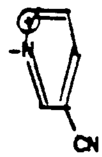 | $-CO_2^{\ominus}$ |
| 27 | $-CH_2CH_2-$ | 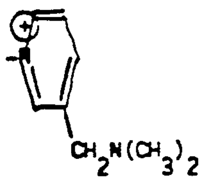 | $-CO_2^{\ominus}$ |
| 28 | $-CH_2CH_2-$ | 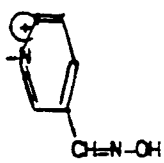 | $-CO_2^{\ominus}$ |
| 29 | $-CH_2CH_2-$ | 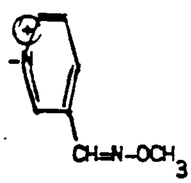 | $-CO_2^{\ominus}$ |
| 30 | $-CH_2CH_2-$ | 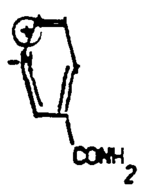 | $-CO_2^{\ominus}$ |
| 31 | $-CH_2CH_2-$ | 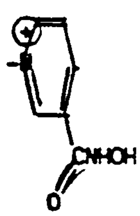 | $-CO_2^{\ominus}$ |

| Compound No. | L | 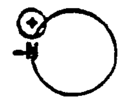 | Y |
|---|---|---|---|
| 32 | $-CH_2CH_2-$ | 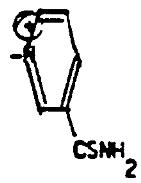 | $-CO_2^{\ominus}$ |
| 33 | $-CH_2CH_2-$ | 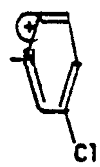 | $-CO_2^{\ominus}$ |
| 34 | $-CH_2CH_2-$ | 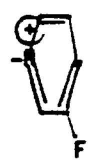 | $-CO_2^{\ominus}$ |
| 35 | $-CH_2CH_2-$ | 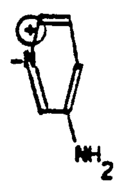 | $-CO_2^{\ominus}$ |
| 36 | $-CH_2CH_2-$ | 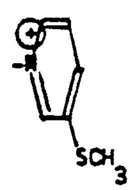 | $-CO_2^{\ominus}$ |
| 37 | $-CH_2CH_2-$ | 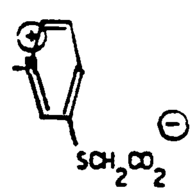 | $-CO_2Na$ |

| Compound No. | L | 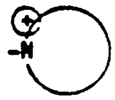 | Y |
|---|---|---|---|
| 38 | $-CH_2CH_2-$ | 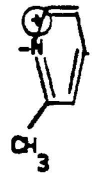 | $-CO_2^{\ominus}$ |
| 39 | $-CH_2CH_2-$ | 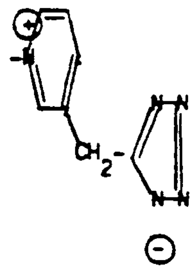 | $-CO_2Na$ |
| 40 | $-CH_2CH_2-$ | 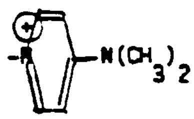 | $-CO_2^{\ominus}$ |
| 41 | $-CH_2CH_2-$ | 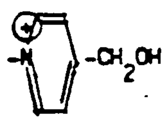 | $-CO_2^{\ominus}$ |
| 42 | $-CH_2CH_2-$ | 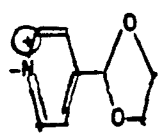 | $-CO_2^{\ominus}$ |
| 43 | $-CH_2CH_2-$ | 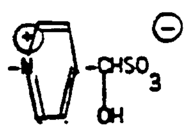 | $-CO_2Na$ |

| Compound No. | L | 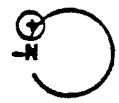 | Y |
|---|---|---|---|
| 44 | $-CH_2CH_2-$ | 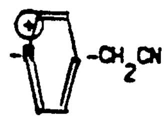 | $-CO_2^{\ominus}$ |
| 45 | $-CH_2CH_2-$ | 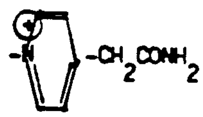 | $-CO_2^{\ominus}$ |
| 46 | $-CH_2CH_2-$ | 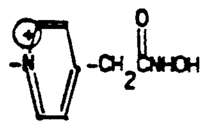 | $-CO_2^{\ominus}$ |
| 47 | $-CH_2CH_2-$ | 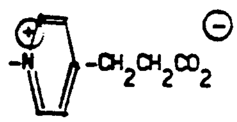 | $-CO_2Na$ |
| 48 | $-CH_2CH_2-$ | 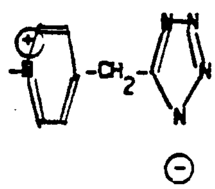 | $-CO_2Na$ |
| 49 | $-CH_2CH_2-$ | 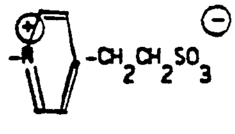 | $-CO_2Na$ |

| Compound No. | L | 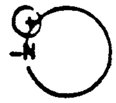 | Y |
|---|---|---|---|
| 50 | $-CH_2CH_2-$ | 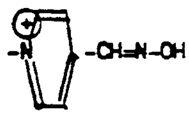 | $-CO_2^{\ominus}$ |
| 51 | $-CH_2CH_2-$ | 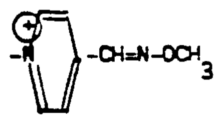 | $-CO_2^{\ominus}$ |
| 52 | $-CH_2$ | 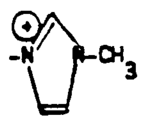 | $-CO_2^{\ominus}$ |
| 53 | $-CH_2CH_2-$ | 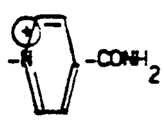 | $-CO_2^{\ominus}$ |
| 54 | $-CH_2CH_2-$ | 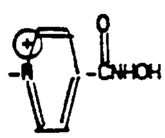 | $-CO_2^{\ominus}$ |
| 55 | $-CH_2CH_2-$ | 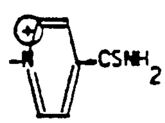 | $-CO_2^{\ominus}$ |

| Compound No. | L | 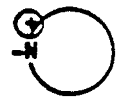 | Y |
|---|---|---|---|
| 56 | $-CH_2CH_2-$ | 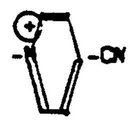 | $-CO_2^{\ominus}$ |
| 57 | $-CH_2CH_2-$ | 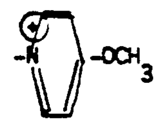 | $-CO_2^{\ominus}$ |
| 58 | $-CH_2CH_2-$ | 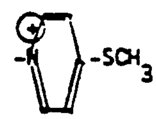 | $-CO_2^{\ominus}$ |
| 59 | $-CH_2CH_2-$ | 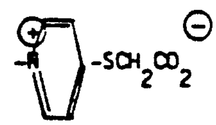 | $-CO_2Na$ |
| 60 | $-CH_2CH_2-$ | 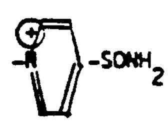 | $-CO_2^{\ominus}$ |
| 61 | $-CH_2CH_2-$ | 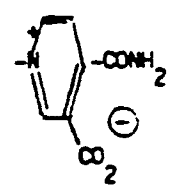 | $-CO_2Na$ |

| Compound No. | L | 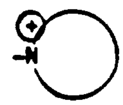 | Y |
|---|---|---|---|
| 62 | $-CH_2CH_2-$ | 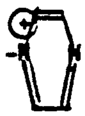 | $-CO_2^{\ominus}$ |
| 63 | $-CH_2CH_2-$ | 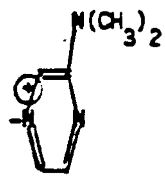 | $-CO_2^{\ominus}$ |
| 64 | $-CH_2CH_2-$ | 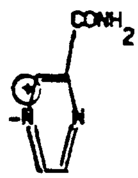 | $-CO_2^{\ominus}$ |
| 65 | $-CH_2CH_2-$ | 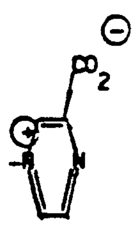 | $-CO_2Na$ |
| 66 | $-CH(CH_3)CH_2-$ | 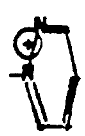 | $-CO_2^{\ominus}$ |
| 67 | $-CH_2CH_2-$ | 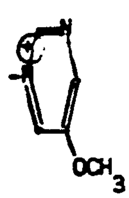 | $-CO_2^{\ominus}$ |

| Compound No. | L | 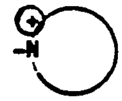 | Y |
|---|---|---|---|
| 68 | $-CH_2CH_2-$ | 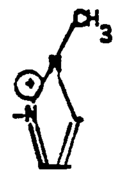 | $-CO_2^{\ominus}$ |
| 69 | $-CH_2CH_2-$ | 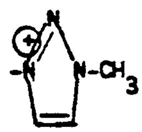 | $-CO_2^{\ominus}$ |
| 70 | $-CH_2CH_2-$ | 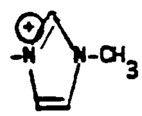 | $-CO_2^{\ominus}$ |
| 71 | $-CH_2CH_2-$ | 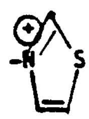 | $-CO_2^{\ominus}$ |
| 72 | $-CH(CH_3)CH_2-$ | 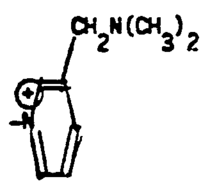 | $-CO_2^{\ominus}$ |
| 73 | $-CH(CH_3)CH_2-$ | 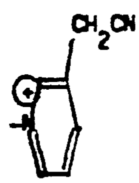 | $-CO_2^{\ominus}$ |

| Compound No. | L | 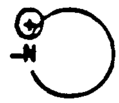 | Y |
|---|---|---|---|
| 74 | $-CH(CH_3)CH_2-$ | 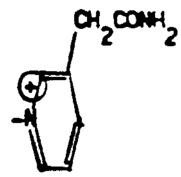 | $-CO_2^{\ominus}$ |
| 75 | $-CH(CH_3)CH_2-$ | 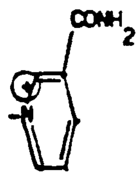 | $-CO_2^{\ominus}$ |
| 76 | $-CH(CH_3)CH_2-$ | 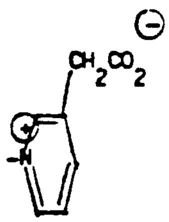 | $-CO_2Na$ |
| 77 | $-CH(CH_3)CH_2-$ | 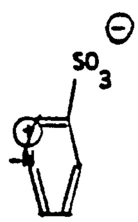 | $-CO_2Na$ |
| 78 | $-CH(CH_3)CH_2-$ | 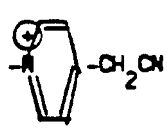 | $-CO_2^{\ominus}$ |
| 79 | $-CH(CH_3)CH_2-$ | 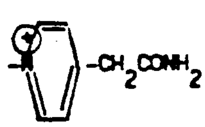 | $-CO_2^{\ominus}$ |

0168707

| Compound No. | L | 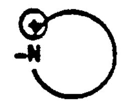 | Y |
| --- | --- | --- | --- |
| 80 | $-CH(CH_3)CH_2-$ | 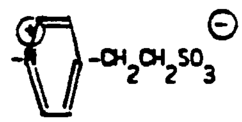 | $-CO_2Na$ |
| 81 | $-CH(CH_3)CH_2-$ | 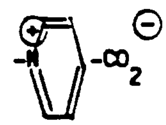 | $-CO_2Na$ |
| 82 | $-CH(CH_3)CH_2-$ | 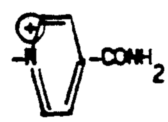 | $-CO_2^{\ominus}$ |
| 83 | $-CH_2$ | 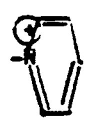 | $-CO_2^{\ominus}$ |
| 84 | $-CH_2$ | 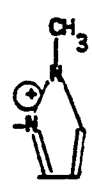 | $-CO_2^{\ominus}$ |
| 85 | $-CH_2$ | 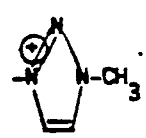 | $-CO_2^{\ominus}$ |

| Compound No. | L | 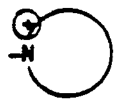 | Y |
|---|---|---|---|
| 86 | $-CH(CH_3)-$ | 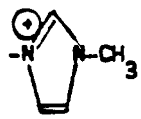 | $-CO_2^{\ominus}$ |
| 87 | $-CH_2CH_2CH_2-$ | 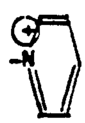 | $-CO_2^{\ominus}$ |
| 88 | $-CH_2CH_2-$ | 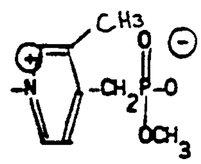 | $-CO_2Na$ |
| 89 | $-CH_2CH_2-$ | 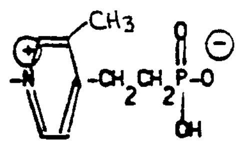 | $-CO_2Na$ |
| 90 | $-CH_2CH_2-$ | 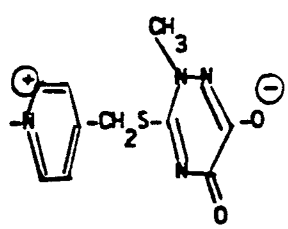 | $-CO_2Na$ |
| 91 | $-CH_2$ | 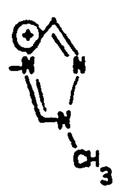 | $-CO_2^{\ominus}$ |

| Compound No. | L | 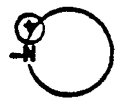 | Y |
|---|---|---|---|
| 92 | $-CH_2-$ | 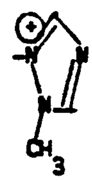 | $-CO_2^{\ominus}$ |
| 93 | $-CH_2-$ | 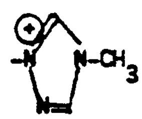 | $-CO_2^{\ominus}$ |
| 94 | $-CH_2-$ | 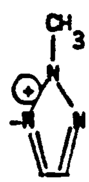 | $-CO_2^{\ominus}$ |
| 95 | $-CH_2-$ | 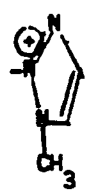 | $-CO_2^{\ominus}$ |
| 96 | $-CH_2-$ | 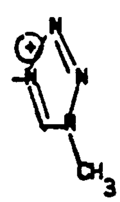 | $-CO_2^{\ominus}$ |
| 97 | $-CH_2-$ | 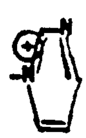 | $-CO_2^{\ominus}$ |
| 98 | $-CH_2-$ | 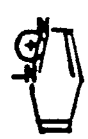 | $-CO_2^{\ominus}$ |

| Compound No. | L | 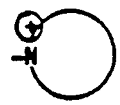 | Y |
|---|---|---|---|
| 99 | $-CH_2-$ | 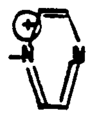 | $-CO_2^{\ominus}$ |
| 100 | $-CH_2-$ | 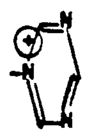 | $-CO_2^{\ominus}$ |
| 101 | $-CH_2-$ | 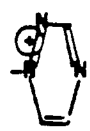 | $-CO_2^{\ominus}$ |
| 102 | $-CH_2-$ | 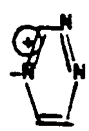 | $-CO_2^{\ominus}$ |
| 103 | $-CH_2-$ | 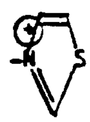 | $-CO_2^{\ominus}$ |
| 104 | $-CH_2-$ | 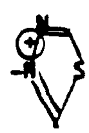 | $-CO_2^{\ominus}$ |
| 105 | $-CH_2-$ | 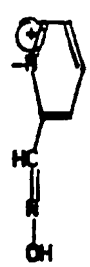 | $-CO_2^{\ominus}$ |

| Compound No. | L | 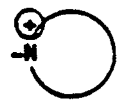 | Y |
|---|---|---|---|
| 106 | $-CH_2CH_2-$ | 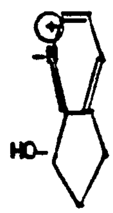 | $-CO_2^{\ominus}$ |
| 107 | $-CH_2CH_2-$ | 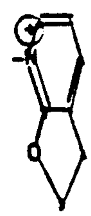 | $-CO_2^{\ominus}$ |
| 108. | $-CH_2CH_2-$ | 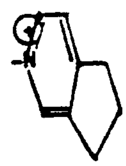 | $-CO_2^{\ominus}$ |
| 109 | $-CH_2CH_2-$ | 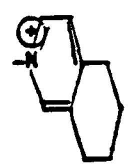 | $-CO_2^{\ominus}$ |
| 110 | $-CH_2CH_2-$ | 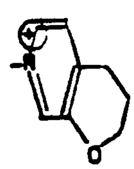 | $-CO_2^{\ominus}$ |

| Compound No. | L | 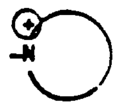 | Y |
|---|---|---|---|
| 111 | $-CH_2CH_2-$ | 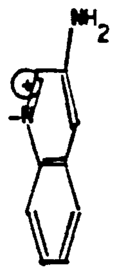 | $-CO_2^{\ominus}$ |
| 112 | $-CH_2CH_2-$ | 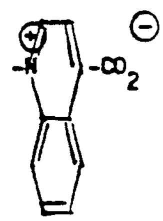 | $-CO_2Na$ |
| 113 | $-CH_2CH_2-$ | 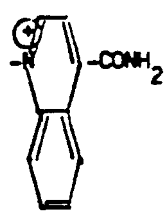 | $-CO_2^{\ominus}$ |
| 114 | $-CH_2CH_2-$ | 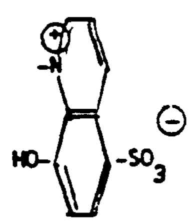 | $-CO_2Na$ |
| 115 | $-CH_2CH_2-$ | 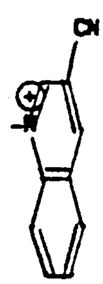 | $-CO_2^{\ominus}$ |

| Compound No. | L | 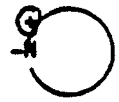 | Y |
|---|---|---|---|
| 116 | $-CH_2CH_2-$ | 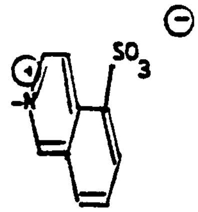 | $-CO_2Na$ |
| 117 | $-CH_2CH_2-$ | 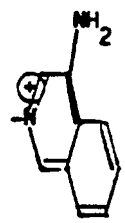 | $-CO_2^{\ominus}$ |
| 118 | $-CH_2CH_2-$ | 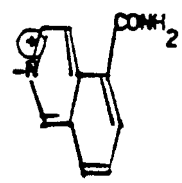 | $-CO_2^{\ominus}$ |
| 119 | $-CH_2CH_2-$ | 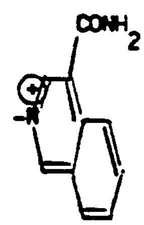 | $-CO_2^{\ominus}$ |
| 120 | $-CH_2CH_2-$ | 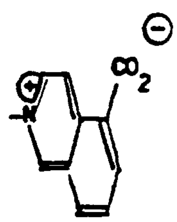 | $-CO_2Na$ |

| Compound No. | L | 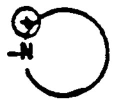 | Y |
|---|---|---|---|
| 121 | $-CH_2CH_2-$ | 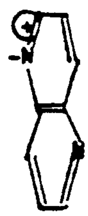 | $-CO_2^{\ominus}$ |
| 122 | $-CH_2CH_2-$ | 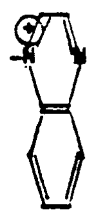 | $-CO_2^{\ominus}$ |
| 123 | $-CH_2CH_2-$ | 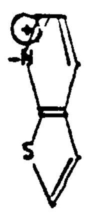 | $-CO_2^{\ominus}$ |
| 124 | $-CH_2CH_2-$ | 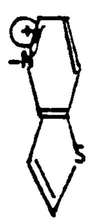 | $-CO_2^{\ominus}$ |
| 125 | $-CH_2CH_2-$ | 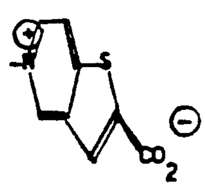 | $-CO_2Na$ |

| Compound No. | L | 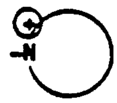 | Y |
|---|---|---|---|
| 126 | $-CH_2CH_2-$ | 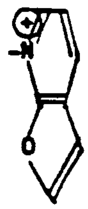 | $-CO_2^{\ominus}$ |
| 127 | $-CH_2CH_2-$ | 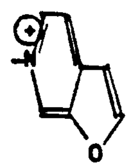 | $-CO_2^{\ominus}$ |
| 128 | $-CH_2CH_2-$ | 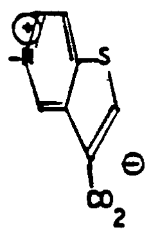 | $-CO_2Na$ |
| 129 | $-CH_2CH_2-$ | 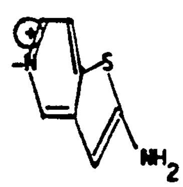 | $-CO_2^{\ominus}$ |
| 130 | $-CH_2CH_2-$ | 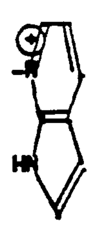 | $-CO_2^{\ominus}$ |

| Compound No. | L | 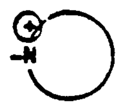 | Y |
|---|---|---|---|
| 131 | $-CH_2CH_2-$ | 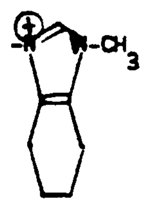 | $-CO_2^{\ominus}$ |
| 132 | $-CH_2CH_2-$ | 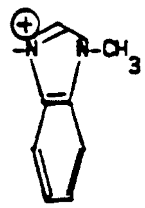 | $-CO_2^{\ominus}$ |
| 133 | $-CH_2CH_2-$ | 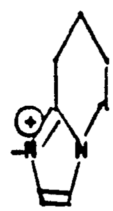 | $-CO_2^{\ominus}$ |
| 134 | $-CH_2CH_2-$ | 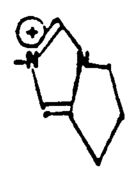 | $-CO_2^{\ominus}$ |
| 135 | $-CH_2CH_2-$ | 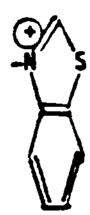 | $-CO_2Na$ |

| Compound No. | L | 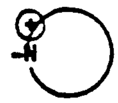 | Y |
|---|---|---|---|
| 136 | $-CH_2CH_2-$ | 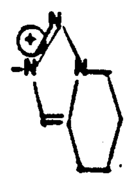 | $-CO_2^{\ominus}$ |
| 137 | $-CH_2CH_2-$ | 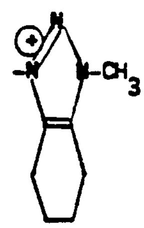 | $-CO_2^{\ominus}$ |
| 138. | $-CH_2CH_2-$ | 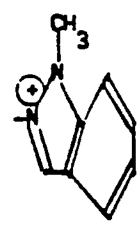 | $-CO_2^{\ominus}$ |
| 139 | $-CH_2CH_2-$ | 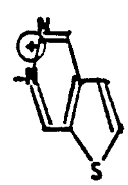 | $-CO_2^{\ominus}$ |
| 140 | $-CH_2CH_2-$ | 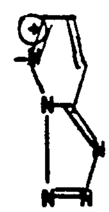 | $-CO_2Na$ |

| Compound No. | L | 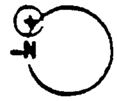 | Y |
|---|---|---|---|
| 141 | $-CH_2CH_2-$ | 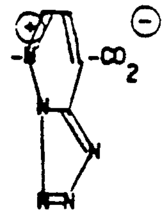 | $-CO_2Na$ |
| 142 | $-CH_2CH_2-$ | 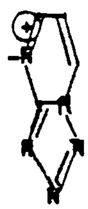 | $-CO_2^{\ominus}$ |
| 143 | $-CH_2CH_2-$ | 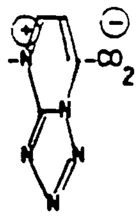 | $-CO_2Na$ |
| 144 | $-CH_2CH_2-$ | 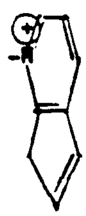 | $-CO_2^{\ominus}$ |
| 145 | $-CH_2CH_2-$ | 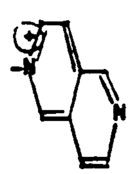 | $-CO_2^{\ominus}$ |

| Compound No. | L | 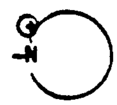 | Y |
|---|---|---|---|
| 146 | $CH_3$<br>$-CH-CH_2-$ | 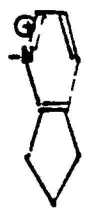 | $-CO_2^{\ominus}$ |
| 147 | $-CH_2-$ | 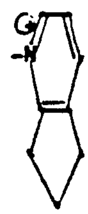 | $-CO_2^{\ominus}$ |
| 148 | $-CH_2-$ | 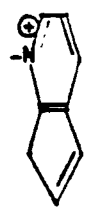 | $-CO_2^{\ominus}$ |
| 149 | $-CH_2-$ | 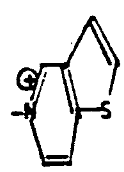 | $-CO_2^{\ominus}$ |
| 150 | $-CH_2-$ | 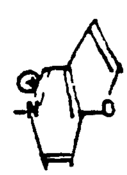 | $-CO_2^{\ominus}$ |

| Compound No. | L | 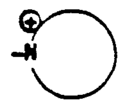 | Y |
|---|---|---|---|
| 151 | $-CH_2-$ | 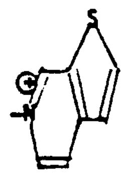 | $-CO_2^{\ominus}$ |
| 152 | $-CH_2-$ | 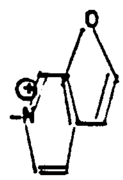 | $-CO_2^{\ominus}$ |
| 153 | $-CH_2-$ | 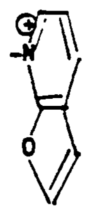 | $-CO_2^{\ominus}$ |
| 154 | $-CH_2-$ | 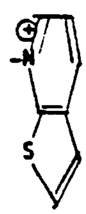 | $-CO_2^{\ominus}$ |
| 155 | $-CH_2-$ | 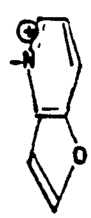 | $-CO_2^{\ominus}$ |

| Compound No. | L | 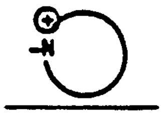 | Y |
|---|---|---|---|
| 156 | $-CH_2-$ | 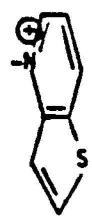 | $-CO_2^{\ominus}$ |
| 157 | $-CH_2-$ | 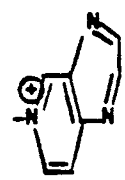 | $-CO_2^{\ominus}$ |
| 158 | $-CH_2-$ | 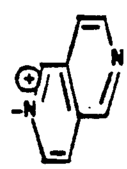 | $-CO_2^{\ominus}$ |
| 159 | $-CH_2-$ | 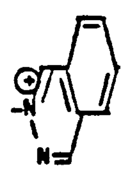 | $-CO_2^{\ominus}$ |
| 160 | $-CH_2-$ | 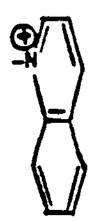 | $-CO_2^{\ominus}$ |
| 161 | $-CH_2CH_2-$ | 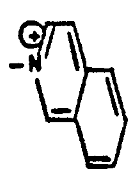 | $-CO_2^{\ominus}$ |

| Compound No. | L | 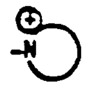 | Y |
|---|---|---|---|
| 162 | $-CH_2-$ | 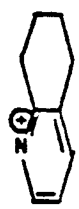 | $-CO_2^{\ominus}$ |
| 163 | $-CH_2CH_2-$ | 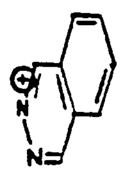 | $-CO_2^{\ominus}$ |
| 164 | $-CH_2CH_2-$ | 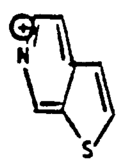 | $-CO_2^{\ominus}$ |
| 165 | $-CH_2CH_2-$ | 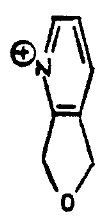 | $-CO_2^{\ominus}$ |
| 166 | $-CH_2CH_2-$ | 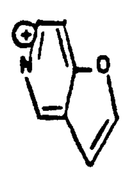 | $-CO_2^{\ominus}$ |

| Compound No. | L | 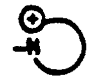 | Y |
| --- | --- | --- | --- |
| 167 | $-CH(CH_3)CH_2-$ | 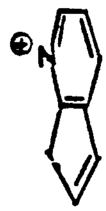 | $-CO_2^{\ominus}$ |
| 168 | $-CH_2CH_2-$ | 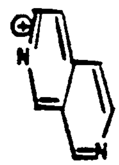 | $-CO_2^{\ominus}$ |
| 169 | $-CH_2CH_2-$ | 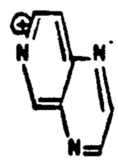 | $-CO_2^{\ominus}$ |
| 170 | $-CH_2CH_2-$ | 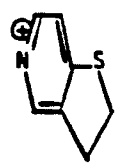 | $-CO_2^{\ominus}$ |
| 171 | $-CH_2CH_2-$ | 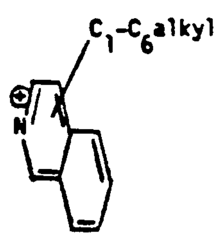 | $-CO_2^{\ominus}$ |
| 172 | $-CH_2CH_2-$ | 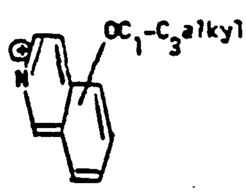 | $-CO_2^{\ominus}$ |

| Compound No. | L | 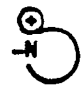 | Y |
|---|---|---|---|
| 173 | $-CH_2CH_2-$ | 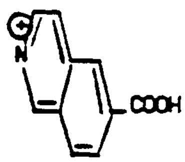 | $-CO_2Na$ |
| 174 | $-CH_2CH_2-$ | 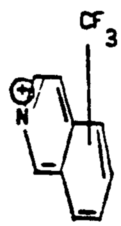 | $-CO_2^{\ominus}$ |
| 175 | $-CH_2CH_2-$ | 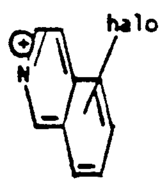 | $-CO_2^{\ominus}$ |
| 176 | $-CH(CH_3)CH_2-$ | 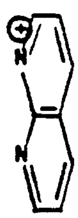 | $-CO_2^{\ominus}$ |
| 177 | $-CH(CH_3)CH_2-$ | 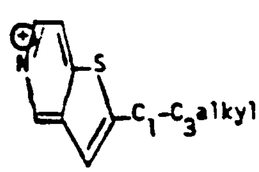 | $-CO_2^{\ominus}$ |

| Compound No. | L | 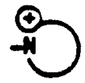 | Y |
|---|---|---|---|
| 178 | $-CH(CH_3)CH_2-$ | 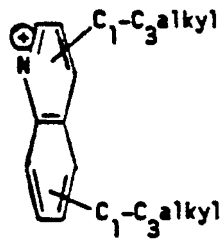 | $-CO_2^{\ominus}$ |
| 179 | $-CH(CH_3)CH_2-$ | 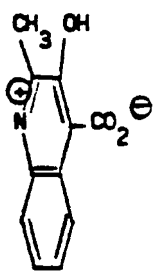 | $-CO_2Na$ |
| 180 | $-CH(CH_3)CH_2-$ | 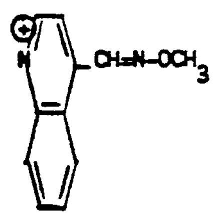 | $-CO_2^{\ominus}$ |
| 181 | $-CH(CH_3)CH_2-$ | 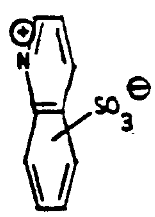 | $-CO_2Na$ |
| 182 | $-CH(CH_3)CH_2-$ | 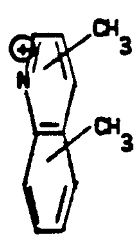 | $-CO_2^{\ominus}$ |

| Compound No | L | 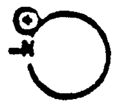 | Y |
| --- | --- | --- | --- |
| 183 | $-CH_2CH_2-$ | 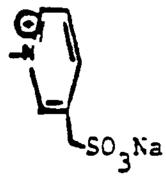 | $-CO_2^{\ominus}$ |
| 184 | $-CH_2CH_2-$ | 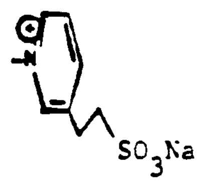 | $-CO_2^{\ominus}$ |
| 185 | $-CH_2CH_2-$ | 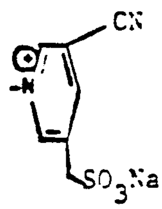 | $-CO_2^{\ominus}$ |
| 186 | $-CH_2CH_2-$ | 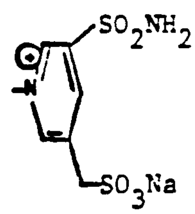 | $-CO_2^{\ominus}$ |
| 187 | $-CH_2CH_2-$ | 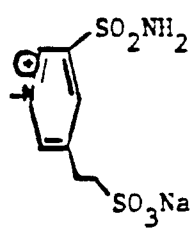 | $-CO_2^{\ominus}$ |

| Compound No | L | 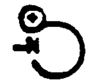 | Y |
|---|---|---|---|
| 188 | $-CH_2CH_2-$ | 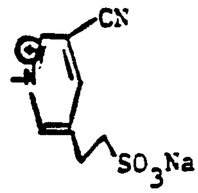 | $-CO_2^{\ominus}$ |
| 189 | $-CH_2CH_2-$ | 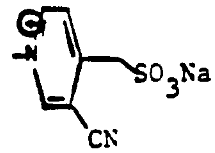 | $-CO_2^{\ominus}$ |
| 190 | $-CH_2CH_2-$ | 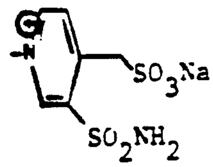 | $-CO_2^{\ominus}$ |
| 191 | $-CH_2CH_2-$ | 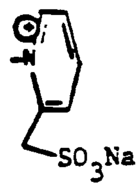 | $-CO_2^{\ominus}$ |
| 192 | $-CH_2CH_2-$ | 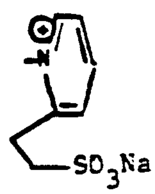 | $-CO_2^{\ominus}$ |
| 193 | $-CH_2CH_2-$ | 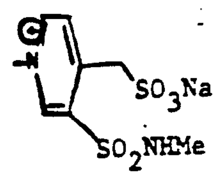 | $-CO_2^{\ominus}$ |

| Compound No | L | 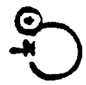 | Y |
|---|---|---|---|
| 194 | $-CH_2CH_2-$ | 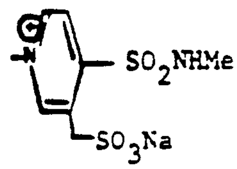 | $-CO_2^{\ominus}$ |
| 195 | $-CH_2CH_2-$ | 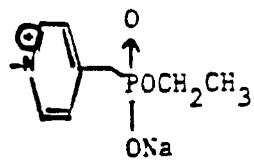 | $-CO_2^{\ominus}$ |
| 196 | $-CH_2CH_2-$ | 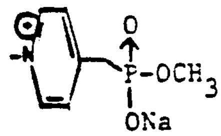 | $-CO_2^{\ominus}$ |
| 197 | $-CH_2CH_2-$ | 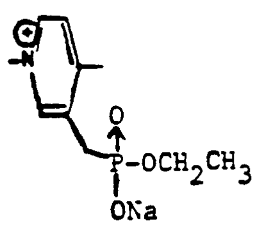 | $-CO_2^{\ominus}$ |
| 198 | $-CH_2CH_2-$ | 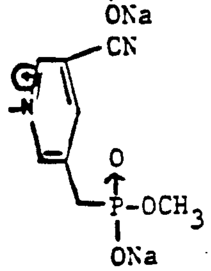 | $-CO_2^{\ominus}$ |
| 199 | $-CH_2CH_2-$ | 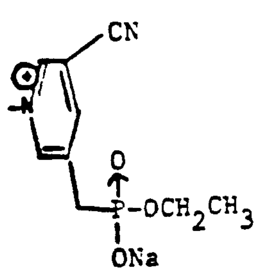 | $-CO_2^{\ominus}$ |

| Compound No. | L | 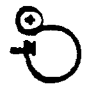 | Y |
|---|---|---|---|
| 200 | $-CH_2CH_2-$ | 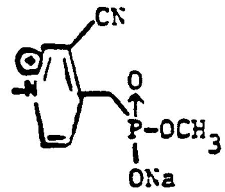 | $-CO_2^{\ominus}$ |
| 201 | $-CH_2CH_2-$ | 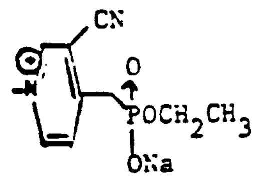 | $-CO_2^{\ominus}$ |
| 202 | $-CH_2CH_2-$ | 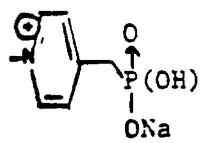 | $-CO_2^{\ominus}$ |
| 203 | $-CH_2CH_2-$ | 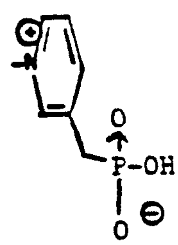 | $-CO_2^{\ominus}$ |
| 204 | $-CH_2CH_2-$ | 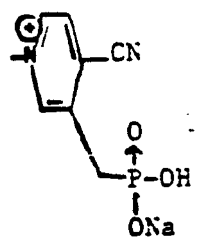 | $-CO_2^{\ominus}$ |
| 205 | $-CH_2CH_2-$ | 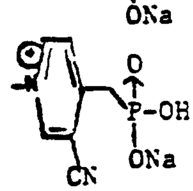 | $-CO_2^{\ominus}$ |

| Compound No | L | 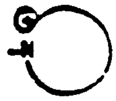 | Y |
| --- | --- | --- | --- |
| 206 | $-CH_2CH_2-$ | 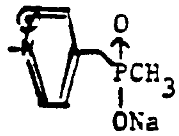 | $-CO_2^{\ominus}$ |
| 207 | $-CH_2CH_2-$ | 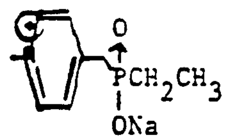 | $-CO_2^{\ominus}$ |
| 208 | $-CH_2CH_2-$ | 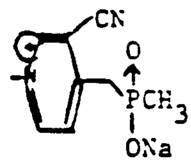 | $-CO_2^{\ominus}$ |
| 209 | $-CH_2CH_2-$ | 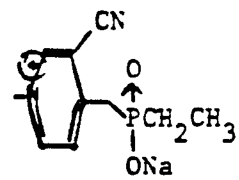 | $-CO_2^{\ominus}$ |
| 210 | $-CH_2CH_2-$ | 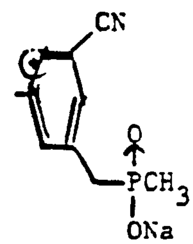 | $-CO_2^{\ominus}$ |
| 211 | $-CH_2CH_2-$ | 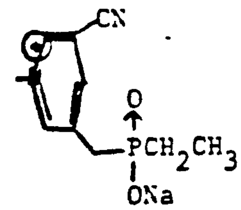 | $-CO_2^{\ominus}$ |

| Compound No | L | 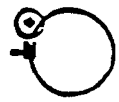 | Y |
|---|---|---|---|
| 212 | $-CH_2CH_2-$ | 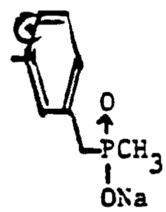 | $-CO_2^{\ominus}$ |
| 213 | $-CH_2CH_2-$ | 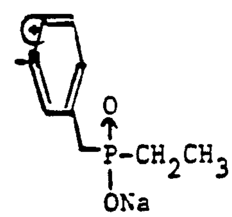 | $-CO_2^{\ominus}$ |
| 214 | $-CH_2CH_2-$ | 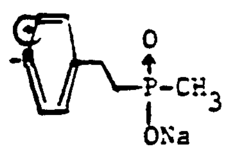 | $-CO_2^{\ominus}$ |
| 215 | $-CH_2CH_2-$ | 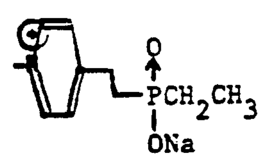 | $-CO_2^{\ominus}$ |
| 216 | $-CH_2CH_2-$ | 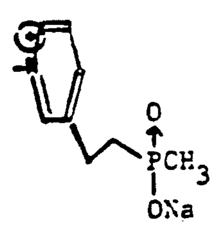 | $-CO_2^{\ominus}$ |
| 217 | $-CH_2CH_2-$ | 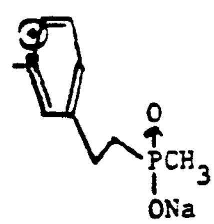 | $-CO_2^{\ominus}$ |

| Compound No | L | 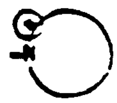 | Y |
|---|---|---|---|
| 218 | $-CH_2CH_2-$ | 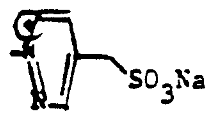 | $-CO_2^{\ominus}$ |
| 219 | $-CH_2CH_2-$ | 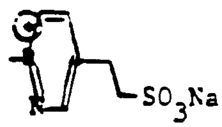 | $-CO_2^{\ominus}$ |
| 220 | $-CH_2CH_2-$ | 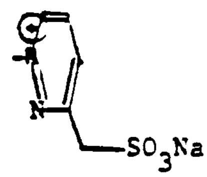 | $-CO_2$ |
| 221 | $-CH_2CH_2-$ | 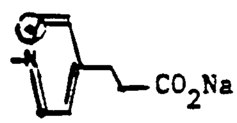 | $-CO_2^{\ominus}$ |
| 222 | $-CH_2CH_2-$ | 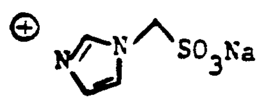 | $-CO_2^{\ominus}$ |
| 223 | $-CH_2CH_2-$ | 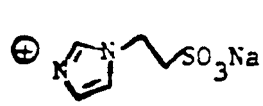 | $-CO_2^{\ominus}$ |

| Compound No | L | 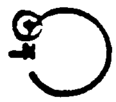 | Y |
|---|---|---|---|
| 224 | $-CH_2CH_2-$ | 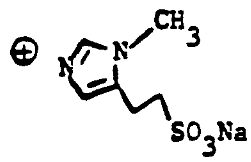 | $-CO_2^{\ominus}$ |
| 225 | $-CH_2CH_2-$ | 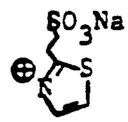 | $-CO_2^{\ominus}$ |
| 226 | $-CH_2CH_2-$ | 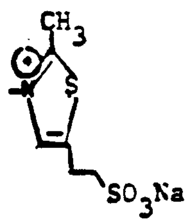 | $-CO_2^{\ominus}$ |
| 227 | $-CH_2CH_2-$ | 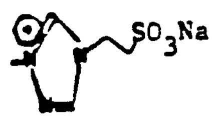 | $-CO_2^{\ominus}$ |
| 228 | $-CH_2CH_2-$ | 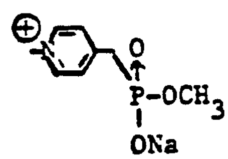 | $-CO_2^{\ominus}$ |

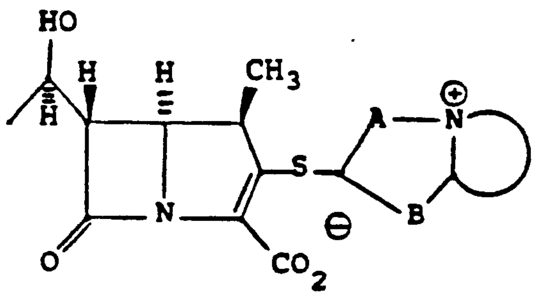 where 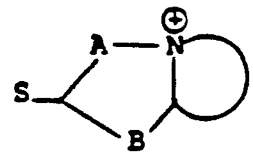 is

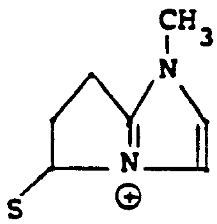 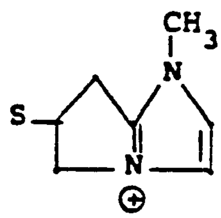 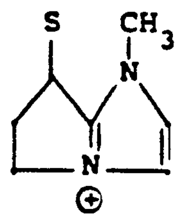

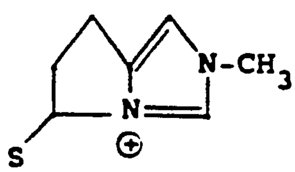 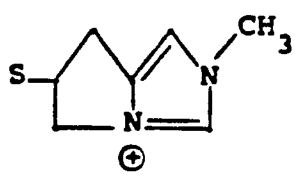 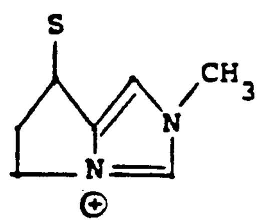

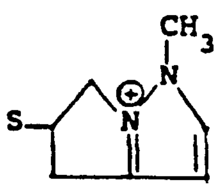 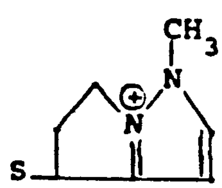 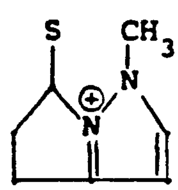

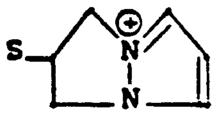 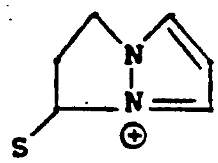 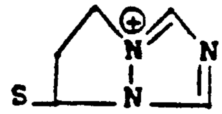

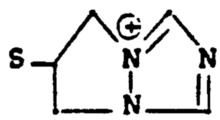 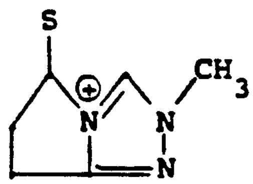 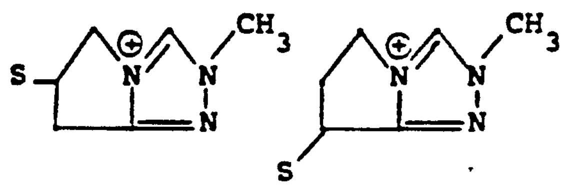

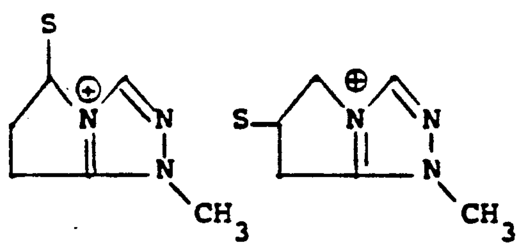 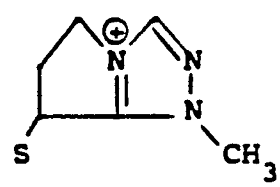

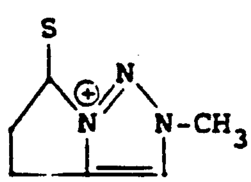 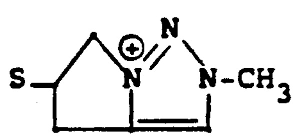 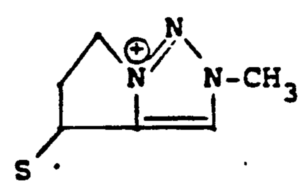

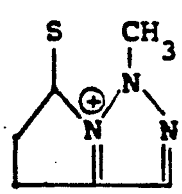 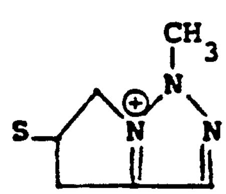 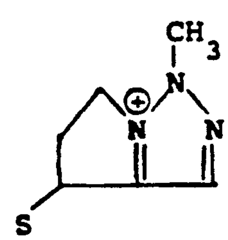

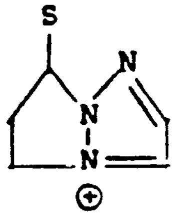
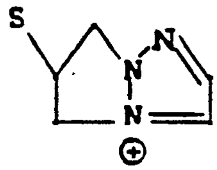
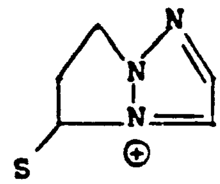

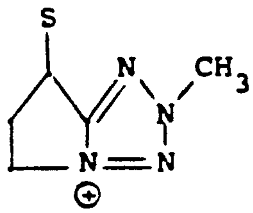
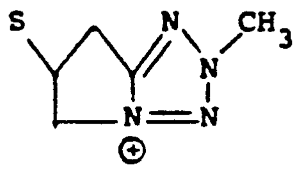
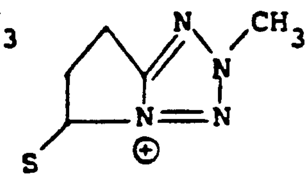

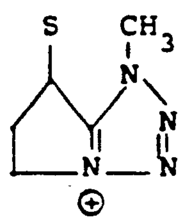
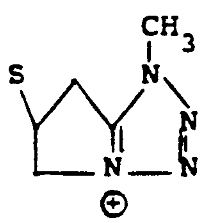
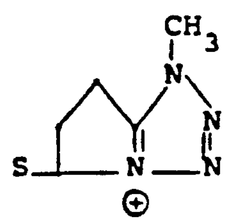

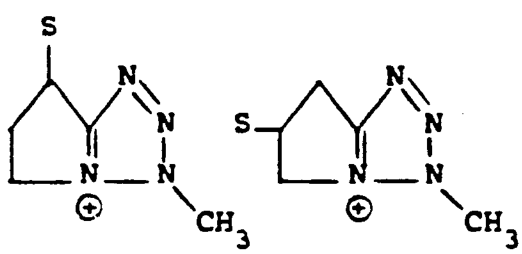
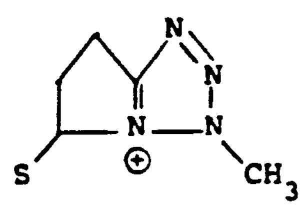

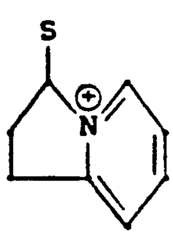
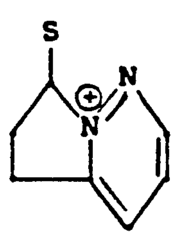
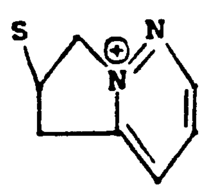

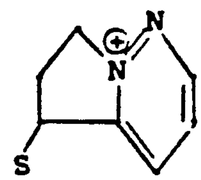
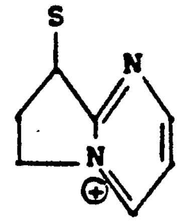
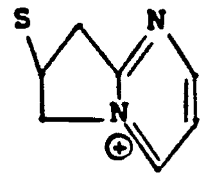

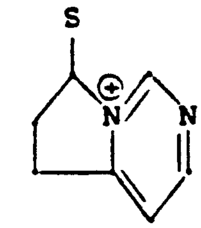
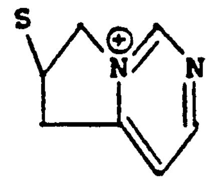
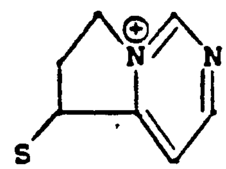

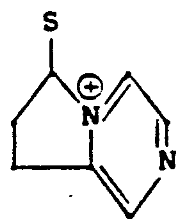
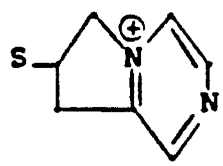
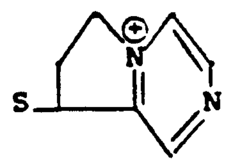

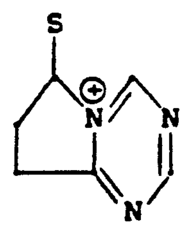
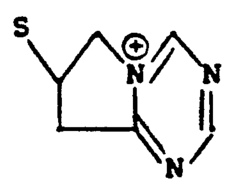
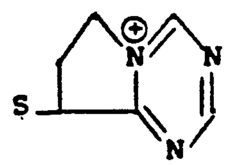

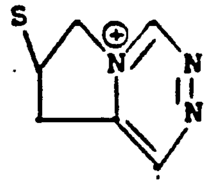
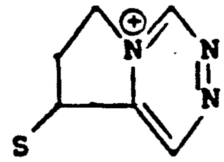
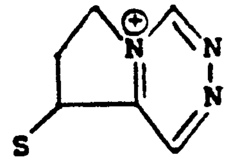

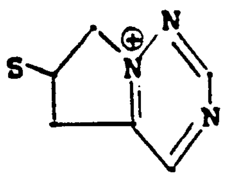
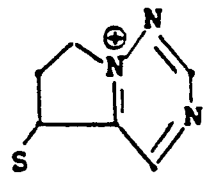
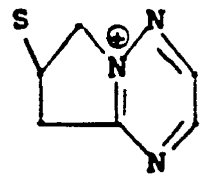
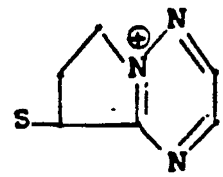

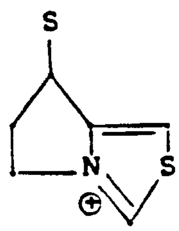
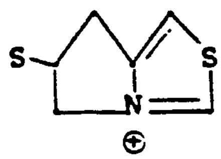
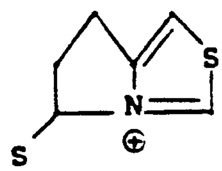

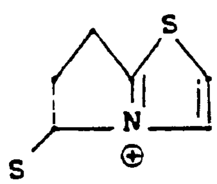
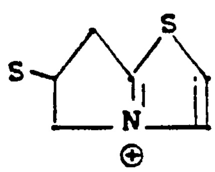
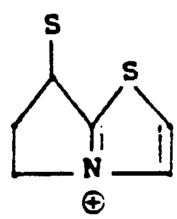

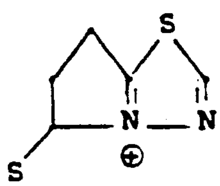
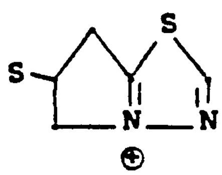
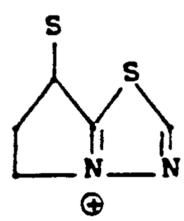

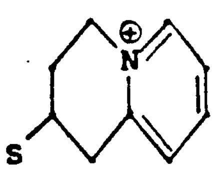
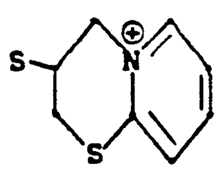
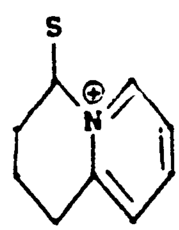

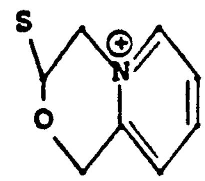
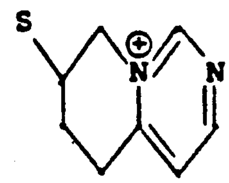
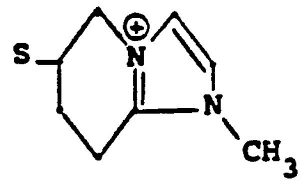

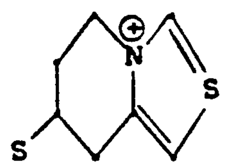
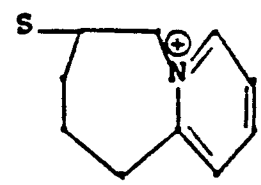
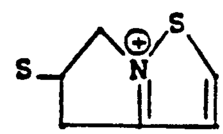

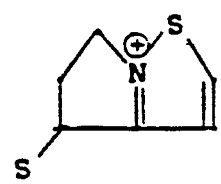
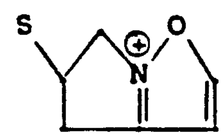
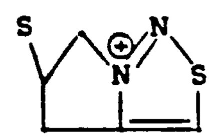

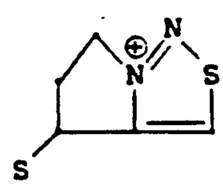
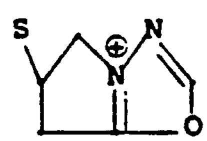
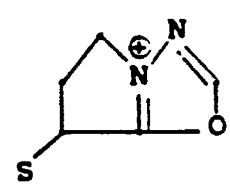

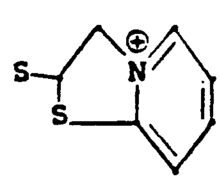
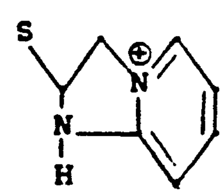
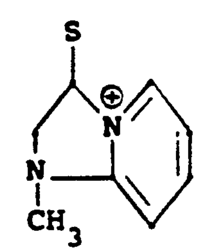

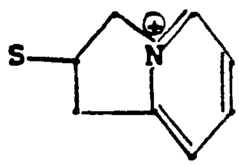

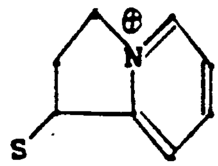

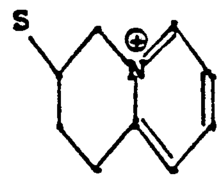

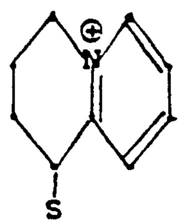

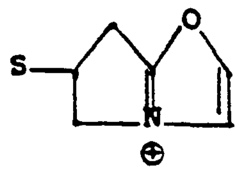

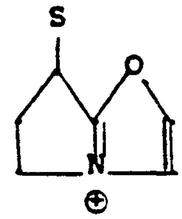

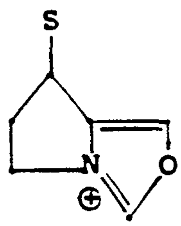

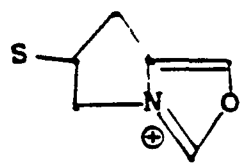

3. A compound of Claim 1 wherein the compound is a member selected from the group consisting of:

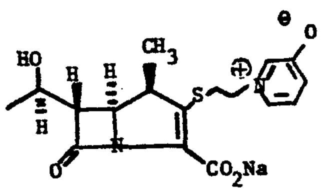
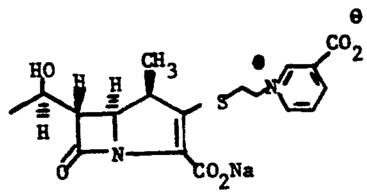
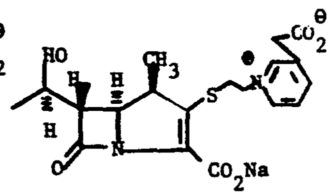

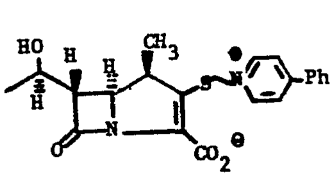
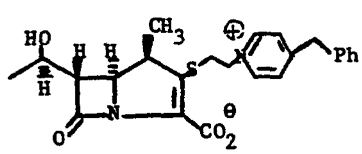
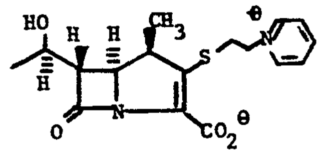

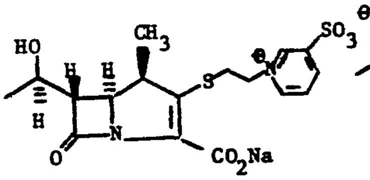
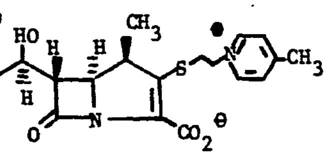
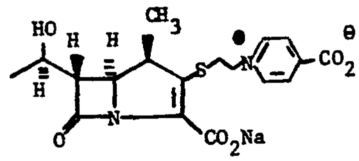

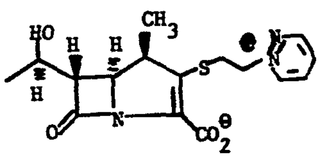
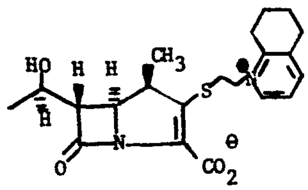
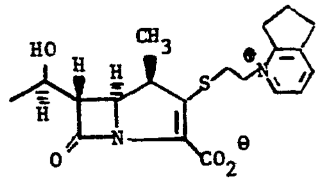

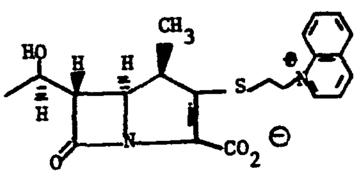
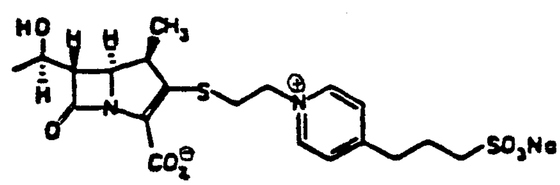

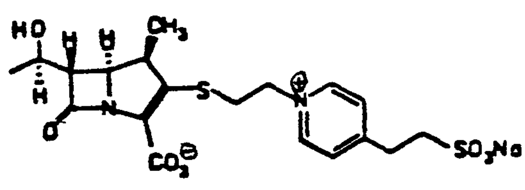
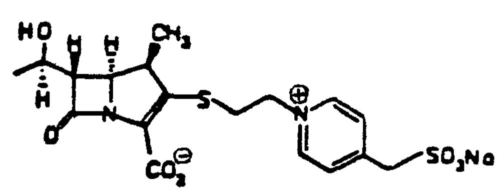

4. The combination of a compound of Claim 1 and a DHP inhibitor.

5. A combination of Claim 4wherein the DHP inhibitor is 7-(L-2-amino-2-carboxyethylthio)-2-(2,2-dimethylcyclopropanecarboxamide)-2-heptenoic acid.

6. A pharmaceutical composition for antibiotic use comprising an antibacterially effective amount of a compound of Claim 1, an inhibitorily effective amount of a DHP inhibitor, and, optionally, a pharmaceutically acceptable carrier.

7. A compound of Claim 1 of the structure:

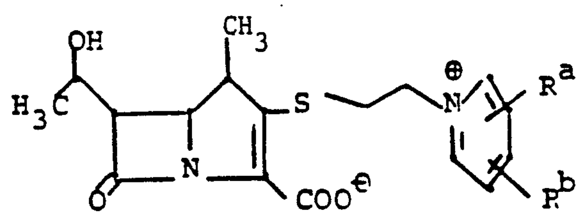

wherein

$R^a$ is hydrogen or cyano; and

$R^b$ is an acidic side-chain of the structure $-(CH_2)_n-X-(CH_2)_m-Y-A$ where:

n = 0-4

m = 0-4

X = $CHR^s$, CH=CH, phenylene ($-C_6H_4-$), NH, N(C1-C4 alkyl), O, S, S=O, C=O, $SO_2$, $SO_2NH$, $CO_2$, CONH, $OCO_2$, OC=O, NHC=O;

$R^s$ = H, O(C1-C4 alkyl), $NH_2$, NH(C1-C4 alkyl), N(C1-C4 alkyl)$_2$, CN, $CONH_2$, CON(C1-C4 alkyl)$_2$, $CO_2H$, $SO_2NH_2$, $SO_2NH$(C1-C4 alkyl);

Y = single bond, NH, N(C1-C4 alkyl), O, S; and

A = an acidic function.

8. A compound of Claim 7 wherein the acidic function A is a member selected from the group consisting essentially of carboxy ($CO_2H$), phosphono [$P{=}O(OH)_2$], alkylphosphono {$P{=}O(OH)$-[$C(C_1$-$C_4$ alkyl)]}, alkylphosphinyl [$P{=}O(OH)$-($C_1$-$C_4$ alkyl)], substituted phosphoramido [$P{=}O(OH)NH(C_1$-$C_4$ alkyl) and $P{=}O(OH)NHR^x$], sulfino ($SO_2H$), sulfo ($SO_3H$), 5-tetrazolyl ($CN_4H$), arylsulfonamido ($SO_2NHR^x$) and acylsulfonamides represented by the structures $CONHSO_2(C_1$-$C_4$ alkyl), $CONHSO_2N(C_1$-$C_4$alkyl$)_2$ - $SO_2NHCO(C_1$-$C_4$ alkyl) and $SO_2NHCOR^x$, wherein $R^x$ = aryl or heteroaryl.

9. A pharmaceutical composition for antibiotic use comprising an antibacterially effective amount of a compound of Claim 8 , an inhibitorily effective amount of a DHP inhibitor, and, optionally, a pharmaceutical carrier.

European Patent Office

**EUROPEAN SEARCH REPORT**

0168707

Application number

EP 85108134.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | DE - A1 - 3 312 533 (BRISTOL MYERS CO.)<br>* Claims 1,2,4,6,7,96 *<br>-- | 1-3,6-9 | C 07 D 487/04<br>A 61 K 31/40<br>C 07 D 519/00 |
| P,A | DE - A1 - 3 408 347 (BRISTOL MYERS CO.)<br>* Claim 1; page 70 *<br>-- | 1-3,6-9 | |
| A | DE - A1 - 3 321 864 (BRISTOL MYERS CO.)<br>* Claims 1,131 *<br>-- | 1-3,6-9 | |
| D,A | EP - A1 - 0 007 614 (MERCK & CO. INC.)<br>* Claims 1,7; page 17, lines 5,6*<br>-- | 4-6,9 | **TECHNICAL FIELDS SEARCHED (Int. Cl 4)** |
| D,A | EP - A1 - 0 072 014 (MERCK & CO. INC.)<br>* Claims 1,7,14 *<br>---- | 4-6,9 | C 07 D 487/00<br>A 61 K 31/00<br>C 07 D 519/00 |

The present search report has been drawn up for all claims

| Place of search | Date of completion of the search | Examiner |
|---|---|---|
| VIENNA | 25-09-1985 | PETROUSEK |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82